# EUROPEAN PATENT SPECIFICATION

(11) **EP 0 812 207 B1**
(45) Date of publication and mention of the grant of the patent: **02.11.2000**
(21) Application number: 96904646.5
(22) Date of filing: 14.02.1996
(51) Int. Cl.: A61K 38/18

(54) **MORPHOGEN-INDUCED DENTINE REGENERATION**
MORPHOGENINDUZIERTE REGENERIERUNG DER DENTIN
REGENERATION DE LA DENTINE INDUITE PAR UN MORPHOGENE

(30) Priority: 01.03.1995 US 396930
(43) Date of publication of application: 17.12.1997
(73) Proprietor: STRYKER CORPORATION, Kalamazoo, MI 49001 (US)
(72) Inventor: CHARETTE, Marc, F., Needham, MA 02192 (US); RUTHERFORD, Robert, Bruce, Farmington, CT 02032 (US)
(74) Representative: Price, Vincent Andrew
(86) International application number: US9602169
(87) International publication number: WO9626737

(56) References cited:
- WO-A-92/15323
- WO-A-94/06399
- ARCH. ORAL BIOL., vol. 38, no. 7, 1993, pages 571-576, XP000573091 R. BRUCE RUTHERFORD ET AL.: "Induction of reparative dentine formation in monkeys by recombinant human osteogenic protein-1" cited in the application
- ARCHS. ORAL BIOL., vol. 35, no. 7, 1990, pages 493-497, XP000573089 M. NAKASHIMA: "The induction of reparative dentine in the amputated dental pulp of the dog by bone morphogenetic protein" cited in the application
- MOL. PATHOG. PERIODONTAL DIS. (1994), 427-37. EDITOR(S): GENCO, ROBERT. PUBLISHER: AM. SOC. MICROBIOL., WASHINGTON, D. C. CODEN: 61LAA2, 1994, XP000576244 RUTHERFORD, BRUCE ET AL: "Role of osteogenic ( bone morphogenetic ) protein and platelet-derived growth factor in periodontal wound healing"
- RUTHERFORD B ET AL: "OSTEOGENIC PROTEIN-1 INDUCES FORMATION OF REPARATIVE DENTIN." , JOINT MEETING OF THE INTERNATIONAL ASSOCIATION FOR DENTAL RESEARCH, THE AMERICAN ASSOCIATION OF DENTAL RESEARCH AND THE CANADIAN ASSOCIATION OF DENTAL RESEARCH, CHICAGO, ILLINOIS, USA, MARCH 10-14, 1993. J DENT RES 72 (ABSTR. SPEC. ISSUE). 1993. 213. XP002008041 see abstract

### SEQUENCE LISTING

(1) GENERAL INFORMATION:
   (i) APPLICANT:
      (A) NAME: CREATIVE BIOMOLECULES, INC
      (B) STREET: 45 SOUTH STREET
      (C) CITY: HOPKINTON
      (D) STATE: MA
      (E) COUNTRY: USA
      (F) POSTAL CODE (ZIP): 01748
      (G) TELEPHONE: 1-508-435-9001
      (H) TELEFAX: 1-508-435-0454
      (I) TELEX:
   (ii) TITLE OF INVENTION: MORPHOGEN-INDUCED DENTINE REGENERATION
   (iii) NUMBER OF SEQUENCES: 32
   (iv) CORRESPONDENCE ADDRESS:
      (A) ADDRESSEE: CREATIVE BIOMOLECULES, INC
      (B) STREET: 45 SOUTH STREET
      (C) CITY: HOPKINTON
      (D) STATE: MA
      (E) COUNTRY: USA
      (F) ZIP: 01748
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: FENTON, GILLIAN M
      (B) REGISTRATION NUMBER: 36,508
      (C) REFERENCE/DOCKET NUMBER: CRP-088PC
   (ix) TELECOMMUNICATION INFORMATION:
      (A) TELEPHONE: (617) 248-7000
      (B) TELEFAX: (617) 248-7100
(2) INFORMATION FOR SEQ ID NO:1:
   (i) SEQUENCE CHARACTERISTICS:
      (A) LENGTH: 97 amino acids
      (B) TYPE: amino acid
      (C) STRANDEDNESS:
      (D) TOPOLOGY: linear
   (ii) MOLECULE TYPE: protein
   (ix) FEATURE:
      (A) NAME/KEY: Protein
      (B) LOCATION: 1..97
      (D) OTHER INFORMATION: /label= Generic-Seq-7
         /note= "wherein each Xaa is independently selected from a group of one or more specified amino acids as defined in the specification."
   (xi) SEQUENCE DESCRIPTION: SEQ ID NO:1:
   Leu Xaa Xaa Xaa Phe Xaa Xaa Xaa Gly Trp Xaa Xaa Xaa Xaa Xaa Xaa
   1               5                   10                  15
   Pro Xaa Xaa Xaa Xaa Ala Xaa Tyr Cys Xaa Gly Xaa Cys Xaa Xaa Pro
               20                  25                  30
   Xaa Xaa Xaa Xaa Xaa Xaa Xaa Xaa Asn His Ala Xaa Xaa Xaa Xaa Xaa
           35                  40                  45
   Xaa Xaa Xaa Xaa Xaa Xaa Xaa Xaa Xaa Xaa Xaa Xaa Cys Cys Xaa Pro
       50                  55                  60
   Xaa Xaa Xaa Xaa Xaa Xaa Xaa Xaa Leu Xaa Xaa Xaa Xaa Xaa Xaa Xaa
   65                  70                  75                  80
   Val Xaa Leu Xaa Xaa Xaa Xaa Xaa Met Xaa Val Xaa Xaa Cys Xaa Cys
                   85                  90                  95
   Xaa
(2) INFORMATION FOR SEQ ID NO:2:
   (i) SEQUENCE CHARACTERISTICS:
      (A) LENGTH: 102 amino acids
      (B) TYPE: amino acid
      (C) STRANDEDNESS:
      (D) TOPOLOGY: linear
   (ii) MOLECULE TYPE: protein
   (ix) FEATURE:
      (A) NAME/KEY: Protein
      (B) LOCATION: 1..102
      (D) OTHER INFORMATION: /label= Generic-Seq-8
         /note= "wherein each Xaa is independently selected from a group of one or more specified amino acids as defined in the specification."
   (xi) SEQUENCE DESCRIPTION: SEQ ID NO:2:
   Cys Xaa Xaa Xaa Xaa Leu Xaa Xaa Xaa Phe Xaa Xaa Xaa Gly Trp Xaa
   1               5                   10                  15
   Xaa Xaa Xaa Xaa Xaa Pro Xaa Xaa Xaa Xaa Ala Xaa Tyr Cys Xaa Gly
               20                  25                  30
   Xaa Cys Xaa Xaa Pro Xaa Xaa Xaa Xaa Xaa Xaa Xaa Xaa Asn His Ala
           35                  40                  45
   Xaa Xaa Xaa Xaa Xaa Xaa Xaa Xaa Xaa Xaa Xaa Xaa Xaa Xaa Xaa Xaa
       50                  55                  60
   Xaa Cys Cys Xaa Pro Xaa Xaa Xaa Xaa Xaa Xaa Xaa Xaa Leu Xaa Xaa
   65                  70                  75                  80
   Xaa Xaa Xaa Xaa Xaa Val Xaa Leu Xaa Xaa Xaa Xaa Xaa Met Xaa Val
                   85                  90                  95
   Xaa Xaa Cys Xaa Cys Xaa
               100
(2) INFORMATION FOR SEQ ID NO:3:
   (i) SEQUENCE CHARACTERISTICS:
      (A) LENGTH: 102 amino acids
      (B) TYPE: amino acid
      (C) STRANDEDNESS:
      (D) TOPOLOGY: linear
   (ii) MOLECULE TYPE: protein
   (ix) FEATURE:
      (A) NAME/KEY: Protein
      (B) LOCATION: 1..102
      (D) OTHER INFORMATION: /label= OPX
         /note= "wherein each Xaa is independently selected from a group of one or more specified amino acids as defined in the specification."
   (xi) SEQUENCE DESCRIPTION: SEQ ID NO:3:
   Cys Xaa Xaa His Glu Leu Tyr Val Xaa Phe Xaa Asp Leu Gly Trp Xaa
   1               5                   10                  15
   Asp Trp Xaa Ile Ala Pro Xaa Gly Tyr Xaa Ala Tyr Tyr Cys Glu Gly
               20                  25                  30
   Glu Cys Xaa Phe Pro Leu Xaa Ser Xaa Met Asn Ala Thr Asn His Ala
           35                  40                  45
   Ile Xaa Gln Xaa Leu Val His Xaa Xaa Xaa Pro Xaa Xaa Val Pro Lys
       50                  55                  60
   Xaa Cys Cys Ala Pro Thr Xaa Leu Xaa Ala Xaa Ser Val Leu Tyr Xaa
   65                  70                  75                  80
   Asp Xaa Ser Xaa Asn Val Xaa Leu Xaa Lys Xaa Arg Asn Met Val Val
                   85                  90                  95
   Xaa Ala Cys Gly Cys His
               100
(2) INFORMATION FOR SEQ ID NO:4:
   (i) SEQUENCE CHARACTERISTICS:
      (A) LENGTH: 139 amino acids
      (B) TYPE: amino acid
      (C) STRANDEDNESS:
      (D) TOPOLOGY: linear
   (ii) MOLECULE TYPE: protein
   (vi) ORIGINAL SOURCE:
      (A) ORGANISM: Homo sapiens
      (F) TISSUE TYPE: HIPPOCAMPUS
   (ix) FEATURE:
      (A) NAME/KEY: Protein
      (B) LOCATION: 1..139
      (D) OTHER INFORMATION: /label= hOP1-MATURE
   (xi) SEQUENCE DESCRIPTION: SEQ ID NO:4:
   Ser Thr Gly Ser Lys Gln Arg Ser Gln Asn Arg Ser Lys Thr Pro Lys
   1               5                   10                  15
   Asn Gln Glu Ala Leu Arg Met Ala Asn Val Ala Glu Asn Ser Ser Ser
               20                  25                  30
   Asp Gln Arg Gln Ala Cys Lys Lys His Glu Leu Tyr Val Ser Phe Arg
           35                  40                  45
   Asp Leu Gly Trp Gln Asp Trp Ile Ile Ala Pro Glu Gly Tyr Ala Ala
       50                  55                  60
   Tyr Tyr Cys Glu Gly Glu Cys Ala Phe Pro Leu Asn Ser Tyr Met Asn
   65                  70                  75                  80
   Ala Thr Asn His Ala Ile Val Gln Thr Leu Val His Phe Ile Asn Pro
                   85                  90                  95
   Glu Thr Val Pro Lys Pro Cys Cys Ala Pro Thr Gln Leu Asn Ala Ile
               100                 105                 110
   Ser Val Leu Tyr Phe Asp Asp Ser Ser Asn Val Ile Leu Lys Lys Tyr
           115                 120                 125
   Arg Asn Met Val Val Arg Ala Cys Gly Cys His
       130                 135
(2) INFORMATION FOR SEQ ID NO:5:
   (i) SEQUENCE CHARACTERISTICS:
      (A) LENGTH: 139 amino acids
      (B) TYPE: amino acid
      (C) STRANDEDNESS:
      (D) TOPOLOGY: linear
   (ii) MOLECULE TYPE: protein
   (vi) ORIGINAL SOURCE:
      (A) ORGANISM: MURIDAE
      (F) TISSUE TYPE: EMBRYO
   (ix) FEATURE:
      (A) NAME/KEY: Protein
      (B) LOCATION: 1..139
      (D) OTHER INFORMATION: /label= mOP1-MATURE
   (xi) SEQUENCE DESCRIPTION: SEQ ID NO:5:
   Ser Thr Gly Gly Lys Gln Arg Ser Gln Asn Arg Ser Lys Thr Pro Lys
   1               5                   10                  15
   Asn Gln Glu Ala Leu Arg Met Ala Ser Val Ala Glu Asn Ser Ser Ser
               20                  25                  30
   Asp Gln Arg Gln Ala Cys Lys Lys His Glu Leu Tyr Val Ser Phe Arg
           35                  40                  45
   Asp Leu Gly Trp Gln Asp Trp Ile Ile Ala Pro Glu Gly Tyr Ala Ala
       50                  55                  60
   Tyr Tyr Cys Glu Gly Glu Cys Ala Phe Pro Leu Asn Ser Tyr Met Asn
   65                  70                  75                  80
   Ala Thr Asn His Ala Ile Val Gln Thr Leu Val His Phe Ile Asn Pro
                   85                  90                  95
   Asp Thr Val Pro Lys Pro Cys Cys Ala Pro Thr Gln Leu Asn Ala Ile
               100                 105                 110
   Ser Val Leu Tyr Phe Asp Asp Ser Ser Asn Val Ile Leu Lys Lys Tyr
           115                 120                 125
   Arg Asn Met Val Val Arg Ala Cys Gly Cys His
       130                 135
(2) INFORMATION FOR SEQ ID NO:6:
   (i) SEQUENCE CHARACTERISTICS:
      (A) LENGTH: 139 amino acids
      (B) TYPE: amino acid
      (C) STRANDEDNESS:
      (D) TOPOLOGY: linear
   (ii) MOLECULE TYPE: protein
   (vi) ORIGINAL SOURCE:
      (A) ORGANISM: HOMO SAPIENS
      (F) TISSUE TYPE: HIPPOCAMPUS
   (ix) FEATURE:
      (A) NAME/KEY: Protein
      (B) LOCATION: 1..139
      (D) OTHER INFORMATION: /label= HOP2-MATURE
   (xi) SEQUENCE DESCRIPTION: SEQ ID NO:6:
   Ala Val Arg Pro Leu Arg Arg Arg Gln Pro Lys Lys Ser Asn Glu Leu
   1               5                   10                  15
   Pro Gln Ala Asn Arg Leu Pro Gly Ile Phe Asp Asp Val His Gly Ser
               20                  25                  30
   His Gly Arg Gln Val Cys Arg Arg His Glu Leu Tyr Val Ser Phe Gln
           35                  40                  45
   Asp Leu Gly Trp Leu Asp Trp Val Ile Ala Pro Gln Gly Tyr Ser Ala
       50                  55                  60
   Tyr Tyr Cys Glu Gly Glu Cys Ser Phe Pro Leu Asp Ser Cys Met Asn
   65                  70                  75                  80
   Ala Thr Asn His Ala Ile Leu Gln Ser Leu Val His Leu Met Lys Pro
                   85                  90                  95
   Asn Ala Val Pro Lys Ala Cys Cys Ala Pro Thr Lys Leu Ser Ala Thr
               100                 105                 110
   Ser Val Leu Tyr Tyr Asp Ser Ser Asn Asn Val Ile Leu Arg Lys His
           115                 120                 125
   Arg Asn Met Val Val Lys Ala Cys Gly Cys His
       130                 135
(2) INFORMATION FOR SEQ ID NO:7:
   (i) SEQUENCE CHARACTERISTICS:
      (A) LENGTH: 139 amino acids
      (B) TYPE: amino acid
      (C) STRANDEDNESS:
      (D) TOPOLOGY: linear
   (ii) MOLECULE TYPE: protein
   (vi) ORIGINAL SOURCE:
      (A) ORGANISM: MURIDAE
      (F) TISSUE TYPE: EMBRYO
   (ix) FEATURE:
      (A) NAME/KEY: Protein
      (B) LOCATION: 1..139
      (D) OTHER INFORMATION: /label= MOP2-MATURE
   (xi) SEQUENCE DESCRIPTION: SEQ ID NO:7:
   Ala Ala Arg Pro Leu Lys Arg Arg Gln Pro Lys Lys Thr Asn Glu Leu
   1               5                   10                  15
   Pro His Pro Asn Lys Leu Pro Gly Ile Phe Asp Asp Gly His Gly Ser
               20                  25                  30
   Arg Gly Arg Glu Val Cys Arg Arg His Glu Leu Tyr Val Ser Phe Arg
           35                  40                  45
   Asp Leu Gly Trp Leu Asp Trp Val Ile Ala Pro Gln Gly Tyr Ser Ala
       50                  55                  60
   Tyr Tyr Cys Glu Gly Glu Cys Ala Phe Pro Leu Asp Ser Cys Met Asn
   65                  70                  75                  80
   Ala Thr Asn His Ala Ile Leu Gln Ser Leu Val His Leu Met Lys Pro
                   85                  90                  95
   Asp Val Val Pro Lys Ala Cys Cys Ala Pro Thr Lys Leu Ser Ala Thr
               100                 105                 110
   Ser Val Leu Tyr Tyr Asp Ser Ser Asn Asn Val Ile Leu Arg Lys His
           115                 120                 125
   Arg Asn Met Val Val Lys Ala Cys Gly Cys His
       130                 135
(2) INFORMATION FOR SEQ ID NO:8:
   (i) SEQUENCE CHARACTERISTICS:
      (A) LENGTH: 101 amino acids
      (B) TYPE: amino acid
      (C) STRANDEDNESS:
      (D) TOPOLOGY: linear
   (ii) MOLECULE TYPE: protein
   (vi) ORIGINAL SOURCE:
      (A) ORGANISM: bovinae
   (ix) FEATURE:
      (A) NAME/KEY: Protein
      (B) LOCATION: 1..101
      (D) OTHER INFORMATION: /label= CBMP-2A-FX
   (xi) SEQUENCE DESCRIPTION: SEQ ID NO:8:
   Cys Lys Arg His Pro Leu Tyr Val Asp Phe Ser Asp Val Gly Trp Asn
   1 5 10 15
   Asp Trp Ile Val Ala Pro Pro Gly Tyr His Ala Phe Tyr Cys His Gly
   20 25 30
   Glu Cys Pro Phe Pro Leu Ala Asp His Leu Asn Ser Thr Asn His Ala
   35 40 45
   Ile Val Gln Thr Leu Val Asn Ser Val Asn Ser Lys Ile Pro Lys Ala
   50 55 60
   Cys Cys Val Pro Thr Glu Leu Ser Ala Ile Ser Met Leu Tyr Leu Asp
   65 70 75 80
   Glu Asn Glu Lys Val Val Leu Lys Asn Tyr Gln Asp Met Val Val Glu
   85 90 95
   Gly Cys Gly Cys Arg
   100
(2) INFORMATION FOR SEQ ID NO:9:
   (i) SEQUENCE CHARACTERISTICS:
      (A) LENGTH: 101 amino acids
      (B) TYPE: amino acid
      (C) STRANDEDNESS:
      (D) TOPOLOGY: linear
   (ii) MOLECULE TYPE: protein
   (vi) ORIGINAL SOURCE:
      (A) ORGANISM: HOMO SAPIENS
      (F) TISSUE TYPE: hippocampus
   (ix) FEATURE:
      (A) NAME/KEY: Protein
      (B) LOCATION: 1..101
      (D) OTHER INFORMATION: /label= CBMP-2B-FX
   (xi) SEQUENCE DESCRIPTION: SEQ ID NO:9:
   Cys Arg Arg His Ser Leu Tyr Val Asp Phe Ser Asp Val Gly Trp Asn
   1               5                   10                  15
   Asp Trp Ile Val Ala Pro Pro Gly Tyr Gln Ala Phe Tyr Cys His Gly
               20                  25                  30
   Asp Cys Pro Phe Pro Leu Ala Asp His Leu Asn Ser Thr Asn His Ala
           35                  40                  45
   Ile Val Gln Thr Leu Val Asn Ser Val Asn Ser Ser Ile Pro Lys Ala
       50                  55                  60
   Cys Cys Val Pro Thr Glu Leu Ser Ala Ile Ser Met Leu Tyr Leu Asp
   65                  70                  75                      80
   Glu Tyr Asp Lys Val Val Leu Lys Asn Tyr Gln Glu Met Val Val Glu
                   85                  90                  95
   Gly Cys Gly Cys Arg
               100
(2) INFORMATION FOR SEQ ID NO:10:
   (i) SEQUENCE CHARACTERISTICS:
      (A) LENGTH: 102 amino acids
      (B) TYPE: amino acid
      (C) STRANDEDNESS:
      (D) TOPOLOGY: linear
   (ii) MOLECULE TYPE: protein
   (vi) ORIGINAL SOURCE:
      (A) ORGANISM: DROSOPHILA MELANOGASTER
(ix) FEATURE:
   (A) NAME/KEY: Protein
   (B) LOCATION: 1..102
   (D) OTHER INFORMATION: /label= DPP-FX
(xi) SEQUENCE DESCRIPTION: SEQ ID NO:10:
   Cys Arg Arg His Ser Leu Tyr Val Asp Phe Ser Asp Val Gly Trp Asp
   1               5                   10                  15
   Asp Trp Ile Val Ala Pro Leu Gly Tyr Asp Ala Tyr Tyr Cys His Gly
               20                  25                  30
   Lys Cys Pro Phe Pro Leu Ala Asp His Phe Asn Ser Thr Asn His Ala
           35                  40                      45
   Val Val Gln Thr Leu Val Asn Asn Asn Asn Pro Gly Lys Val Pro Lys
       50                  55                  60
   Ala Cys Cys Val Pro Thr Gln Leu Asp Ser Val Ala Met Leu Tyr Leu
   65                  70                  75                  80
   Asn Asp Gln Ser Thr Val Val Leu Lys Asn Tyr Gln Glu Met Thr Val
                   85                  90                  95
   Val Gly Cys Gly Cys Arg
               100
(2) INFORMATION FOR SEQ ID NO:11:
   (i) SEQUENCE CHARACTERISTICS:
      (A) LENGTH: 102 amino acids
      (B) TYPE: amino acid
      (C) STRANDEDNESS:
      (D) TOPOLOGY: linear
   (ii) MOLECULE TYPE: protein
   (vi) ORIGINAL SOURCE:
      (A) ORGANISM: XENOPUS
   (ix) FEATURE:
      (A) NAME/KEY: Protein
      (B) LOCATION: 1..102
      (D) OTHER INFORMATION: /label= VGL-FX
   (xi) SEQUENCE DESCRIPTION: SEQ ID NO:11:
   Cys Lys Lys Arg His Leu Tyr Val Glu Phe Lys Asp Val Gly Trp Gln
   1               5                   10                  15
   Asn Trp Val Ile Ala Pro Gln Gly Tyr Met Ala Asn Tyr Cys Tyr Gly
               20                  25                  30
   Glu Cys Pro Tyr Pro Leu Thr Glu Ile Leu Asn Gly Ser Asn His Ala
           35                  40                  45
   Ile Leu Gln Thr Leu Val His Ser Ile Glu Pro Glu Asp Ile Pro Leu
       50                  55                  60
   Pro Cys Cys Val Pro Thr Lys Met Ser Pro Ile Ser Met Leu Phe Tyr
   65                  70                  75                  80
   Asp Asn Asn Asp Asn Val Val Leu Arg His Tyr Glu Asn Met Ala Val
                   85                  90                  95
   Asp Glu Cys Gly Cys Arg
               100
(2) INFORMATION FOR SEQ ID NO:12:
   (i) SEQUENCE CHARACTERISTICS:
      (A) LENGTH: 102 amino acids
      (B) TYPE: amino acid
      (C) STRANDEDNESS:
      (D) TOPOLOGY: linear
   (ii) MOLECULE TYPE: protein
   (vi) ORIGINAL SOURCE:
      (A) ORGANISM: MURIDAE
   (ix) FEATURE:
      (A) NAME/KEY: Protein
      (B) LOCATION: 1..102
      (D) OTHER INFORMATION: /label= VGR-1-FX
   (xi) SEQUENCE DESCRIPTION: SEQ ID NO:12:
   Cys Lys Lys His Glu Leu Tyr Val Ser Phe Gln Asp Val Gly Trp Gln
   1               5                   10                  15
   Asp Trp Ile Ile Ala Pro Lys Gly Tyr Ala Ala Asn Tyr Cys Asp Gly
               20                  25                  30
   Glu Cys Ser Phe Pro Leu Asn Ala His Met Asn Ala Thr Asn His Ala
           35                  40                  45
   Ile Val Gln Thr Leu Val His Val Met Asn Pro Glu Tyr Val Pro Lys
       50                  55                  60
   Pro Cys Cys Ala Pro Thr Lys Val Asn Ala Ile Ser Val Leu Tyr Phe
   65                  70                  75                  80
   Asp Asp Asn Ser Asn Val Ile Leu Lys Lys Tyr Arg Asn Met Val Val
                   85                  90                  95
   Arg Ala Cys Gly Cys His
               100
(2) INFORMATION FOR SEQ ID NO:13:
   (i) SEQUENCE CHARACTERISTICS:
      (A) LENGTH: 106 amino acids
      (B) TYPE: amino acid
      (C) STRANDEDNESS:
      (D) TOPOLOGY: linear
   (ii) MOLECULE TYPE: protein
   (vi) ORIGINAL SOURCE:
      (A) ORGANISM: Homo sapiens
      (F) TISSUE TYPE: brain
   (ix) FEATURE:
      (A) NAME/KEY: Protein
      (B) LOCATION: 1..106
      (D) OTHER INFORMATION: /note= "GDF-1 (fx)"
      (xi) SEQUENCE DESCRIPTION: SEQ ID NO:13:
   Cys Arg Ala Arg Arg Leu Tyr Val Ser Phe Arg Glu Val Gly Trp His
   1               5                   10                  15
   Arg Trp Val Ile Ala Pro Arg Gly Phe Leu Ala Asn Tyr Cys Gln Gly
               20                  25                  30
   Gln Cys Ala Leu Pro Val Ala Leu Ser Gly Ser Gly Gly Pro Pro Ala
           35                  40                  45
   Leu Asn His Ala Val Leu Arg Ala Leu Met His Ala Ala Ala Pro Gly
       50                  55                  60
   Ala Ala Asp Leu Pro Cys Cys Val Pro Ala Arg Leu Ser Pro Ile Ser
   65                  70                  75                  80
   Val Leu Phe Phe Asp Asn Ser Asp Asn Val Val Leu Arg Gln Tyr Glu
                   85                  90                  95
   Asp Met Val Val Asp Glu Cys Gly Cys Arg
               100                 105
(2) INFORMATION FOR SEQ ID NO:14:
   (i) SEQUENCE CHARACTERISTICS:
      (A) LENGTH: 5 amino acids
      (B) TYPE: amino acid
      (C) STRANDEDNESS:
      (D) TOPOLOGY: linear
   (ii) MOLECULE TYPE: peptide
   (xi) SEQUENCE DESCRIPTION: SEQ ID NO:14:
   Cys Xaa Xaa Xaa Xaa
   1               5
(2) INFORMATION FOR SEQ ID NO:15:
   (i) SEQUENCE CHARACTERISTICS:
      (A) LENGTH: 1822 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: HOMO SAPIENS
      (F) TISSUE TYPE: HIPPOCAMPUS
   (ix) FEATURE:
      (A) NAME/KEY: CDS
      (B) LOCATION: 49..1341
      (C) IDENTIFICATION METHOD: experimental
      (D) OTHER INFORMATION: /function=
         "OSTEOGENIC PROTEIN"
         /product= "OP1"
         /evidence= EXPERIMENTAL
         /standard_name= "OP1"
   (xi) SEQUENCE DESCRIPTION: SEQ ID NO:15:
   GGTGCGGGCC CGGAGCCCGG AGCCCGGGTA GCGCGTAGAG CCGGCGCG ATG CAC GTG      57
                                                        Met His Val
                                                         1
   CGC TCA CTG CGA GCT GCG GCG CCG CAC AGC TTC GTG GCG CTC TGG GCA      105
   Arg Ser Leu Arg Ala Ala Ala Pro His Ser Phe Val Ala Leu Trp Ala
        5                  10                  15
   CCC CTG TTC CTG CTG CGC TCC GCC CTG GCC GAC TTC AGC CTG GAC AAC      153
   Pro Leu Phe Leu Leu Arg Ser Ala Leu Ala Asp Phe Ser Leu Asp Asn
    20                  25                  30                  35
   GAG GTG CAC TCG AGC TTC ATC CAC CGG CGC CTC CGC AGC CAG GAG CGG      201
   Glu Val His Ser Ser Phe Ile His Arg Arg Leu Arg Ser Gln Glu Arg
                   40                  45                  50
   CGG GAG ATG CAG CGC GAG ATC CTC TCC ATT TTG GGC TTG CCC CAC CGC      249
   Arg Glu Met Gln Arg Glu Ile Leu Ser Ile Leu Gly Leu Pro His Arg
               55                  60                  65
   CCG CGC CCG CAC CTC CAG GGC AAG CAC AAC TCG GCA CCC ATG TTC ATG      297
   Pro Arg Pro His Leu Gln Gly Lys His Asn Ser Ala Pro Met Phe Met
           70                  75                  80
   CTG GAC CTG TAC AAC GCC ATG GCG GTG GAG GAG GGC GGC GGG CCC GGC      345
   Leu Asp Leu Tyr Asn Ala Met Ala Val Glu Glu Gly Gly Gly Pro Gly
        85                  90                  95
   GGC CAG GGC TTC TCC TAC CCC TAC AAG GCC GTC TTC AGT ACC CAG GGC      393
   Gly Gln Gly Phe Ser Tyr Pro Tyr Lys Ala Val Phe Ser Thr Gln Gly
   100                 105                 110                 115
   CCC CCT CTG GCC AGC CTG CAA GAT AGC CAT TTC CTC ACC GAC GCC GAC      441
   Pro Pro Leu Ala Ser Leu Gln Asp Ser His Phe Leu Thr Asp Ala Asp
                   120                 125                 130
   ATG GTC ATG AGC TTC GTC AAC CTC GTG GAA CAT GAC AAG GAA TTC TTC      489
   Met Val Met Ser Phe Val Asn Leu Val Glu His Asp Lys Glu Phe Phe
               135                 140                 145
   CAC CCA CGC TAC CAC CAT CGA GAG TTC CGG TTT GAT CTT TCC AAG ATC      537
   His Pro Arg Tyr His His Arg Glu Phe Arg Phe Asp Leu Ser Lys Ile
           150                 155                 160
   CCA GAA GGG GAA GCT GTC ACG GCA GCC GAA TTC CGG ATC TAC AAG GAC      585
   Pro Glu Gly Glu Ala Val Thr Ala Ala Glu Phe Arg Ile Tyr Lys Asp
       165                 170                 175
   TAC ATC CGG GAA CGC TTC GAC AAT GAG ACG TTC CGG ATC AGC GTT TAT      633
   Tyr Ile Arg Glu Arg Phe Asp Asn Glu Thr Phe Arg Ile Ser Val Tyr
   180                 185                 190                 195
   CAG GTG CTC CAG GAG CAC TTG GGC AGG GAA TCG GAT CTC TTC CTG CTC      681
   Gln Val Leu Gln Glu His Leu Gly Arg Glu Ser Asp Leu Phe Leu Leu
                   200                 205                 210
   GAC AGC CGT ACC CTC TGG GCC TCG GAG GAG GGC TGG CTG GTG TTT GAC        729
   Asp Ser Arg Thr Leu Trp Ala Ser Glu Glu Gly Trp Leu Val Phe Asp
               215                 220                 225
   ATC ACA GCC ACC AGC AAC CAC TGG GTG GTC AAT CCG CGG CAC AAC CTG        777
   Ile Thr Ala Thr Ser Asn His Trp Val Val Asn Pro Arg His Asn Leu
           230                 235                 240
   GGC CTG CAG CTC TCG GTG GAG ACG CTG GAT GGG CAG AGC ATC AAC CCC        825
   Gly Leu Gln Leu Ser Val Glu Thr Leu Asp Gly Gln Ser Ile Asn Pro
       245                 250                 255
   AAG TTG GCG GGC CTG ATT GGG CGG CAC GGG CCC CAG AAC AAG CAG CCC        873
   Lys Leu Ala Gly Leu Ile Gly Arg His Gly Pro Gln Asn Lys Gln Pro
   260                 265                 270                 275
   TTC ATG GTG GCT TTC TTC AAG GCC ACG GAG GTC CAC TTC CGC AGC ATC        921
   Phe Met Val Ala Phe Phe Lys Ala Thr Glu Val His Phe Arg Ser Ile
                   280                 285                 290
   CGG TCC ACG GGG AGC AAA CAG CGC AGC CAG AAC CGC TCC AAG ACG CCC        969
   Arg Ser Thr Gly Ser Lys Gln Arg Ser Gln Asn Arg Ser Lys Thr Pro
               295                 300                 305
   AAG AAC CAG GAA GCC CTG CGG ATG GCC AAC GTG GCA GAG AAC AGC AGC       1017
   Lys Asn Gln Glu Ala Leu Arg Met Ala Asn Val Ala Glu Asn Ser Ser
           310                 315                 320
   AGC GAC CAG AGG CAG GCC TGT AAG AAG CAC GAG CTG TAT GTC AGC TTC       1065
   Ser Asp Gln Arg Gln Ala Cys Lys Lys His Glu Leu Tyr Val Ser Phe
       325                 330                 335
   CGA GAC CTG GGC TGG CAG GAC TGG ATC ATC GCG CCT GAA GGC TAC GCC       1113
   Arg Asp Leu Gly Trp Gln Asp Trp Ile Ile Ala Pro Glu Gly Tyr Ala
   340                 345                 350                 355
   GCC TAC TAC TGT GAG GGG GAG TGT GCC TTC CCT CTG AAC TCC TAC ATG       1161
   Ala Tyr Tyr Cys Glu Gly Glu Cys Ala Phe Pro Leu Asn Ser Tyr Met
                   360                 365                 370
   AAC GCC ACC AAC CAC GCC ATC GTG CAG ACG CTG GTC CAC TTC ATC AAC       1209
   Asn Ala Thr Asn His Ala Ile Val Gln Thr Leu Val His Phe Ile Asn
               375                 380                 385
   CCG GAA ACG GTG CCC AAG CCC TGC TGT GCG CCC ACG CAG CTC AAT GCC       1257
   Pro Glu Thr Val Pro Lys Pro Cys Cys Ala Pro Thr Gln Leu Asn Ala
           390                 395                 400
   ATC TCC GTC CTC TAC TTC GAT GAC AGC TCC AAC GTC ATC CTG AAG AAA       1305
   Ile Ser Val Leu Tyr Phe Asp Asp Ser Ser Asn Val Ile Leu Lys Lys
       405                 410                 415
   TAC AGA AAC ATG GTG GTC CGG GCC TGT GGC TGC CAC TAGCTCCTCC            1351
   Tyr Arg Asn Met Val Val Arg Ala Cys Gly Cys His
   420                 425                 430
   GAGAATTCAG ACCCTTTGGG GCCAAGTTTT TCTGGATCCT CCATTGCTCG CCTTGGCCAG     1411
   GAACCAGCAG ACCAACTGCC TTTTGTGAGA CCTTCCCCTC CCTATCCCCA ACTTTAAAGG     1471
   TGTGAGAGTA TTAGGAAACA TGAGCAGCAT ATGGCTTTTG ATCAGTTTTT CAGTGGCAGC     1531
   ATCCAATGAA CAAGATCCTA CAAGCTGTGC AGGCAAAACC TAGCAGGAAA AAAAAACAAC    1591
   GCATAAAGAA AAATGGCCGG GCCAGGTCAT TGGCTGGGAA GTCTCAGCCA TGCACGGACT    1651
   CGTTTCCAGA GGTAATTATG AGCGCCTACC AGCCAGGCCA CCCAGCCGTG GGAGGAAGGG    1711
   GGCGTGGCAA GGGGTGGGCA CATTGGTGTC TGTGCGAAAG GAAAATTGAC CCGGAAGTTC    1771
   CTGTAATAAA TGTCACAATA AAACGAATGA ATGAAAAAAA AAAAAAAAAA A             1822
(2) INFORMATION FOR SEQ ID NO:16:
   (i) SEQUENCE CHARACTERISTICS:
      (A) LENGTH: 431 amino acids
      (B) TYPE: amino acid
      (D) TOPOLOGY: linear
   (ii) MOLECULE TYPE: protein
   (xi) SEQUENCE DESCRIPTION: SEQ ID NO:16:
   Met His Val Arg Ser Leu Arg Ala Ala Ala Pro His Ser Phe Val Ala
    1               5                  10                  15
   Leu Trp Ala Pro Leu Phe Leu Leu Arg Ser Ala Leu Ala Asp Phe Ser
               20                  25                  30
   Leu Asp Asn Glu Val His Ser Ser Phe Ile His Arg Arg Leu Arg Ser
           35                  40                  45
   Gln Glu Arg Arg Glu Met Gln Arg Glu Ile Leu Ser Ile Leu Gly Leu
       50                  55                  60
   Pro His Arg Pro Arg Pro His Leu Gln Gly Lys His Asn Ser Ala Pro
   65                  70                  75                  80
   Met Phe Met Leu Asp Leu Tyr Asn Ala Met Ala Val Glu Glu Gly Gly
                   85                  90                  95
   Gly Pro Gly Gly Gln Gly Phe Ser Tyr Pro Tyr Lys Ala Val Phe Ser
               100                 105                 110
   Thr Gln Gly Pro Pro Leu Ala Ser Leu Gln Asp Ser His Phe Leu Thr
           115                 120                 125
   Asp Ala Asp Met Val Met Ser Phe Val Asn Leu Val Glu His Asp Lys
       130                 135                 140
   Glu Phe Phe His Pro Arg Tyr His His Arg Glu Phe Arg Phe Asp Leu
   145                 150                 155                 160
   Ser Lys Ile Pro Glu Gly Glu Ala Val Thr Ala Ala Glu Phe Arg Ile
                   165                 170                 175
   Tyr Lys Asp Tyr Ile Arg Glu Arg Phe Asp Asn Glu Thr Phe Arg Ile
               180                 185                 190
   Ser Val Tyr Gln Val Leu Gln Glu His Leu Gly Arg Glu Ser Asp Leu
           195                 200                 205
   Phe Leu Leu Asp Ser Arg Thr Leu Trp Ala Ser Glu Glu Gly Trp Leu
       210                 215                 220
   Val Phe Asp Ile Thr Ala Thr Ser Asn His Trp Val Val Asn Pro Arg
   225                 230                 235                 240
   His Asn Leu Gly Leu Gln Leu Ser Val Glu Thr Leu Asp Gly Gln Ser
                   245                 250                 255
   Ile Asn Pro Lys Leu Ala Gly Leu Ile Gly Arg His Gly Pro Gln Asn
               260                 265                 270
   Lys Gln Pro Phe Met Val Ala Phe Phe Lys Ala Thr Glu Val His Phe
           275                 280                 285
   Arg Ser Ile Arg Ser Thr Gly Ser Lys Gln Arg Ser Gln Asn Arg Ser
       290                 295                 300
   Lys Thr Pro Lys Asn Gln Glu Ala Leu Arg Met Ala Asn Val Ala Glu
   305                 310                 315                 320
   Asn Ser Ser Ser Asp Gln Arg Gln Ala Cys Lys Lys His Glu Leu Tyr
                   325                 330                 335
   Val Ser Phe Arg Asp Leu Gly Trp Gln Asp Trp Ile Ile Ala Pro Glu
               340                 345                 350
   Gly Tyr Ala Ala Tyr Tyr Cys Glu Gly Glu Cys Ala Phe Pro Leu Asn
           355                 360                 365
   Ser Tyr Met Asn Ala Thr Asn His Ala Ile Val Gln Thr Leu Val His
       370                 375                 380
   Phe Ile Asn Pro Glu Thr Val Pro Lys Pro Cys Cys Ala Pro Thr Gln
   385                 390                 395                 400
   Leu Asn Ala Ile Ser Val Leu Tyr Phe Asp Asp Ser Ser Asn Val Ile
                   405                 410                 415
   Leu Lys Lys Tyr Arg Asn Met Val Val Arg Ala Cys Gly Cys His
               420                 425                 430
(2) INFORMATION FOR SEQ ID NO:17:
   (i) SEQUENCE CHARACTERISTICS:
      (A) LENGTH: 1873 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: MURIDAE
      (F) TISSUE TYPE: EMBRYO
   (ix) FEATURE:
      (A) NAME/KEY: CDS
      (B) LOCATION: 104..1393
      (D) OTHER INFORMATION: /function= "OSTEOGENIC PROTEIN"
         /product= "MOP1"
         /note= "MOP1 (CDNA)"
   (xi) SEQUENCE DESCRIPTION: SEQ ID NO:17:
   CTGCAGCAAG TGACCTCGGG TCGTGGACCG CTGCCCTGCC CCCTCCGCTG CCACCTGGGG      60
   CGGCGCGGGC CCGGTGCCCC GGATCGCGCG TAGAGCCGGC GCG ATG CAC GTG CGC      115
                                                   Met His Val Arg
                                                     1
   TCG CTG CGC GCT GCG GCG CCA CAC AGC TTC GTG GCG CTC TGG GCG CCT      163
   Ser Leu Arg Ala Ala Ala Pro His Ser Phe Val Ala Leu Trp Ala Pro
     5                  10                  15                  20
   CTG TTC TTG CTG CGC TCC GCC CTG GCC GAT TTC AGC CTG GAC AAC GAG      211
   Leu Phe Leu Leu Arg Ser Ala Leu Ala Asp Phe Ser Leu Asp Asn Glu
                    25                  30                  35
   GTG CAC TCC AGC TTC ATC CAC CGG CGC CTC CGC AGC CAG GAG CGG CGG      259
   Val His Ser Ser Phe Ile His Arg Arg Leu Arg Ser Gln Glu Arg Arg
                40                  45                  50
   GAG ATG CAG CGG GAG ATC CTG TCC ATC TTA GGG TTG CCC CAT CGC CCG      307
   Glu Met Gln Arg Glu Ile Leu Ser Ile Leu Gly Leu Pro His Arg Pro
            55                  60                  65
   CGC CCG CAC CTC CAG GGA AAG CAT AAT TCG GCG CCC ATG TTC ATG TTG      355
   Arg Pro His Leu Gln Gly Lys His Asn Ser Ala Pro Met Phe Met Leu
        70                  75                  80
   GAC CTG TAC AAC GCC ATG GCG GTG GAG GAG AGC GGG CCG GAC GGA CAG      403
   Asp Leu Tyr Asn Ala Met Ala Val Glu Glu Ser Gly Pro Asp Gly Gln
    85                  90                  95                 100
   GGC TTC TCC TAC CCC TAC AAG GCC GTC TTC AGT ACC CAG GGC CCC CCT      451
   Gly Phe Ser Tyr Pro Tyr Lys Ala Val Phe Ser Thr Gln Gly Pro Pro
                   105                 110                 115
   TTA GCC AGC CTG CAG GAC AGC CAT TTC CTC ACT GAC GCC GAC ATG GTC      499
   Leu Ala Ser Leu Gln Asp Ser His Phe Leu Thr Asp Ala Asp Met Val
               120                 125                 130
   ATG AGC TTC GTC AAC CTA GTG GAA CAT GAC AAA GAA TTC TTC CAC CCT      547
   Met Ser Phe Val Asn Leu Val Glu His Asp Lys Glu Phe Phe His Pro
           135                 140                 145
   CGA TAC CAC CAT CGG GAG TTC CGG TTT GAT CTT TCC AAG ATC CCC GAG      595
   Arg Tyr His His Arg Glu Phe Arg Phe Asp Leu Ser Lys Ile Pro Glu
       150                 155                 160
   GGC GAA CGG GTG ACC GCA GCC GAA TTC AGG ATC TAT AAG GAC TAC ATC      643
   Gly Glu Arg Val Thr Ala Ala Glu Phe Arg Ile Tyr Lys Asp Tyr Ile
   165                 170                 175                 180
   CGG GAG CGA TTT GAC AAC GAG ACC TTC CAG ATC ACA GTC TAT CAG GTG      691
   Arg Glu Arg Phe Asp Asn Glu Thr Phe Gln Ile Thr Val Tyr Gln Val
                   185                 190                 195
   CTC CAG GAG CAC TCA GGC AGG GAG TCG GAC CTC TTC TTG CTG GAC AGC      739
   Leu Gln Glu His Ser Gly Arg Glu Ser Asp Leu Phe Leu Leu Asp Ser
               200                 205                 210
   CGC ACC ATC TGG GCT TCT GAG GAG GGC TGG TTG GTG TTT GAT ATC ACA        787
   Arg Thr Ile Trp Ala Ser Glu Glu Gly Trp Leu Val Phe Asp Ile Thr
           215                 220                 225
   GCC ACC AGC AAC CAC TGG GTG GTC AAC CCT CGG CAC AAC CTG GGC TTA        835
   Ala Thr Ser Asn His Trp Val Val Asn Pro Arg His Asn Leu Gly Leu
       230                 235                 240
   CAG CTC TCT GTG GAG ACC CTG GAT GGG CAG AGC ATC AAC CCC AAG TTG        883
   Gln Leu Ser Val Glu Thr Leu Asp Gly Gln Ser Ile Asn Pro Lys Leu
   245                 250                 255                 260
   GCA GGC CTG ATT GGA CGG CAT GGA CCC CAG AAC AAG CAA CCC TTC ATG        931
   Ala Gly Leu Ile Gly Arg His Gly Pro Gln Asn Lys Gln Pro Phe Met
                   265                 270                 275
   GTG GCC TTC TTC AAG GCC ACG GAA GTC CAT CTC CGT AGT ATC CGG TCC        979
   Val Ala Phe Phe Lys Ala Thr Glu Val His Leu Arg Ser Ile Arg Ser
               280                 285                 290
   ACG GGG GGC AAG CAG CGC AGC CAG AAT CGC TCC AAG ACG CCA AAG AAC       1027
   Thr Gly Gly Lys Gln Arg Ser Gln Asn Arg Ser Lys Thr Pro Lys Asn
           295                 300                 305
   CAA GAG GCC CTG AGG ATG GCC AGT GTG GCA GAA AAC AGC AGC AGT GAC       1075
   Gln Glu Ala Leu Arg Met Ala Ser Val Ala Glu Asn Ser Ser Ser Asp
       310                 315                 320
   CAG AGG CAG GCC TGC AAG AAA CAT GAG CTG TAC GTC AGC TTC CGA GAC       1123
   Gln Arg Gln Ala Cys Lys Lys His Glu Leu Tyr Val Ser Phe Arg Asp
   325                 330                 335                 340
   CTT GGC TGG CAG GAC TGG ATC ATT GCA CCT GAA GGC TAT GCT GCC TAC       1171
   Leu Gly Trp Gln Asp Trp Ile Ile Ala Pro Glu Gly Tyr Ala Ala Tyr
                   345                 350                 355
   TAC TGT GAG GGA GAG TGC GCC TTC CCT CTG AAC TCC TAC ATG AAC GCC       1219
   Tyr Cys Glu Gly Glu Cys Ala Phe Pro Leu Asn Ser Tyr Met Asn Ala
               360                 365                 370
   ACC AAC CAC GCC ATC GTC CAG ACA CTG GTT CAC TTC ATC AAC CCA GAC       1267
   Thr Asn His Ala Ile Val Gln Thr Leu Val His Phe Ile Asn Pro Asp
           375                 380                 385
   ACA GTA CCC AAG CCC TGC TGT GCG CCC ACC CAG CTC AAC GCC ATC TCT       1315
   Thr Val Pro Lys Pro Cys Cys Ala Pro Thr Gln Leu Asn Ala Ile Ser
       390                 395                 400
   GTC CTC TAC TTC GAC GAC AGC TCT AAT GTC ATC CTG AAG AAG TAC AGA       1363
   Val Leu Tyr Phe Asp Asp Ser Ser Asn Val Ile Leu Lys Lys Tyr Arg
   405                 410                 415                 420
   AAC ATG GTG GTC CGG GCC TGT GGC TGC CAC TAGCTCTTCC TGAGACCCTG         1413
   Asn Met Val Val Arg Ala Cys Gly Cys His
                   425                 430
   ACCTTTGCGG GGCCACACCT TTCCAAATCT TCGATGTCTC ACCATCTAAG TCTCTCACTG     1473
   CCCACCTTGG CGAGGAGAAC AGACCAACCT CTCCTGAGCC TTCCCTCACC TCCCAACCGG     1533
   AAGCATGTAA GGGTTCCAGA AACCTGAGCG TGCAGCAGCT GATGAGCGCC CTTTCCTTCT     1593
   GGCACGTGAC GGACAAGATC CTACCAGCTA CCACAGCAAA CGCCTAAGAG CAGGAAAAAT     1653
   GTCTGCCAGG AAAGTGTCCA GTGTCCACAT GGCCCCTGGC GCTCTGAGTC TTTGAGGAGT 1713
   AATCGCAAGC CTCGTTCAGC TGCAGCAGAA GGAAGGGCTT AGCCAGGGTG GGCGCTGGCG 1773
   TCTGTGTTGA AGGGAAACCA AGCAGAAGCC ACTGTAATGA TATGTCACAA TAAAACCCAT 1833
   GAATGAAAAA AAAAAAAAAA AAAAAAAAAA AAAAGAATTC 1873
(2) INFORMATION FOR SEQ ID NO:18:
   (i) SEQUENCE CHARACTERISTICS:
      (A) LENGTH: 430 amino acids
      (B) TYPE: amino acid
      (D) TOPOLOGY: linear
   (ii) MOLECULE TYPE: protein
   (xi) SEQUENCE DESCRIPTION: SEQ ID NO:18:
   Met His Val Arg Ser Leu Arg Ala Ala Ala Pro His Ser Phe Val Ala
    1               5                  10                  15
   Leu Trp Ala Pro Leu Phe Leu Leu Arg Ser Ala Leu Ala Asp Phe Ser
               20                  25                  30
   Leu Asp Asn Glu Val His Ser Ser Phe Ile His Arg Arg Leu Arg Ser
           35                  40                  45
   Gln Glu Arg Arg Glu Met Gln Arg Glu Ile Leu Ser Ile Leu Gly Leu
       50                  55                  60
   Pro His Arg Pro Arg Pro His Leu Gln Gly Lys His Asn Ser Ala Pro
   65                  70                  75                  80
   Met Phe Met Leu Asp Leu Tyr Asn Ala Met Ala Val Glu Glu Ser Gly
                   85                  90                  95
   Pro Asp Gly Gln Gly Phe Ser Tyr Pro Tyr Lys Ala Val Phe Ser Thr
               100                 105                 110
   Gln Gly Pro Pro Leu Ala Ser Leu Gln Asp Ser His Phe Leu Thr Asp
           115                 120                 125
   Ala Asp Met Val Met Ser Phe Val Asn Leu Val Glu His Asp Lys Glu
       130                 135                 140
   Phe Phe His Pro Arg Tyr His His Arg Glu Phe Arg Phe Asp Leu Ser
   145                 150                 155                 160
   Lys Ile Pro Glu Gly Glu Arg Val Thr Ala Ala Glu Phe Arg Ile Tyr
                   165                 170                 175
   Lys Asp Tyr Ile Arg Glu Arg Phe Asp Asn Glu Thr Phe Gln Ile Thr
               180                 185                 190
   Val Tyr Gln Val Leu Gln Glu His Ser Gly Arg Glu Ser Asp Leu Phe
           195                 200                 205
   Leu Leu Asp Ser Arg Thr Ile Trp Ala Ser Glu Glu Gly Trp Leu Val
       210                 215                 220
   Phe Asp Ile Thr Ala Thr Ser Asn His Trp Val Val Asn Pro Arg His
   225                 230                 235                 240
   Asn Leu Gly Leu Gln Leu Ser Val Glu Thr Leu Asp Gly Gln Ser Ile
                   245                 250                 255
   Asn Pro Lys Leu Ala Gly Leu Ile Gly Arg His Gly Pro Gln Asn Lys
               260                 265                 270
   Gln Pro Phe Met Val Ala Phe Phe Lys Ala Thr Glu Val His Leu Arg
           275                 280                 285
   Ser Ile Arg Ser Thr Gly Gly Lys Gln Arg Ser Gln Asn Arg Ser Lys
       290                 295                 300
   Thr Pro Lys Asn Gln Glu Ala Leu Arg Met Ala Ser Val Ala Glu Asn
   305                 310                 315                 320
   Ser Ser Ser Asp Gln Arg Gln Ala Cys Lys Lys His Glu Leu Tyr Val
                   325                 330                 335
   Ser Phe Arg Asp Leu Gly Trp Gln Asp Trp Ile Ile Ala Pro Glu Gly
               340                 345                 350
   Tyr Ala Ala Tyr Tyr Cys Glu Gly Glu Cys Ala Phe Pro Leu Asn Ser
           355                 360                 365
   Tyr Met Asn Ala Thr Asn His Ala Ile Val Gln Thr Leu Val His Phe
       370                 375                 380
   Ile Asn Pro Asp Thr Val Pro Lys Pro Cys Cys Ala Pro Thr Gln Leu
   385                 390                 395                 400
   Asn Ala Ile Ser Val Leu Tyr Phe Asp Asp Ser Ser Asn Val Ile Leu
                   405                 410                 415
   Lys Lys Tyr Arg Asn Met Val Val Arg Ala Cys Gly Cys His
               420                 425                 430
(2) INFORMATION FOR SEQ ID NO:19:
   (i) SEQUENCE CHARACTERISTICS:
      (A) LENGTH: 1723 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: HOMO SAPIENS
      (F) TISSUE TYPE: HIPPOCAMPUS
   (ix) FEATURE:
      (A) NAME/KEY: CDS
      (B) LOCATION: 490..1695
      (D) OTHER INFORMATION: /function= "OSTEOGENIC PROTEIN"
         /product= "hOP2-PP"
         /note= "hOP2 (cDNA)"
   (xi) SEQUENCE DESCRIPTION: SEQ ID NO:19:
   GGCGCCGGCA GAGCAGGAGT GGCTGGAGGA GCTGTGGTTG GAGCAGGAGG TGGCACGGCA     60
   GGGCTGGAGG GCTCCCTATG AGTGGCGGAG ACGGCCCAGG AGGCGCTGGA GCAACAGCTC    120
   CCACACCGCA CCAAGCGGTG GCTGCAGGAG CTCGCCCATC GCCCCTGCGC TGCTCGGACC    180
   GCGGCCACAG CCGGACTGGC GGGTACGGCG GCGACAGAGG CATTGGCCGA GAGTCCCAGT    240
   CCGCAGAGTA GCCCCGGCCT CGAGGCGGTG GCGTCCCGGT CCTCTCCGTC CAGGAGCCAG    300
   GACAGGTGTC GCGCGGCGGG GCTCCAGGGA CCGCGCCTGA GGCCGGCTGC CCGCCCGTCC    360
   CGCCCCGCCC CGCCGCCCGC CGCCCGCCGA GCCCAGCCTC CTTGCCGTCG GGGCGTCCCC     420
   AGGCCCTGGG TCGGCCGCGG AGCCGATGCG CGCCCGCTGA GCGCCCCAGC TGAGCGCCCC    480
   CGGCCTGCC ATG ACC GCG CTC CCC GGC CCG CTC TGG CTC CTG GGC CTG        528
   GCG CTA TGC GCG CTG GGC GGG GGC GGC CCC GGC CTG CGA CCC CCG CCC      576
   GGC TGT CCC CAG CGA CGT CTG GGC GCG CGC GAG CGC CGG GAC GTG CAG      624
   CGC GAG ATC CTG GCG GTG CTC GGG CTG CCT GGG CGG CCC CGG CCC CGC      672
   GCG CCA CCC GCC GCC TCC CGG CTG CCC GCG TCC GCG CCG CTC TTC ATG      720
   CTG GAC CTG TAC CAC GCC ATG GCC GGC GAC GAC GAC GAG GAC GGC GCG      768
   CCC GCG GAG CGG CGC CTG GGC CGC GCC GAC CTG GTC ATG AGC TTC GTT      816
   AAC ATG GTG GAG CGA GAC CGT GCC CTG GGC CAC CAG GAG CCC CAT TGG      864
   AAG GAG TTC CGC TTT GAC CTG ACC CAG ATC CCG GCT GGG GAG GCG GTC      912
   ACA GCT GCG GAG TTC CGG ATT TAC AAG GTG CCC AGC ATC CAC CTG CTC      960
   AAC AGG ACC CTC CAC GTC AGC ATG TTC CAG GTG GTC CAG GAG CAG TCC     1008
   AAC AGG GAG TCT GAC TTG TTC TTT TTG GAT CTT CAG ACG CTC CGA GCT      1056
   GGA GAC GAG GGC TGG CTG GTG CTG GAT GTC ACA GCA GCC AGT GAC TGC      1104
   TGG TTG CTG AAG CGT CAC AAG GAC CTG GGA CTC CGC CTC TAT GTG GAG      1152
   ACT GAG GAC GGG CAC AGC GTG GAT CCT GGC CTG GCC GGC CTG CTG GGT      1200
   CAA CGG GCC CCA CGC TCC CAA CAG CCT TTC GTG GTC ACT TTC TTC AGG      1248
   GCC AGT CCG AGT CCC ATC CGC ACC CCT CGG GCA GTG AGG CCA CTG AGG      1296
   AGG AGG CAG CCG AAG AAA AGC AAC GAG CTG CCG CAG GCC AAC CGA CTC      1344
   CCA GGG ATC TTT GAT GAC GTC CAC GGC TCC CAC GGC CGG CAG GTC TGC      1392
   CGT CGG CAC GAG CTC TAC GTC AGC TTC CAG GAC CTC GGC TGG CTG GAC      1440
   TGG GTC ATC GCT CCC CAA GGC TAC TCG GCC TAT TAC TGT GAG GGG GAG      1488
   TGC TCC TTC CCA CTG GAC TCC TGC ATG AAT GCC ACC AAC CAC GCC ATC      1536
   CTG CAG TCC CTG GTG CAC CTG ATG AAG CCA AAC GCA GTC CCC AAG GCG      1584
   TGC TGT GCA CCC ACC AAG CTG AGC GCC ACC TCT GTG CTC TAC TAT GAC      1632
   AGC AGC AAC AAC GTC ATC CTG CGC AAA CAC CGC AAC ATG GTG GTC AAG      1680
   GCC TGC GGC TGC CAC TGAGTCAGCC CGCCCAGCCC TACTGCAG                   1723
(2) INFORMATION FOR SEQ ID NO:20:
   (i) SEQUENCE CHARACTERISTICS:
      (A) LENGTH: 402 amino acids
      (B) TYPE: amino acid
      (D) TOPOLOGY: linear
   (ii) MOLECULE TYPE: protein
   (xi) SEQUENCE DESCRIPTION: SEQ ID NO:20:
(2) INFORMATION FOR SEQ ID NO:21:
   (i) SEQUENCE CHARACTERISTICS:
      (A) LENGTH: 1926 base pairs
      (B) TYPE: nucleic acid
      (C) STRANDEDNESS: single
      (D) TOPOLOGY: linear
   (vi) ORIGINAL SOURCE:
      (A) ORGANISM: MURIDAE
      (F) TISSUE TYPE: EMBRYO
   (ix) FEATURE:
      (A) NAME/KEY: CDS
      (B) LOCATION: 93..1289
      (D) OTHER INFORMATION: /function= "OSTEOGENIC PROTEIN"
         /product= "mOP2-PP"
         /note= "mOP2 cDNA"
   (xi) SEQUENCE DESCRIPTION: SEQ ID NO:21:
   GCCAGGCACA GGTGCGCCGT CTGGTCCTCC CCGTCTGGCG TCAGCCGAGC CCGACCAGCT      60
   ACCAGTGGAT GCGCGCCGGC TGAAAGTCCG AG ATG GCT ATG CGT CCC GGG CCA       113
                                       Met Ala Met Arg Pro Gly Pro
                                        1               5
   CTC TGG CTA TTG GGC CTT GCT CTG TGC GCG CTG GGA GGC GGC CAC GGT       161
   Leu Trp Leu Leu Gly Leu Ala Leu Cys Ala Leu Gly Gly Gly His Gly
            10                  15                  20
   CCG CGT CCC CCG CAC ACC TGT CCC CAG CGT CGC CTG GGA GCG CGC GAG       209
   Pro Arg Pro Pro His Thr Cys Pro Gln Arg Arg Leu Gly Ala Arg Glu
        25                  30                  35
   CGC CGC GAC ATG CAG CGT GAA ATC CTG GCG GTG CTC GGG CTA CCG GGA      257
   Arg Arg Asp Met Gln Arg Glu Ile Leu Ala Val Leu Gly Leu Pro Gly
    40                  45                  50                  55
   CGG CCC CGA CCC CGT GCA CAA CCC GCC GCT GCC CGG CAG CCA GCG TCC      305
   Arg Pro Arg Pro Arg Ala Gln Pro Ala Ala Ala Arg Gln Pro Ala Ser
                    60                  65                  70
   GCG CCC CTC TTC ATG TTG GAC CTA TAC CAC GCC ATG ACC GAT GAC GAC      353
   Ala Pro Leu Phe Met Leu Asp Leu Tyr His Ala Met Thr Asp Asp Asp
                75                  80                  85
   GAC GGC GGG CCA CCA CAG GCT CAC TTA GGC CGT GCC GAC CTG GTC ATG      401
   Asp Gly Gly Pro Pro Gln Ala His Leu Gly Arg Ala Asp Leu Val Met
            90                  95                 100
   AGC TTC GTC AAC ATG GTG GAA CGC GAC CGT ACC CTG GGC TAC CAG GAG      449
   Ser Phe Val Asn Met Val Glu Arg Asp Arg Thr Leu Gly Tyr Gln Glu
       105                 110                 115
   CCA CAC TGG AAG GAA TTC CAC TTT GAC CTA ACC CAG ATC CCT GCT GGG      497
   Pro His Trp Lys Glu Phe His Phe Asp Leu Thr Gln Ile Pro Ala Gly
   120                 125                 130                 135
   GAG GCT GTC ACA GCT GCT GAG TTC CGG ATC TAC AAA GAA CCC AGC ACC      545
   Glu Ala Val Thr Ala Ala Glu Phe Arg Ile Tyr Lys Glu Pro Ser Thr
                   140                 145                 150
   CAC CCG CTC AAC ACA ACC CTC CAC ATC AGC ATG TTC GAA GTG GTC CAA      593
   His Pro Leu Asn Thr Thr Leu His Ile Ser Met Phe Glu Val Val Gln
               155                 160                 165
   GAG CAC TCC AAC AGG GAG TCT GAC TTG TTC TTT TTG GAT CTT CAG ACG      641
   Glu His Ser Asn Arg Glu Ser Asp Leu Phe Phe Leu Asp Leu Gln Thr
           170                 175                 180
   CTC CGA TCT GGG GAC GAG GGC TGG CTG GTG CTG GAC ATC ACA GCA GCC      689
   Leu Arg Ser Gly Asp Glu Gly Trp Leu Val Leu Asp Ile Thr Ala Ala
       185                 190                 195
   AGT GAC CGA TGG CTG CTG AAC CAT CAC AAG GAC CTG GGA CTC CGC CTC      737
   Ser Asp Arg Trp Leu Leu Asn His His Lys Asp Leu Gly Leu Arg Leu
   200                 205                 210                 215
   TAT GTG GAA ACC GCG GAT GGG CAC AGC ATG GAT CCT GGC CTG GCT GGT      785
   Tyr Val Glu Thr Ala Asp Gly His Ser Met Asp Pro Gly Leu Ala Gly
                   220                 225                 230
   CTG CTT GGA CGA CAA GCA CCA CGC TCC AGA CAG CCT TTC ATG GTA ACC      833
   Leu Leu Gly Arg Gln Ala Pro Arg Ser Arg Gln Pro Phe Met Val Thr
               235                 240                 245
   TTC TTC AGG GCC AGC CAG AGT CCT GTG CGG GCC CCT CGG GCA GCG AGA      881
   Phe Phe Arg Ala Ser Gln Ser Pro Val Arg Ala Pro Arg Ala Ala Arg
           250                 255                 260
   CCA CTG AAG AGG AGG CAG CCA AAG AAA ACG AAC GAG CTT CCG CAC CCC      929
   Pro Leu Lys Arg Arg Gln Pro Lys Lys Thr Asn Glu Leu Pro His Pro
       265                 270                 275
   AAC AAA CTC CCA GGG ATC TTT GAT GAT GGC CAC GGT TCC CGC GGC AGA        977
   Asn Lys Leu Pro Gly Ile Phe Asp Asp Gly His Gly Ser Arg Gly Arg
   280                 285                 290                 295
   GAG GTT TGC CGC AGG CAT GAG CTC TAC GTC AGC TTC CGT GAC CTT GGC       1025
   Glu Val Cys Arg Arg His Glu Leu Tyr Val Ser Phe Arg Asp Leu Gly
                   300                 305                 310
   TGG CTG GAC TGG GTC ATC GCC CCC CAG GGC TAC TCT GCC TAT TAC TGT       1073
   Trp Leu Asp Trp Val Ile Ala Pro Gln Gly Tyr Ser Ala Tyr Tyr Cys
               315                 320                 325
   GAG GGG GAG TGT GCT TTC CCA CTG GAC TCC TGT ATG AAC GCC ACC AAC       1121
   Glu Gly Glu Cys Ala Phe Pro Leu Asp Ser Cys Met Asn Ala Thr Asn
           330                 335                 340
   CAT GCC ATC TTG CAG TCT CTG GTG CAC CTG ATG AAG CCA GAT GTT GTC       1169
   His Ala Ile Leu Gln Ser Leu Val His Leu Met Lys Pro Asp Val Val
       345                 350                 355
   CCC AAG GCA TGC TGT GCA CCC ACC AAA CTG AGT GCC ACC TCT GTG CTG       1217
   Pro Lys Ala Cys Cys Ala Pro Thr Lys Leu Ser Ala Thr Ser Val Leu
   360                 365                 370                 375
   TAC TAT GAC AGC AGC AAC AAT GTC ATC CTG CGT AAA CAC CGT AAC ATG       1265
   Tyr Tyr Asp Ser Ser Asn Asn Val Ile Leu Arg Lys His Arg Asn Met
                   380                 385                 390
   GTG GTC AAG GCC TGT GGC TGC CAC TGAGGCCCCG CCCAGCATCC TGCTTCTACT      1319
   Val Val Lys Ala Cys Gly Cys His
               395
   ACCTTACCAT CTGGCCGGGC CCCTCTCCAG AGGCAGAAAC CCTTCTATGT TATCATAGCT     1379
   CAGACAGGGG CAATGGGAGG CCCTTCACTT CCCCTGGCCA CTTCCTGCTA AAATTCTGGT     1439
   CTTTCCCAGT TCCTCTGTCC TTCATGGGGT TTCGGGGCTA TCACCCCGCC CTCTCCATCC     1499
   TCCTACCCCA AGCATAGACT GAATGCACAC AGCATCCCAG AGCTATGCTA ACTGAGAGGT     1559
   CTGGGGTCAG CACTGAAGGC CCACATGAGG AAGACTGATC CTTGGCCATC CTCAGCCCAC     1619
   AATGGCAAAT TCTGGATGGT CTAAGAAGGC CCTGGAATTC TAAACTAGAT GATCTGGGCT     1679
   CTCTGCACCA TTCATTGTGG CAGTTGGGAC ATTTTTAGGT ATAACAGACA CATACACTTA     1739
   GATCAATGCA TCGCTGTACT CCTTGAAATC AGAGCTAGCT TGTTAGAAAA AGAATCAGAG     1799
   CCAGGTATAG CGGTGCATGT CATTAATCCC AGCGCTAAAG AGACAGAGAC AGGAGAATCT     1859
   CTGTGAGTTC AAGGCCACAT AGAAAGAGCC TGTCTCGGGA GCAGGAAAAA AAAAAAAAAC     1919
   GGAATTC                                                               1926
(2) INFORMATION FOR SEQ ID NO:22:
   (i) SEQUENCE CHARACTERISTICS:
      (A) LENGTH: 399 amino acids
      (B) TYPE: amino acid
      (D) TOPOLOGY: linear
   (ii) MOLECULE TYPE: protein
   (xi) SEQUENCE DESCRIPTION: SEQ ID NO:22:
   Met Ala Met Arg Pro Gly Pro Leu Trp Leu Leu Gly Leu Ala Leu Cys
    1               5                  10                  15
   Ala Leu Gly Gly Gly His Gly Pro Arg Pro Pro His Thr Cys Pro Gln
               20                  25                  30
   Arg Arg Leu Gly Ala Arg Glu Arg Arg Asp Met Gln Arg Glu Ile Leu
           35                  40                  45
   Ala Val Leu Gly Leu Pro Gly Arg Pro Arg Pro Arg Ala Gln Pro Ala
       50                  55                  60
   Ala Ala Arg Gln Pro Ala Ser Ala Pro Leu Phe Met Leu Asp Leu Tyr
   65                  70                  75                  80
   His Ala Met Thr Asp Asp Asp Asp Gly Gly Pro Pro Gln Ala His Leu
                   85                  90                  95
   Gly Arg Ala Asp Leu Val Met Ser Phe Val Asn Met Val Glu Arg Asp
               100                 105                 110
   Arg Thr Leu Gly Tyr Gln Glu Pro His Trp Lys Glu Phe His Phe Asp
           115                 120                 125
   Leu Thr Gln Ile Pro Ala Gly Glu Ala Val Thr Ala Ala Glu Phe Arg
       130                 135                 140
   Ile Tyr Lys Glu Pro Ser Thr His Pro Leu Asn Thr Thr Leu His Ile
   145                 150                 155                 160
   Ser Met Phe Glu Val Val Gln Glu His Ser Asn Arg Glu Ser Asp Leu
                   165                 170                 175
   Phe Phe Leu Asp Leu Gln Thr Leu Arg Ser Gly Asp Glu Gly Trp Leu
               180                 185                 190
   Val Leu Asp Ile Thr Ala Ala Ser Asp Arg Trp Leu Leu Asn His His
           195                 200                 205
   Lys Asp Leu Gly Leu Arg Leu Tyr Val Glu Thr Ala Asp Gly His Ser
       210                 215                 220
   Met Asp Pro Gly Leu Ala Gly Leu Leu Gly Arg Gln Ala Pro Arg Ser
   225                 230                 235                 240
   Arg Gln Pro Phe Met Val Thr Phe Phe Arg Ala Ser Gln Ser Pro Val
                   245                 250                 255
   Arg Ala Pro Arg Ala Ala Arg Pro Leu Lys Arg Arg Gln Pro Lys Lys
               260                 265                 270
   Thr Asn Glu Leu Pro His Pro Asn Lys Leu Pro Gly Ile Phe Asp Asp
           275                 280                 285
   Gly His Gly Ser Arg Gly Arg Glu Val Cys Arg Arg His Glu Leu Tyr
       290                 295                 300
   Val Ser Phe Arg Asp Leu Gly Trp Leu Asp Trp Val Ile Ala Pro Gln
   305                 310                 315                 320
   Gly Tyr Ser Ala Tyr Tyr Cys Glu Gly Glu Cys Ala Phe Pro Leu Asp
                   325                 330                 335
   Ser Cys Met Asn Ala Thr Asn His Ala Ile Leu Gln Ser Leu Val His
               340                 345                 350
   Leu Met Lys Pro Asp Val Val Pro Lys Ala Cys Cys Ala Pro Thr Lys
           355                 360                 365
   Leu Ser Ala Thr Ser Val Leu Tyr Tyr Asp Ser Ser Asn Asn Val Ile
       370                 375                 380
   Leu Arg Lys His Arg Asn Met Val Val Lys Ala Cys Gly Cys His
   385                 390                 395
(2) INFORMATION FOR SEQ ID NO:23:
   (i) SEQUENCE CHARACTERISTICS:
      (A) LENGTH: 1368 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..1365
      (D) OTHER INFORMATION: /label= 60A
   (xi) SEQUENCE DESCRIPTION: SEQ ID NO:23:
   ATG TCG GGA CTG CGA AAC ACC TCG GAG GCC GTT GCA GTG CTC GCC TCC       48
   Met Ser Gly Leu Arg Asn Thr Ser Glu Ala Val Ala Val Leu Ala Ser
    1               5                  10                  15
   CTG GGA CTC GGA ATG GTT CTG CTC ATG TTC GTG GCG ACC ACG CCG CCG       96
   Leu Gly Leu Gly Met Val Leu Leu Met Phe Val Ala Thr Thr Pro Pro
               20                  25                  30
   GCC GTT GAG GCC ACC CAG TCG GGG ATT TAC ATA GAC AAC GGC AAG GAC      144
   Ala Val Glu Ala Thr Gln Ser Gly Ile Tyr Ile Asp Asn Gly Lys Asp
           35                  40                  45
   CAG ACG ATC ATG CAC AGA GTG CTG AGC GAG GAC GAC AAG CTG GAC GTC      192
   Gln Thr Ile Met His Arg Val Leu Ser Glu Asp Asp Lys Leu Asp Val
       50                  55                  60
   TCG TAC GAG ATC CTC GAG TTC CTG GGC ATC GCC GAA CGG CCG ACG CAC      240
   Ser Tyr Glu Ile Leu Glu Phe Leu Gly Ile Ala Glu Arg Pro Thr His
   65                  70                  75                  80
   CTG AGC AGC CAC CAG TTG TCG CTG AGG AAG TCG GCT CCC AAG TTC CTG      288
   Leu Ser Ser His Gln Leu Ser Leu Arg Lys Ser Ala Pro Lys Phe Leu
                   85                  90                  95
   CTG GAC GTC TAC CAC CGC ATC ACG GCG GAG GAG GGT CTC AGC GAT CAG      336
   Leu Asp Val Tyr His Arg Ile Thr Ala Glu Glu Gly Leu Ser Asp Gln
                   100                     105                     110
   GAT GAG GAC GAC GAC TAC GAA CGC GGC CAT CGG TCC AGG AGG AGC GCC        384
   Asp Glu Asp Asp Asp Tyr Glu Arg Gly His Arg Ser Arg Arg Ser Ala
               115                     120                     125
   GAC CTC GAG GAG GAT GAG GGC GAG CAG CAG AAG AAC TTC ATC ACC GAC        432
   Asp Leu Glu Glu Asp Glu Gly Glu Gln Gln Lys Asn Phe Ile Thr Asp
           130                     135                     140
   CTG GAC AAG CGG GCC ATC GAC GAG AGC GAC ATC ATC ATG ACC TTC CTG        480
   Leu Asp Lys Arg Ala Ile Asp Glu Ser Asp Ile Ile Met Thr Phe Leu
   145                     150                     155                     160
   AAC AAG CGC CAC CAC AAT GTG GAC GAA CTG CGT CAC GAG CAC GGC CGT        528
   Asn Lys Arg His His Asn Val Asp Glu Leu Arg His Glu His Gly Arg
                   165                     170                     175
   CGC CTG TGG TTC GAC GTC TCC AAC GTG CCC AAC GAC AAC TAC CTG GTG        576
   Arg Leu Trp Phe Asp Val Ser Asn Val Pro Asn Asp Asn Tyr Leu Val
                   180                     185                     190
   ATG GCC GAG CTG CGC ATC TAT CAG AAC GCC AAC GAG GGC AAG TGG CTG        624
   Met Ala Glu Leu Arg Ile Tyr Gln Asn Ala Asn Glu Gly Lys Trp Leu
               195                     200                     205
   ACC GCC AAC AGG GAG TTC ACC ATC ACG GTA TAC GCC ATT GGC ACC GGC        672
   Thr Ala Asn Arg Glu Phe Thr Ile Thr Val Tyr Ala Ile Gly Thr Gly
           210                     215                     220
   ACG CTG GGC CAG CAC ACC ATG GAG CCG CTG TCC TCG GTG AAC ACC ACC        720
   Thr Leu Gly Gln His Thr Met Glu Pro Leu Ser Ser Val Asn Thr Thr
   225                     230                     235                     240
   GGG GAC TAC GTG GGC TGG TTG GAG CTC AAC GTG ACC GAG GGC CTG CAC        768
   Gly Asp Tyr Val Gly Trp Leu Glu Leu Asn Val Thr Glu Gly Leu His
                   245                     250                     255
   GAG TGG CTG GTC AAG TCG AAG GAC AAT CAT GGC ATC TAC ATT GGA GCA        816
   Glu Trp Leu Val Lys Ser Lys Asp Asn His Gly Ile Tyr Ile Gly Ala
                   260                     265                     270
   CAC GCT GTC AAC CGA CCC GAC CGC GAG GTG AAG CTG GAC GAC ATT GGA        864
   His Ala Val Asn Arg Pro Asp Arg Glu Val Lys Leu Asp Asp Ile Gly
               275                     280                     285
   CTG ATC CAC CGC AAG GTG GAC GAC GAG TTC CAG CCC TTC ATG ATC GGC        912
   Leu Ile His Arg Lys Val Asp Asp Glu Phe Gln Pro Phe Met Ile Gly
           290                     295                     300
   TTC TTC CGC GGA CCG GAG CTG ATC AAG GCG ACG GCC CAC AGC AGC CAC        960
   Phe Phe Arg Gly Pro Glu Leu Ile Lys Ala Thr Ala His Ser Ser His
   305                     310                     315                     320
   CAC AGG AGC AAG CGA AGC GCC AGC CAT CCA CGC AAG CGC AAG AAG TCG       1008
   His Arg Ser Lys Arg Ser Ala Ser His Pro Arg Lys Arg Lys Lys Ser
                   325                     330                     335
   GTG TCG CCC AAC AAC GTG CCG CTG CTG GAA CCG ATG GAG AGC ACG CGC       1056
   Val Ser Pro Asn Asn Val Pro Leu Leu Glu Pro Met Glu Ser Thr Arg
                   340                     345                     350
   AGC TGC CAG ATG CAG ACC CTG TAC ATA GAC TTC AAG GAT CTG GGC TGG      1104
   Ser Cys Gln Met Gln Thr Leu Tyr Ile Asp Phe Lys Asp Leu Gly Trp
           355                 360                 365
   CAT GAC TGG ATC ATC GCA CCA GAG GGC TAT GGC GCC TTC TAC TGC AGC      1152
   His Asp Trp Ile Ile Ala Pro Glu Gly Tyr Gly Ala Phe Tyr Cys Ser
       370                 375                 380
   GGC GAG TGC AAT TTC CCG CTC AAT GCG CAC ATG AAC GCC ACG AAC CAT      1200
   Gly Glu Cys Asn Phe Pro Leu Asn Ala His Met Asn Ala Thr Asn His
   385                 390                 395                 400
   GCG ATC GTC CAG ACC CTG GTC CAC CTG CTG GAG CCC AAG AAG GTG CCC      1248
   Ala Ile Val Gln Thr Leu Val His Leu Leu Glu Pro Lys Lys Val Pro
                   405                 410                 415
   AAG CCC TGC TGC GCT CCG ACC AGG CTG GGA GCA CTA CCC GTT CTG TAC      1296
   Lys Pro Cys Cys Ala Pro Thr Arg Leu Gly Ala Leu Pro Val Leu Tyr
               420                 425                 430
   CAC CTG AAC GAC GAG AAT GTG AAC CTG AAA AAG TAT AGA AAC ATG ATT      1344
   His Leu Asn Asp Glu Asn Val Asn Leu Lys Lys Tyr Arg Asn Met Ile
           435                 440                 445
   GTG AAA TCC TGC GGG TGC CAT TGA                                      1368
   Val Lys Ser Cys Gly Cys His
       450                 455
(2) INFORMATION FOR SEQ ID NO:24:
   (i) SEQUENCE CHARACTERISTICS:
      (A) LENGTH: 455 amino acids
      (B) TYPE: amino acid
      (D) TOPOLOGY: linear
   (ii) MOLECULE TYPE: protein
   (xi) SEQUENCE DESCRIPTION: SEQ ID NO:24:
   Met Ser Gly Leu Arg Asn Thr Ser Glu Ala Val Ala Val Leu Ala Ser
   1               5                   10                  15
   Leu Gly Leu Gly Met Val Leu Leu Met Phe Val Ala Thr Thr Pro Pro
               20                  25                  30
   Ala Val Glu Ala Thr Gln Ser Gly Ile Tyr Ile Asp Asn Gly Lys Asp
           35                  40                  45
   Gln Thr Ile Met His Arg Val Leu Ser Glu Asp Asp Lys Leu Asp Val
       50                  55                  60
   Ser Tyr Glu Ile Leu Glu Phe Leu Gly Ile Ala Glu Arg Pro Thr His
   65                  70                  75                  80
   Leu Ser Ser His Gln Leu Ser Leu Arg Lys Ser Ala Pro Lys Phe Leu
                   85                  90                  95
   Leu Asp Val Tyr His Arg Ile Thr Ala Glu Glu Gly Leu Ser Asp Gln
               100                 105                 110
   Asp Glu Asp Asp Asp Tyr Glu Arg Gly His Arg Ser Arg Arg Ser Ala
   115 120 125
   Asp Leu Glu Glu Asp Glu Gly Glu Gln Gln Lys Asn Phe Ile Thr Asp
   130 135 140
   Leu Asp Lys Arg Ala Ile Asp Glu Ser Asp Ile Ile Met Thr Phe Leu
   145 150 155 160
   Asn Lys Arg His His Asn Val Asp Glu Leu Arg His Glu His Gly Arg
   165 170 175
   Arg Leu Trp Phe Asp Val Ser Asn Val Pro Asn Asp Asn Tyr Leu Val
   180 185 190
   Met Ala Glu Leu Arg Ile Tyr Gln Asn Ala Asn Glu Gly Lys Trp Leu
   195 200 205
   Thr Ala Asn Arg Glu Phe Thr Ile Thr Val Tyr Ala Ile Gly Thr Gly
   210 215 220
   Thr Leu Gly Gln His Thr Met Glu Pro Leu Ser Ser Val Asn Thr Thr
   225 230 235 240
   Gly Asp Tyr Val Gly Trp Leu Glu Leu Asn Val Thr Glu Gly Leu His
   245 250 255
   Glu Trp Leu Val Lys Ser Lys Asp Asn His Gly Ile Tyr Ile Gly Ala
   260 265 270
   His Ala Val Asn Arg Pro Asp Arg Glu Val Lys Leu Asp Asp Ile Gly
   275 280 285
   Leu Ile His Arg Lys Val Asp Asp Glu Phe Gln Pro Phe Met Ile Gly
   290 295 300
   Phe Phe Arg Gly Pro Glu Leu Ile Lys Ala Thr Ala His Ser Ser His
   305 310 315 320
   His Arg Ser Lys Arg Ser Ala Ser His Pro Arg Lys Arg Lys Lys Ser
   325 330 335
   Val Ser Pro Asn Asn Val Pro Leu Leu Glu Pro Met Glu Ser Thr Arg
   340 345 350
   Ser Cys Gln Met Gln Thr Leu Tyr Ile Asp Phe Lys Asp Leu Gly Trp
   355 360 365
   His Asp Trp Ile Ile Ala Pro Glu Gly Tyr Gly Ala Phe Tyr Cys Ser
   370 375 380
   Gly Glu Cys Asn Phe Pro Leu Asn Ala His Met Asn Ala Thr Asn His
   385 390 395 400
   Ala Ile Val Gln Thr Leu Val His Leu Leu Glu Pro Lys Lys Val Pro
   405 410 415
   Lys Pro Cys Cys Ala Pro Thr Arg Leu Gly Ala Leu Pro Val Leu Tyr
   420 425 430
   His Leu Asn Asp Glu Asn Val Asn Leu Lys Lys Tyr Arg Asn Met Ile
   435 440 445
   Val Lys Ser Cys Gly Cys His
       450                 455
(2) INFORMATION FOR SEQ ID NO:25:
   (i) SEQUENCE CHARACTERISTICS:
      (A) LENGTH: 1674 base pairs
      (B) TYPE: nucleic acid
      (C) STRANDEDNESS: single
      (D) TOPOLOGY: linear
   (ix) FEATURE:
      (A) NAME/KEY: CDS
      (B) LOCATION: 69..1265
      (D) OTHER INFORMATION: /note= "mOP3-PP"
   (xi) SEQUENCE DESCRIPTION: SEQ ID NO:25:
   GGATCCGCGG CGCTGTCCCA TCCTTGTCGT CGAGGCGTCG CTGGATGCGA GTCCGCTAAA      60
   CGTCCGAG ATG GCT GCG CGT CCG GGA CTC CTA TGG CTA CTG GGC CTG GCT      110
            Met Ala Ala Arg Pro Gly Leu Leu Trp Leu Leu Gly Leu Ala
             1               5                   10
   CTG TGC GTG TTG GGC GGC GGT CAC CTC TCG CAT CCC CCG CAC GTC TTT      158
   Leu Cys Val Leu Gly Gly Gly His Leu Ser His Pro Pro His Val Phe
    15                  20                  25                  30
   CCC CAG CGT CGA CTA GGA GTA CGC GAG CCC CGC GAC ATG CAG CGC GAG      206
   Pro Gln Arg Arg Leu Gly Val Arg Glu Pro Arg Asp Met Gln Arg Glu
                    35                  40                  45
   ATT CGG GAG GTG CTG GGG CTA GCC GGG CGG CCC CGA TCC CGA GCA CCG      254
   Ile Arg Glu Val Leu Gly Leu Ala Gly Arg Pro Arg Ser Arg Ala Pro
                50                  55                  60
   GTC GGG GCT GCC CAG CAG CCA GCG TCT GCG CCC CTC TTT ATG TTG GAC      302
   Val Gly Ala Ala Gln Gln Pro Ala Ser Ala Pro Leu Phe Met Leu Asp
            65                  70                  75
   CTG TAC CGT GCC ATG ACG GAT GAC AGT GGC GGT GGG ACC CCG CAG CCT      350
   Leu Tyr Arg Ala Met Thr Asp Asp Ser Gly Gly Gly Thr Pro Gln Pro
        80                  85                  90
   CAC TTG GAC CGT GCT GAC CTG ATT ATG AGC TTT GTC AAC ATA GTG GAA      398
   His Leu Asp Arg Ala Asp Leu Ile Met Ser Phe Val Asn Ile Val Glu
    95                 100                 105                 110
   CGC GAC CGT ACC CTG GGC TAC CAG GAG CCA CAC TGG AAG GAA TTC CAC      446
   Arg Asp Arg Thr Leu Gly Tyr Gln Glu Pro His Trp Lys Glu Phe His
                   115                 120                 125
   TTT GAC CTA ACC CAG ATC CCT GCT GGG GAG GCT GTC ACA GCT GCT GAG      494
   Phe Asp Leu Thr Gln Ile Pro Ala Gly Glu Ala Val Thr Ala Ala Glu
               130                 135                 140
   TTC CGG ATC TAC AAA GAA CCC AGT ACC CAC CCG CTC AAC ACA ACC CTC      542
   Phe Arg Ile Tyr Lys Glu Pro Ser Thr His Pro Leu Asn Thr Thr Leu
           145                 150                 155
   CAC ATC AGC ATG TTC GAA GTG GTC CAA GAG CAC TCC AAC AGG GAG TCT      590
   His Ile Ser Met Phe Glu Val Val Gln Glu His Ser Asn Arg Glu Ser
       160                 165                 170
   GAC TTG TTC TTT TTG GAT CTT CAG ACG CTC CGA TCT GGG GAC GAG GGC      638
   Asp Leu Phe Phe Leu Asp Leu Gln Thr Leu Arg Ser Gly Asp Glu Gly
   175                 180                 185                 190
   TGG CTG GTG CTG GAC ATC ACA GCA GCC AGT GAC CGA TGG CTG CTG AAC      686
   Trp Leu Val Leu Asp Ile Thr Ala Ala Ser Asp Arg Trp Leu Leu Asn
                   195                 200                 205
   CAT CAC AAG GAC CTA GGA CTC CGC CTC TAT GTG GAA ACC GAG GAT GGG      734
   His His Lys Asp Leu Gly Leu Arg Leu Tyr Val Glu Thr Glu Asp Gly
               210                 215                 220
   CAC AGC ATA GAT CCT GGC CTA GCT GGT CTG CTT GGA CGA CAA GCA CCA      782
   His Ser Ile Asp Pro Gly Leu Ala Gly Leu Leu Gly Arg Gln Ala Pro
           225                 230                 235
   CGC TCC AGA CAG CCT TTC ATG GTT GGT TTC TTC AGG GCC AAC CAG AGT      830
   Arg Ser Arg Gln Pro Phe Met Val Gly Phe Phe Arg Ala Asn Gln Ser
       240                 245                 250
   CCT GTG CGG GCC CCT CGA ACA GCA AGA CCA CTG AAG AAG AAG CAG CTA      878
   Pro Val Arg Ala Pro Arg Thr Ala Arg Pro Leu Lys Lys Lys Gln Leu
   255                 260                 265                 270
   AAT CAA ATC AAC CAG CTG CCG CAC TCC AAC AAA CAC CTA GGA ATC CTT      926
   Asn Gln Ile Asn Gln Leu Pro His Ser Asn Lys His Leu Gly Ile Leu
                   275                 280                 285
   GAT GAT GGC CAC GGT TCT CAC GGC AGA GAA GTT TGC CGC AGG CAT GAG      974
   Asp Asp Gly His Gly Ser His Gly Arg Glu Val Cys Arg Arg His Glu
               290                 295                 300
   CTC TAT GTC AGC TTC CGT GAC CTT GGC TGG CTG GAC TCT GTC ATT GCC     1022
   Leu Tyr Val Ser Phe Arg Asp Leu Gly Trp Leu Asp Ser Val Ile Ala
           305                 310                 315
   CCC CAG GGC TAC TCC GCC TAT TAC TGT GCT GGG GAG TGC ATC TAC CCA     1070
   Pro Gln Gly Tyr Ser Ala Tyr Tyr Cys Ala Gly Glu Cys Ile Tyr Pro
       320                 325                 330
   CTG AAC TCC TGT ATG AAC TCC ACC AAC CAC GCC ACT ATG CAG GCC CTG     1118
   Leu Asn Ser Cys Met Asn Ser Thr Asn His Ala Thr Met Gln Ala Leu
   335                 340                 345                 350
   GTA CAT CTG ATG AAG CCA GAT ATC ATC CCC AAG GTG TGC TGT GTG CCT     1166
   Val His Leu Met Lys Pro Asp Ile Ile Pro Lys Val Cys Cys Val Pro
                   355                 360                 365
   ACT GAG CTG AGT GCC ATT TCT CTG CTC TAC TAT GAT AGA AAC AAT AAT     1214
   Thr Glu Leu Ser Ala Ile Ser Leu Leu Tyr Tyr Asp Arg Asn Asn Asn
               370                 375                 380
   GTC ATC CTG CGC AGG GAG CGC AAC ATG GTA GTC CAG GCC TGT GGC TGC     1262
   Val Ile Leu Arg Arg Glu Arg Asn Met Val Val Gln Ala Cys Gly Cys
           385                 390                 395
   CAC TGAGTCCCTG CCCAACAGCC TGCTGCCATC CCATCTATCT AGTCAGGCCT           1315
   His
   CTCTTCCAAG GCAGGAAACC AACAAAGAGG GAAGGCAGTG CTTTCAACTC CATGTCCACA 1375
   TTCACAGTCT TGGCCCTCTC TGTTCTTTTT GCCAAGGCTG AGAAGATGGT CCTAGTTATA 1435
   ACCCTGGTGA CCTCAGTAGC CCGATCTCTC ATCTCCCCAA ACTCCCCAAT GCAGCCAGGG 1495
   GCATCTATGT CCTTTGGGAT TGGGCACAGA AGTCCAATTT ACCAACTTAT TCATGAGTCA 1555
   CTACTGGCCC AGCCTGGACT TGAACCTGGA ACACAGGGTA GAGCTCAGGC TCTTCAGTAT 1615
   CCATCAGAAG ATTTAGGTGT GTGCAGACAT GACCACACTC CCCCTAGCAC TCCATAGCC 1674
(2) INFORMATION FOR SEQ ID NO:26:
   (i) SEQUENCE CHARACTERISTICS:
      (A) LENGTH: 399 amino acids
      (B) TYPE: amino acid
      (D) TOPOLOGY: linear
   (ii) MOLECULE TYPE: protein
   (xi) SEQUENCE DESCRIPTION: SEQ ID NO:26:
   Met Ala Ala Arg Pro Gly Leu Leu Trp Leu Leu Gly Leu Ala Leu Cys
   1               5                   10                  15
   Val Leu Gly Gly Gly His Leu Ser His Pro Pro His Val Phe Pro Gln
               20                  25                  30
   Arg Arg Leu Gly Val Arg Glu Pro Arg Asp Met Gln Arg Glu Ile Arg
           35                  40                  45
   Glu Val Leu Gly Leu Ala Gly Arg Pro Arg Ser Arg Ala Pro Val Gly
       50                  55                  60
   Ala Ala Gln Gln Pro Ala Ser Ala Pro Leu Phe Met Leu Asp Leu Tyr
   65                  70                  75                  80
   Arg Ala Met Thr Asp Asp Ser Gly Gly Gly Thr Pro Gln Pro His Leu
                   85                  90                  95
   Asp Arg Ala Asp Leu Ile Met Ser Phe Val Asn Ile Val Glu Arg Asp
               100                 105                 110
   Arg Thr Leu Gly Tyr Gln Glu Pro His Trp Lys Glu Phe His Phe Asp
           115                 120                 125
   Leu Thr Gln Ile Pro Ala Gly Glu Ala Val Thr Ala Ala Glu Phe Arg
       130                 135                 140
   Ile Tyr Lys Glu Pro Ser Thr His Pro Leu Asn Thr Thr Leu His Ile
   145                 150                 155                 160
   Ser Met Phe Glu Val Val Gln Glu His Ser Asn Arg Glu Ser Asp Leu
                   165                 170                 175
   Phe Phe Leu Asp Leu Gln Thr Leu Arg Ser Gly Asp Glu Gly Trp Leu
                   180                 185                 190
   Val Leu Asp Ile Thr Ala Ala Ser Asp Arg Trp Leu Leu Asn His His
           195                 200                 205
   Lys Asp Leu Gly Leu Arg Leu Tyr Val Glu Thr Glu Asp Gly His Ser
       210                 215                 220
   Ile Asp Pro Gly Leu Ala Gly Leu Leu Gly Arg Gln Ala Pro Arg Ser
   225                 230                 235                 240
   Arg Gln Pro Phe Met Val Gly Phe Phe Arg Ala Asn Gln Ser Pro Val
                   245                 250                 255
   Arg Ala Pro Arg Thr Ala Arg Pro Leu Lys Lys Lys Gln Leu Asn Gln
               260                 265                 270
   Ile Asn Gln Leu Pro His Ser Asn Lys His Leu Gly Ile Leu Asp Asp
           275                 280                 285
   Gly His Gly Ser His Gly Arg Glu Val Cys Arg Arg His Glu Leu Tyr
       290                 295                 300
   Val Ser Phe Arg Asp Leu Gly Trp Leu Asp Ser Val Ile Ala Pro Gln
   305                 310                 315                 320
   Gly Tyr Ser Ala Tyr Tyr Cys Ala Gly Glu Cys Ile Tyr Pro Leu Asn
                   325                 330                 335
   Ser Cys Met Asn Ser Thr Asn His Ala Thr Met Gln Ala Leu Val His
               340                 345                 350
   Leu Met Lys Pro Asp Ile Ile Pro Lys Val Cys Cys Val Pro Thr Glu
           355                 360                 365
   Leu Ser Ala Ile Ser Leu Leu Tyr Tyr Asp Arg Asn Asn Asn Val Ile
       370                 375                 380
   Leu Arg Arg Glu Arg Asn Met Val Val Gln Ala Cys Gly Cys His
   385                 390                 395
(2) INFORMATION FOR SEQ ID NO:27:
   (i) SEQUENCE CHARACTERISTICS:
      (A) LENGTH: 104 amino acids
      (B) TYPE: amino acid
      (C) STRANDEDNESS:
      (D) TOPOLOGY: linear
   (ii) MOLECULE TYPE: protein
   (ix) FEATURE:
      (A) NAME/KEY: Protein
      (B) LOCATION: 1..104
      (D) OTHER INFORMATION: /note= "BMP3"
   (xi) SEQUENCE DESCRIPTION: SEQ ID NO:27;
(2) INFORMATION FOR SEQ ID NO:28:
   (i) SEQUENCE CHARACTERISTICS:
      (A) LENGTH: 102 amino acids
      (B) TYPE: amino acid
      (C) STRANDEDNESS:
      (D) TOPOLOGY: linear
   (ii) MOLECULE TYPE: protein
   (vi) ORIGINAL SOURCE:
      (A) ORGANISM: HOMO SAPIENS
   (ix) FEATURE:
      (A) NAME/KEY: Protein
      (B) LOCATION: 1..102
      (D) OTHER INFORMATION: /note= "BMP5"
   (xi) SEQUENCE DESCRIPTION: SEQ ID NO:28:
   Cys Lys Lys His Glu Leu Tyr Val Ser Phe Arg Asp Leu Gly Trp Gln
   1 5 10 15
   Asp Trp Ile Ile Ala Pro Glu Gly Tyr Ala Ala Phe Tyr Cys Asp Gly
   20 25 30
   Glu Cys Ser Phe Pro Leu Asn Ala His Met Asn Ala Thr Asn His Ala
   35 40 45
   Ile Val Gln Thr Leu Val His Leu Met Phe Pro Asp His Val Pro Lys
   50 55 60
   Pro Cys Cys Ala Pro Thr Lys Leu Asn Ala Ile Ser Val Leu Tyr Phe
   65 70 75 80
   Asp Asp Ser Ser Asn Val Ile Leu Lys Lys Tyr Arg Asn Met Val Val
   85 90 95
   Arg Ser Cys Gly Cys His
   100
(2) INFORMATION FOR SEQ ID NO:29:
   (i) SEQUENCE CHARACTERISTICS:
      (A) LENGTH: 102 amino acids
      (B) TYPE: amino acid
      (C) STRANDEDNESS:
      (D) TOPOLOGY: linear
   (ii) MOLECULE TYPE: protein
   (vi) ORIGINAL SOURCE:
      (A) ORGANISM: HOMO SAPIENS
   (ix) FEATURE:
      (A) NAME/KEY: Protein
      (B) LOCATION: 1..102
      (D) OTHER INFORMATION: /note= "BMP6"
   (xi) SEQUENCE DESCRIPTION: SEQ ID NO:29:
   Cys Arg Lys His Glu Leu Tyr Val Ser Phe Gln Asp Leu Gly Trp Gln
   1               5                   10                  15
   Asp Trp Ile Ile Ala Pro Lys Gly Tyr Ala Ala Asn Tyr Cys Asp Gly
               20                  25                  30
   Glu Cys Ser Phe Pro Leu Asn Ala His Met Asn Ala Thr Asn His Ala
           35                  40                  45
   Ile Val Gln Thr Leu Val His Leu Met Asn Pro Glu Tyr Val Pro Lys
       50                  55                  60
   Pro Cys Cys Ala Pro Thr Lys Leu Asn Ala Ile Ser Val Leu Tyr Phe
   65                  70                  75                      80
   Asp Asp Asn Ser Asn Val Ile Leu Lys Lys Tyr Arg Trp Met Val Val
                   85                  90                  95
   Arg Ala Cys Gly Cys His
               100
(2) INFORMATION FOR SEQ ID NO:30:
   (i) SEQUENCE CHARACTERISTICS:
      (A) LENGTH: 1247 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: HOMO SAPIENS
      (F) TISSUE TYPE: BRAIN
   (ix) FEATURE:
      (A) NAME/KEY: CDS
      (B) LOCATION: 84..1199
      (D) OTHER INFORMATION: /product= "GDF-1"
         /note= "GDF-1 CDNA"
   (xi) SEQUENCE DESCRIPTION: SEQ ID NO:30:
   GGGGACACCG GCCCCGCCCT CAGCCCACTG GTCCCGGGCC GCCGCGGACC CTGCGCACTC    60
   TCTGGTCATC GCCTGGGAGG AAG ATG CCA CCG CCG CAG CAA GGT CCC TGC       110
                             Met Pro Pro Pro Gln Gln Gly Pro Cys
                              1               5
   GGC CAC CAC CTC CTC CTC CTC CTG GCC CTG CTG CTG CCC TCG CTG CCC     158
   Gly His His Leu Leu Leu Leu Leu Ala Leu Leu Leu Pro Ser Leu Pro
    10                  15                  20                  25
   CTG ACC CGC GCC CCC GTG CCC CCA GGC CCA GCC GCC GCC CTG CTC CAG     206
   Leu Thr Arg Ala Pro Val Pro Pro Gly Pro Ala Ala Ala Leu Leu Gln
                    30                  35                  40
   GCT CTA GGA CTG CGC GAT GAG CCC CAG GGT GCC CCC AGG CTC CGG CCG     254
   Ala Leu Gly Leu Arg Asp Glu Pro Gln Gly Ala Pro Arg Leu Arg Pro
                45                  50                  55
   GTT CCC CCG GTC ATG TGG CGC CTG TTT CGA CGC CGG GAC CCC CAG GAG     302
   Val Pro Pro Val Met Trp Arg Leu Phe Arg Arg Arg Asp Pro Gln Glu
            60                  65                  70
   ACC AGG TCT GGC TCG CGG CGG ACG TCC CCA GGG GTC ACC CTG CAA CCG     350
   Thr Arg Ser Gly Ser Arg Arg Thr Ser Pro Gly Val Thr Leu Gln Pro
        75                  80                  85
   TGC CAC GTG GAG GAG CTG GGG GTC GCC GGA AAC ATC GTG CGC CAC ATC     398
   Cys His Val Glu Glu Leu Gly Val Ala Gly Asn Ile Val Arg His Ile
    90                  95                 100                 105
   CCG GAC CGC GGT GCG CCC ACC CGG GCC TCG GAG CCT GTC TCG GCC GCG     446
   Pro Asp Arg Gly Ala Pro Thr Arg Ala Ser Glu Pro Val Ser Ala Ala
                   110                 115                 120
   GGG CAT TGC CCT GAG TGG ACA GTC GTC TTC GAC CTG TCG GCT GTG GAA     494
   Gly His Cys Pro Glu Trp Thr Val Val Phe Asp Leu Ser Ala Val Glu
               125                 130                 135
   CCC GCT GAG CGC CCG AGC CGG GCC CGC CTG GAG CTG CGT TTC GCG GCG     542
   Pro Ala Glu Arg Pro Ser Arg Ala Arg Leu Glu Leu Arg Phe Ala Ala
           140                 145                 150
   GCG GCG GCG GCA GCC CCG GAG GGC GGC TGG GAG CTG AGC GTG GCG CAA     590
   Ala Ala Ala Ala Ala Pro Glu Gly Gly Trp Glu Leu Ser Val Ala Gln
       155                 160                 165
   GCG GGC CAG GGC GCG GGC GCG GAC CCC GGG CCG GTG CTG CTC CGC CAG     638
   Ala Gly Gln Gly Ala Gly Ala Asp Pro Gly Pro Val Leu Leu Arg Gln
   170                 175                 180                 185
   TTG GTG CCC GCC CTG GGG CCG CCA GTG CGC GCG GAG CTG CTG GGC GCC     686
   Leu Val Pro Ala Leu Gly Pro Pro Val Arg Ala Glu Leu Leu Gly Ala
                   190                 195                 200
   GCT TGG GCT CGC AAC GCC TCA TGG CCG CGC AGC CTC CGC CTG GCG CTG     734
   Ala Trp Ala Arg Asn Ala Ser Trp Pro Arg Ser Leu Arg Leu Ala Leu
                   205                      210                      215
   GCG CTA CGC CCC CGG GCC CCT GCC GCC TGC GCG CGC CTG GCC GAG GCC       782
   Ala Leu Arg Pro Arg Ala Pro Ala Ala Cys Ala Arg Leu Ala Glu Ala
           220                     225                     230
   TCG CTG CTG CTG GTG ACC CTC GAC CCG CGC CTG TGC CAC CCC CTG GCC       830
   Ser Leu Leu Leu Val Thr Leu Asp Pro Arg Leu Cys His Pro Leu Ala
       235                     240                     245
   CGG CCG CGG CGC GAC GCC GAA CCC GTG TTG GGC GGC GGC CCC GGG GGC       878
   Arg Pro Arg Arg Asp Ala Glu Pro Val Leu Gly Gly Gly Pro Gly Gly
   250                     255                     260                 265
   GCT TGT CGC GCG CGG CGG CTG TAC GTG AGC TTC CGC GAG GTG GGC TGG       926
   Ala Cys Arg Ala Arg Arg Leu Tyr Val Ser Phe Arg Glu Val Gly Trp
                   270                     275                     280
   CAC CGC TGG GTC ATC GCG CCG CGC GGC TTC CTG GCC AAC TAC TGC CAG       974
   His Arg Trp Val Ile Ala Pro Arg Gly Phe Leu Ala Asn Tyr Cys Gln
               285                     290                     295
   GGT CAG TGC GCG CTG CCC GTC GCG CTG TCG GGG TCC GGG GGG CCG CCG      1022
   Gly Gln Cys Ala Leu Pro Val Ala Leu Ser Gly Ser Gly Gly Pro Pro
           300                     305                     310
   GCG CTC AAC CAC GCT GTG CTG CGC GCG CTC ATG CAC GCG GCC GCC CCG      1070
   Ala Leu Asn His Ala Val Leu Arg Ala Leu Met His Ala Ala Ala Pro
       315                     320                     325
   GGA GCC GCC GAC CTG CCC TGC TGC GTG CCC GCG CGC CTG TCG CCC ATC      1118
   Gly Ala Ala Asp Leu Pro Cys Cys Val Pro Ala Arg Leu Ser Pro Ile
   330                     335                     340                 345
   TCC GTG CTC TTC TTT GAC AAC AGC GAC AAC GTG GTG CTG CGG CAG TAT      1166
   Ser Val Leu Phe Phe Asp Asn Ser Asp Asn Val Val Leu Arg Gln Tyr
                   350                     355                     360
   GAG GAC ATG GTG GTG GAC GAG TGC GGC TGC CGC TAACCCGGGG CGGGCAGGGA    1219
   Glu Asp Met Val Val Asp Glu Cys Gly Cys Arg
               365                     370
   CCCGGGCCCA ACAATAAATG CCGCGTGG                                        1247
(2) INFORMATION FOR SEQ ID NO:31:
   (i) SEQUENCE CHARACTERISTICS:
      (A) LENGTH: 372 amino acids
      (B) TYPE: amino acid
      (D) TOPOLOGY: linear
   (ii) MOLECULE TYPE: protein
   (xi) SEQUENCE DESCRIPTION: SEQ ID NO:31:
   Met Pro Pro Pro Gln Gln Gly Pro Cys Gly His His Leu Leu Leu Leu
    1               5                  10                  15
   Leu Ala Leu Leu Leu Pro Ser Leu Pro Leu Thr Arg Ala Pro Val Pro
               20                  25                  30
   Pro Gly Pro Ala Ala Ala Leu Leu Gln Ala Leu Gly Leu Arg Asp Glu
   35 40 45
   Pro Gln Gly Ala Pro Arg Leu Arg Pro Val Pro Pro Val Met Trp Arg
   50 55 60
   Leu Phe Arg Arg Arg Asp Pro Gln Glu Thr Arg Ser Gly Ser Arg Arg
   65 70 75 80
   Thr Ser Pro Gly Val Thr Leu Gln Pro Cys His Val Glu Glu Leu Gly
   85 90 95
   Val Ala Gly Asn Ile Val Arg His Ile Pro Asp Arg Gly Ala Pro Thr
   100 105 110
   Arg Ala Ser Glu Pro Val Ser Ala Ala Gly His Cys Pro Glu Trp Thr
   115 120 125
   Val Val Phe Asp Leu Ser Ala Val Glu Pro Ala Glu Arg Pro Ser Arg
   130 135 140
   Ala Arg Leu Glu Leu Arg Phe Ala Ala Ala Ala Ala Ala Ala Pro Glu
   145 150 155 160
   Gly Gly Trp Glu Leu Ser Val Ala Gln Ala Gly Gln Gly Ala Gly Ala
   165 170 175
   Asp Pro Gly Pro Val Leu Leu Arg Gln Leu Val Pro Ala Leu Gly Pro
   180 185 190
   Pro Val Arg Ala Glu Leu Leu Gly Ala Ala Trp Ala Arg Asn Ala Ser
   195 200 205
   Trp Pro Arg Ser Leu Arg Leu Ala Leu Ala Leu Arg Pro Arg Ala Pro
   210 215 220
   Ala Ala Cys Ala Arg Leu Ala Glu Ala Ser Leu Leu Leu Val Thr Leu
   225 230 235 240
   Asp Pro Arg Leu Cys His Pro Leu Ala Arg Pro Arg Arg Asp Ala Glu
   245 250 255
   Pro Val Leu Gly Gly Gly Pro Gly Gly Ala Cys Arg Ala Arg Arg Leu
   260 265 270
   Tyr Val Ser Phe Arg Glu Val Gly Trp His Arg Trp Val Ile Ala Pro
   275 280 285
   Arg Gly Phe Leu Ala Asn Tyr Cys Gln Gly Gln Cys Ala Leu Pro Val
   290 295 300
   Ala Leu Ser Gly Ser Gly Gly Pro Pro Ala Leu Asn His Ala Val Leu
   305 310 315 320
   Arg Ala Leu Met His Ala Ala Ala Pro Gly Ala Ala Asp Leu Pro Cys
   325 330 335
   Cys Val Pro Ala Arg Leu Ser Pro Ile Ser Val Leu Phe Phe Asp Asn
   340 345 350
   Ser Asp Asn Val Val Leu Arg Gln Tyr Glu Asp Met Val Val Asp Glu
   355 360 365
   Cys Gly Cys Arg
       370
(2) INFORMATION FOR SEQ ID NO:32:
   (i) SEQUENCE CHARACTERISTICS:
      (A) LENGTH: 4 amino acids
      (B) TYPE: amino acid
      (C) STRANDEDNESS:
      (D) TOPOLOGY: linear
   (xi) SEQUENCE DESCRIPTION: SEQ ID NO:32:
   Arg Xaa Xaa Arg
   1

## Description

### Field of the Invention

The present invention relates generally to the dental and biomedical arts. In certain embodiments, the invention more particularly relates to methods and compositions for stimulating mammalian odontoblasts and inducing morphogenesis of mammalian dentine.

### Background of the Invention

In mammals, periodontal disease, such gingivitis, can arise from the weakening of periodontal tissue by infectious agents (e.g., buccal microorganisms), nutritional deficiency (e.g. scurvy), or neoplastic disease (e.g., leukemia and lymphoma). Periodontal diseases often are characterized by inflammation, bleeding, tissue recession and/or ulceration. If not properly treated, periodontal diseases can contribute to tooth loss. For example, gingival lesions can arise where bacterial plaque adheres to the tooth/gingiva interface and provokes local inflammation and/or recession of the gingiva. In the early stages, gingivitis is associated with tooth sensitivity to perception of pressure and/or temperature. For example, afflicted teeth may ache upon contact with cold or hot stimuli. If untreated, this progresses to severe continual throbbing pain, ultimately associated with infection of the tooth pulp tissue, periodontal ligament, or alveolar bone of the tooth socket. More severe complications, e.g., endocarditis, can arise where untreated lesions provide buccal microorganisms with a portal of entry into the afflicted individual's bloodstream. Harrison's Principles of Internal Medicine, 12th edition, 1991 (Wilson et al., eds.), pp. 242-243. Current treatments include professional cleaning to remove plaque and tartar, use of oral antiseptics, local and/or systemic antibiotic therapies, and/or surgical procedures to remove periodontal pockets formed from periodontal tissue lesions and necrosis. Gingivitis thus is treated by debridement of lesioned gingiva and the affected tooth or tooth root surface adjoining the lesion site. Treated gingival lesions heal through the formation of scar tissue at the lesion site. Where tooth loss is imminent or has already occurred as a result of periodontal disease, a prosthetic tooth or removable bridge is substituted for the natural tooth.

Dental caries also is generally attributable to the weakening of tooth tissue by infectious agents or nutritional causes. A cavity, or carious lesion, often involves colonization and degradation of mineralized tooth tissue (e.g., enamel or dentine) by buccal microorganisms. If untreated, the lesion site expands and can weaken, permeating the mineralized tooth wall and placing the tooth pulp tissue at risk of infection. Thus, an untreated carious lesion site also can provide buccal microorganisms with a portal of entry into the bloodstream. Conventional treatments for dental caries include ablation of lesioned dentine to expose a fresh surface of unaffected residual dentine, followed by sealing and restoration with an inert material suitable for dental use, e.g., silver amalgam, composite plastic, gold or porcelain. If infection has spread to the pulp tissue, it becomes necessary to extract the tooth or remove the contents of the pulp chamber and root canals prior to sealing and reconstruction with inert materials. Both approaches require the construction of permanent dental prostheses, such as bridges or crowns, which can become brittle over time.

Previously disclosed are methods and compositions capable of inducing periodontal tissue morphogenesis and dentinogenesis in a mammal, including a therapeutically effective concentration of a morphogen (U.S.S.N. 08/155,343 (published as WO94/06399)). Yet, needs remain for improved treatment of dental caries and periodontal disease, including gingivitis. Particular needs remain for improved treatment methods and compositions which mitigate loss of teeth and associated tissue, including dentine, gingiva and pulp tissue. Still more particular needs remain for improved methods and compositions which allow for the regenerative healing of functional dental tissues following resection of carious or periodontal lesions, including dentine tissue, pulp, cementum, periodontal ligament, gingiva and the like.

### Summary of the Invention

It is an object of this invention to provide means for inhibiting loss of dental tissue in mammals, as well as means for inducing regeneration thereof. It is an object of the present invention to provide means for stimulating proliferation and differentiation of odontoblasts in mammals, particularly primates. It also is an object of the present invention to provide means for stimulating expression of the odontoblast phenotype, including production of mineralized dentine matrix, by mammalian tooth pulp tissue, including primate tooth pulp tissue such as human tooth pulp tissue. Another object is to provide means for inhibiting the periodontal tissue damage and tooth loss associated with periodontal and other gum diseases, including gingivitis. Additional objects include providing means for desensitizing teeth to perception of pressure or temperature, as well as for sealing a tooth cavity by inducing formation of reparative dentine tissue. These and other objects, along with advantages and features of the invention disclosed herein, will be apparent from the description, drawings and claims that follow.

The invention is defined in the claims appended hereto. The invention provides methods and compositions for inhibiting periodontal and tooth tissue (collectively, dental tissue) loss in a mammal, particularly a human, including regenerating damaged tissue and/or inhibiting additional damage thereto. The methods and compositions of this invention can be used to prevent and/or inhibit tooth loss associated with gingivitis and other periodontal diseases. The present methods and compositions also can be used to desensitize teeth to perception of pressure and/or temperature, and pain associated, therewith in dental caries and gingivitis. The invention further provides methods and compositions for stimulating morphogenesis of mammalian dentine, including stimulating proliferation and differentiation of odontoblasts. In particular, the invention provides methods and compositions for stimulating expression of the odontoblast phenotype, including production of dentine matrix, by tooth pulp tissue in mammals, including primates. The present invention can be used to seal a cavity in a mammalian tooth by inducing the formation of reparative dentine. Thus, the invention reduces the need for tooth extraction or root canal therapy as treatments for dental caries or other dental damage in which pulp tissue is placed at risk.

The methods and compositions of this invention capitalize on the discovery that certain proteins of eukaryotic origin, defined herein as morphogens, induce morphogenesis of functional cells, tissues and organs in higher eukaryotes, particularly mammals, including humans. That is, morphogens induce or reinduce the fully integrated developmental cascade of cellular and molecular morphogenetic events that culminate in the formation of fully differentiated, functional tissue of a type appropriate to the context or local environment in which morphogenesis is induced, including any vascularization, connective tissue formation, ennervation and the like characteristic of the naturally-occurring tissue. Morphogenesis therefore differs significantly from simple reparative healing processes in which scar tissue (e.g., fibrous connective tissue) is formed and fills a lesion or other defect in differentiated, functional tissue. Further, morphogenesis occurs in a "permissive environment" by which is meant a local environment that does not stifle or suppress morphogenesis (e.g., regeneration or regenerative healing). Permissive environments exist, e.g., in embryonic tissue or in wounded or diseased tissue, including tissue subjected to surgical intervention. Often, a permissive environment comprises a suitable matrix or substratum to which cells undergoing differentiation can anchor. Other components of a permissive environment typically include signals, e.g., cell surface markers or extracellular matrix components, that direct the tissue specificity of differentiation.

Generally, morphogens are dimeric proteins that induce morphogenesis of one or more eukaryotic (e.g., mammalian) cells, tissues or organs. Of particular interest herein are morphogens that induce morphogenesis at least of mammalian dentine, including formation of reparative dentine at or apposite to a dental or periodontal lesion site in a mammalian tooth. Morphogens comprise a pair of polypeptides that, when folded, adopt a configuration sufficient for the resulting dimeric protein to elicit morphogenetic responses in cells and tissues displaying receptors specific for said morphogen. That is, morphogens generally induce all of the following biological functions in a morphogenically permissive environment: stimulating proliferation of progenitor cells; stimulating the differentiation of progenitor cells; stimulating the proliferation of differentiated cells; and supporting the growth and maintenance of differentiated cells. "Progenitor" cells are uncommitted cells that are competent to differentiate into one or more specific types of differentiated cells, depending on their genomic repertoire and the tissue specificity of the permissive environment in which morphogenesis is induced. Morphogens further can delay or mitigate the onset of senescence- or quiescence-associated loss of phenotype and/or tissue function. Morphogens still further can stimulate phenotypic expression of differentiated cells, including expression of metabolic and/or functional, e.g., secretory, properties thereof. In addition, morphogens can induce redifferentiation of committed cells under appropriate environmental conditions. As noted above, morphogens that induce proliferation and differentiation at least of mammalian odontoblasts, and/or support the growth, maintenance and functional properties of mammalian odontoblasts, including the formation of dentine matrix, are of particular interest herein. For purposes of the present invention, an "odontoblast" is any differentiated cell occurring or arising in mammalian tooth pulp tissue, that is competent to produce dentine matrix.

In preferred embodiments, the pair of morphogen polypeptides have amino acid sequences each comprising a sequence that shares a defined relationship with an amino acid sequence of a reference morphogen. Herein, preferred morphogen polypeptides share a defined relationship with a sequence present in morphogenically active human OP-1, Seq. ID No. 4. However, any one or more of the naturally occurring or biosynthetic sequences disclosed herein similarly could be used as a reference sequence. Preferred morphogen polypeptides share a defined relationship with at least the C-terminal six cysteine domain of human OP-1, residues 43-139 of Seq. ID No. 4. Preferably, morphogen polypeptides share a defined relationship with at least the C-terminal seven cysteine domain of human OP-1, residues 38-139 of Seq. ID No. 4. That is, preferred morphogen polypeptides in a dimeric protein with morphogenic activity each comprise a sequence that corresponds to a reference sequence or is functionally equivalent thereto.

Functionally equivalent sequences include functionally equivalent arrangements of cysteine residues disposed within the reference sequence, including amino acid insertions or deletions which alter the linear arrangement of these cysteines, but do not materially impair their relationship in the folded structure of the dimeric morphogen protein, including their ability to form such intra- or inter-chain disulfide bonds as may be necessary for morphogenic activity. Functionally equivalent sequences further include those wherein one or more amino acid residues differs from the corresponding residue of a reference morphogen sequence, e.g., the C-terminal seven cysteine domain (also referred to herein as the conserved seven cysteine skeleton) of human OP-1, provided that this difference does not destroy morphogenic activity. Accordingly, conservative substitutions of corresponding amino acids in the reference sequence are preferred. Amino acid residues that are "conservative substitutions" for corresponding residues in a reference sequence are those that are physically or functionally similar to the corresponding reference residues, e.g., that have similar size, shape, electric charge, chemical properties including the ability to form covalent or hydrogen bonds, or the like. Particularly preferred conservative substitutions are those fulfilling the criteria defined for an "accepted point mutation" in Dayhoff et al. (1978), 5 Atlas of Protein Sequence and Structure, Suppl. 3, ch. 22 (pp. 354-352), Natl. Biomed. Res. Found., Washington, D.C. 20007.

In certain embodiments, a polypeptide suspected of being functionally equivalent to a reference morphogen polypeptide is aligned therewith using the method of Needleman et al. (1970), 48 J.Mol. Biol. 443-453, implemented conveniently by computer programs such as the Align program (DNAstar, Inc). As noted above, internal gaps and amino acid insertions in the candidate sequence are ignored for purposes of calculating the defined relationship, conventionally expressed as a level of amino acid sequence homology or identity, between the candidate and reference sequences. "Amino acid sequence homology" is understood herein to mean amino acid sequence similarity. Homologous sequences share identical or similar amino acid residues, where similar residues are conservative substitutions for or "allowed point mutations" of corresponding amino acid residues in an aligned reference sequence. Thus, a candidate polypeptide sequence that shares 70% amino acid homology with a reference sequence is one in which any 70% of the aligned residues are either identical to or are conservative substitutions of the corresponding residues in a reference sequence.

Of particular interest herein are morphogens, which, when provided to the tooth and/or jawbone surfaces in a mammalian tooth socket, induce periodontal tissue formation where periodontal tissue has been lost or damaged. Of still more particular interest herein are morphogens which, when applied to a tooth surface, such as a dentinal surface, induce morphogenesis of new or reparative dentine. Such morphogens can be used to seal a tooth cavity or to desensitize a tooth to perception of pressure and/or temperature.

The present invention alternatively can be practiced with methods and compositions comprising a morphogen stimulating agent in lieu of a morphogen. A "morphogen stimulating agent" is a compound that stimulates *in vivo* production, e.g., expression, of a therapeutically effective concentration of an endogenous morphogen in the body of the mammal sufficient to regenerate damaged dental tissue and/or to inhibit additional damage thereto. Such compounds are understood to include substances which, when administered to a mammal, act on cells of tissue(s) or organ(s) that normally are competent to produce and/or secrete a morphogen encoded within the genome of the mammal, and which cause the endogenous level of the morphogen in the mammal's body to be altered. Endogenous or administered morphogens can act as endocrine, paracrine or autocrine factors. That is, endogenous morphogens can be synthesized by the cells in which morphogenetic responses are induced, by neighboring cells, or by cells of a distant tissue, in which circumstances the secreted endogenous morphogen is transported to the site of morphogenesis e.g., by the individual's bloodstream. In preferred embodiments, the agent stimulates expression and/or secretion of an endogenous morphogen so as to increase amounts thereof in dental tissues, such alveolar bone, periodontium, cementum, dentine or pulp tissue cells.

In certain preferred aspects of the present invention, the morphogens described herein can induce regeneration of damaged or lost dentine tissue in a mammalian tooth. The morphogen can be provided topically or otherwise administered to the tooth tissue. For example, the morphogen can be dispersed in a biocompatible, porous carrier material that then is provided topically to the damaged dentine tissue. A useful carrier can be formulated from suitable organ specific tissue, e.g., bone or dentine, by demineralizing and guanidine-extracting the tissue to create an acellular matrix as described in U.S.S.Nos. 07/971,091 (published as WO94/10203), 08/155,343 (published as WO94/06399) and 08/174,605 (published as WO94/06420). Synthetic materials also can be used. In some embodiments, the existing tooth tissue provides a suitable matrix. If a formulated matrix or carrier is used, it should be a biocompatible, suitably modified acellular matrix having dimensions such that it allows the differentiation and proliferation of migratory progenitor cells, and contributes to a morphogenically permissive environment. Preferably, the matrix allows cellular attachment and is biodegradable or bioresorbable. Where the tissue locus to which the morphogen and matrix are applied lacks sufficient endogenous signals to direct the tissue specificity of morphogenesis, the matrix preferably further comprises tissue-specific components or is derived from tissue of the desired type. Matrices can be generated from dehydrated organ-specific tissue by, e.g., treating the tissue with solvents to substantially remove the cellular, non-structural components therefrom. Alternatively, the matrix can be prepared from a biocompatible, *in vivo* biodegradable structural molecule, optionally formulated with suitable tissue-specific cell attachment factors. Thus, collagen, laminin, hyaluronic and/or the like, can be used, as can synthetic polymers or copolymers of polylactic acid, polybutyric acid, polyglycolic acid and the like. Currently preferred structural molecules include tissue-specific collagens. Currently preferred cell attachment factors include glycosaminoglycans and proteoglycans. Preferably collagens, glycosaminoglycans and/or proteoglyceans are used that are of the same types as those that are naturally found in dental tissues. If needed, the matrix can be treated with an agent effective for enhancing porosity thereof, so as to create a scaffold structure suitable for cell influx and attachment.

Alternatively, the morphogen can be applied in association with a carrier that maintains the morphogen substantially at the site of application, and/or enhances the controlled delivery of morphogen substantially at the site at which morphogenesis is to be induced. Such carriers also are disclosed in U.S.S.Nos. 07/971,091 (published as WO94/10203), 08/155,343 (published as WO94/06399) and 08/174,605 (published as WO94/06420). Useful carriers include compositions having a high viscosity, such as that provided by glycerol and the like, as well as carrier materials formulated from extracellular matrices and/or which contain laminin, collagen, and/or biocompatible synthetic polymers, such as polybutyric, polylactic, polyglycolic acids and copolymers thereof.

Accordingly, the present morphogens can be used to stimulate morphogenesis of new or reparative dentine in a mammalian tooth, including the formation of dentine matrix by mature, differentiated or newly formed odontoblasts, i.e., by competent cells of the tooth pulp tissue. That is, the present morphogens can stimulate proliferation, differentiation and/or phenotypic expression of mammalian cells competent to elaborate dentine matrix, including odontoblasts and/or pulp connective tissue cells. This morphogenetic activity is responsible for the formation of reparative dentine in mammalian teeth. Thus, the present morphogens can be used to increase thickness of a mammalian tooth wall; that is, to increase the thickness of mineralized tissue (dentine, enamel and/or cementum) separating viable tooth pulp tissue from the buccal environment. As a result, the present morphogens can be used to reduce the risk of tooth wall fracture, particularly at sites where the tooth wall is thin or weakened due to association with a gingival lesion site or a cavity.

Thus, the present invention can be used to seal a tooth cavity, up to and including a Stage V cavity, in a mammalian tooth, particularly a primate tooth such as a human tooth. Carious tissue preferably is ablated from the cavity site to expose a fresh surface of residual dentine therein, preferably transverse to luminae of dental canaliculi within the tooth. The residual dentine surface preferably is located up to about 1 mm, more preferably up to about 0.5 mm, still more preferably up to about 0.2 mm from the pulp chamber wall (i.e., from a mature odontoblast layer at the dentine/pulp interface). Application of a morphogen to this surface prior to or concurrently with tooth reconstruction, including filling of the site of the carious lesion with a suitable material, induces formation of reparative dentine matrix within the reconstructed tooth. In this manner, risk of fracture in the residual dentine, and subsequent treatment by root canal therapy or tooth extraction, can be avoided.

Similarly, the present invention can be used to desensitize mammalian teeth to perception of pressure and/or temperature in an individual afflicted with periodontal disease, e.g., gingivitis. Following debridement of surfaces within a gingival lesion, including removal of bacterial plaque or tartar, a morphogen is applied to an exposed dentinal surface therein, preferably in an amount effective for stimulating formation of reparative dentine apposite said surface. Reparative dentine so formed can be within or external to the pulp chamber of the treated tooth, and serves as an enhanced protective barrier between the pulp tissue and the buccal environment. Further, morphogen applied to a healthy gingival surface adjoining the lesion site promotes gingival regeneration and/or retards gingival recession.

In the above-mentioned embodiments, morphogens or morphogen stimulating agents are applied, e.g., topically or by local injection, to a tooth surface e.g., a dentinal surface. Preferably, the surface is transverse to luminae of dental canaliculi within naturally formed tooth dentine, such that fluid microcontact can be established between applied morphogen and odontoblasts or pulp tissue present within the tooth. The morphogen can be applied solubilized or otherwise dispersed (e.g., as a colloidal suspension or emulsion) in a physiologically compatible liquid vehicle, e.g., comprising physiological saline solution, or in a vehicle, e.g., comprising ethanol, that evaporates under physiological conditions to leave a morphogen residue adsorbed on the tooth surface. Alternatively, the morphogen can be sorbed on a matrix such as a biocompatible, acellular matrix suitable for sealing or filling defects in mammalian teeth, e.g., as described above. Morphogen-sealed defects can, if desired, be filled or reconstructed to restore original tooth dimensions using conventional dental reconstruction materials.

In all such embodiments, the morphogen-treated dentinal surface should be rendered essentially free of buccal microoganisms, and aseptic conditions should be maintained in the treated locus during the time period in which morphogenetic activity is induced.

Morphogens and morphogen-stimulating agents of the present invention also can be provided to periodontium and/or tooth tissues together with other molecules ("cofactors") known to have a beneficial effect in treating damaged dental tissues, particularly cofactors capable of mitigating or alleviating symptoms typically associated with dental tissue damage and/or loss. Examples of such cofactors include antiseptics such as chlorohexidine and tibezonium iodide, antibiotics, including tetracycline, aminoglycosides, macrolides, penicillins and cephalosporins, anaesthetics and analgesics, and other non-steroidal anti-inflammatory agents.

### Brief Description of the Drawings

The foregoing and other objects, features and advantages of the present invention, as well as the invention itself, will be more fully understood from the following description of preferred embodiments, when read together with the accompanying drawings, in which:

FIGURE 1 is a schematic illustration of a healthy mammalian tooth in a tooth socket.

FIGURE 2, panels 2-1 through 2-12, depicts alignment of sequences of various naturally occurring morphogens with a preferred reference sequence of human OP-1, residues 38-139 of Seq. ID No. 4. Morphogens shown in FIGURE 4 also are identified in Table I, above and in the Sequence Listing.

FIGURE 3 is a digitized video image of a typical tissue section through a primate tooth treated with morphogen, and shows morphogen-induced reparative dentine therein. Bar is 0.5 mm, original magnification 2.5x.

FIGURE 4 is a bar graph illustration of results establishing that morphogen stimulation of new or reparative dentine formation is dose dependent. In this figure, the dose applied of recombinant human OP-1 is shown in µg on the X-axis, and the surface area in mm of induced dentine is shown on the Y-axis.

FIGURE 5 is a line graph illustration of results establishing that morphogen stimulates new or reparative dentine formation under thin bridges of residual natural dentine. In this figure, equivalent amounts (e.g., 10µg) of recombinant human OP-1 were applied to residual dentine bridges of the thicknesses shown along the X-axis.

FIGURE 6 is a line graph illustration of results comparing the effects of recombinant human OP-1 to a conventional agent, Ca(OH)₂, on stimulation of new or reparative dentine under thin bridges of residual dentine. Here as well, equivalent amounts (e.g., 10µg) of recombinant human OP-1 or Ca(OH)₂ were applied as indicated to residual dentine bridges of the thicknesses shown on the X-axis.

### Detailed Description of Preferred Embodiments

It has been discovered that the morphogens described herein can stimulate tissue formation, including morphogenesis or regeneration of lost or damaged mammalian dental tissue, including dentine. The invention can be used to desensitize teeth, retard gingival recession, seal cavities, increase thickness of the tooth wall, and reduce the risk of tooth wall fracture. The invention is practiced using a morphogen or morphogen-stimulating agent, as defined herein, according to the procedures described herein.

Provided below is a description of tooth anatomy and useful morphogens, including methods for their production and formulation, as well as exemplary, non-limiting examples which (1) demonstrate the suitability of the morphogens described herein in the methods of the invention, and (2) provide assays with which to test candidate morphogens for their efficacy.

### I. Tooth Anatomy

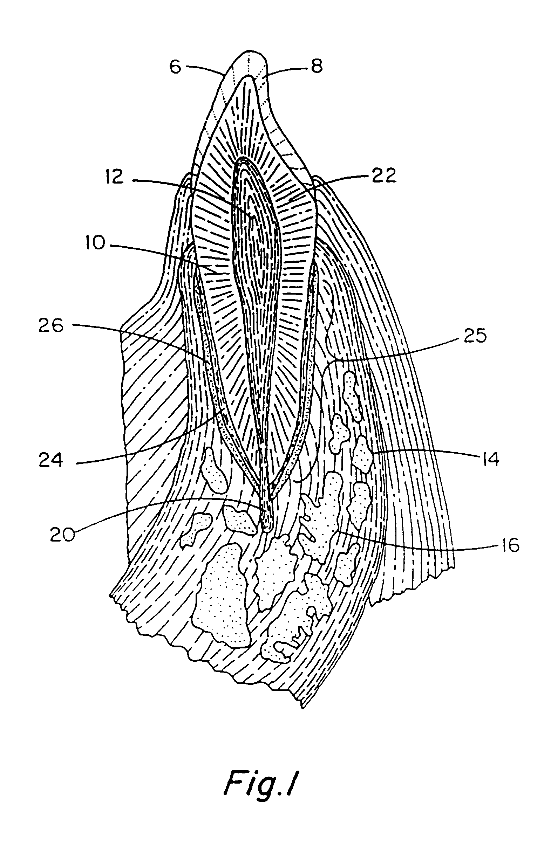

A vertical section of a mammalian tooth in the tooth socket is shown schematically in FIGURE 1. The crown 6 of the tooth is composed of enamel 8 and dentine 22. The pulp chamber 12 is seen in the interior of the crown 6 and the center of the root 10; it extends downward into the bony area 14, 16, 18 and opens by a minute orifice, the apical foramen 20, at the extremity of the root 10. The pulp chamber 12 contains dental pulp, a loose connective tissue richly supplied with blood vessels and nerves, entering the chamber through the apical foramen 20. Some of cells of the pulp tissue, i.e., odontoblasts, the precursors of dentine 22, are arranged generally as a layer on the wall of the pulp chamber 12. During development of the tooth, odontoblasts are columnar, but later, after the dentine 22 is fully formed, they become flattened and resemble osteoblasts.

The solid portion or mineralized wall of the mature tooth includes dentine 22, enamel 8, and a thin layer of cementum 24, which is disposed on the surface of the root 25. Enamel 8 is formed during development of the tooth from amyloblasts, and cementum 24 is formed from cementoblasts. In a fully developed tooth, the principal mass of the tooth comprises dentine 22, which is made up of hydroxyapatite crystals embedded in a strong meshwork of collagen fibers. The dentine includes a number of minute wavy and branching tubes called dental canaliculi, embedded in a dense homogeneous substance, the matrix. The dental canaliculi are parallel with one another and open at their inner ends into the pulp chamber 12. The dentine matrix is translucent and comprises the majority of the inorganic mass of the dentine. It includes a number of fine fibrils, which are continuous with the fibrils of the dental pulp. After the inorganic matter has been removed by steeping a tooth in weak acid, the remaining organic matter may be torn into laminae that run parallel with the pulp chamber 12 across the direction of the tubes.

The cementum 24 is disposed as a thin mineralized layer covering the tooth root. It extends from where the enamel terminates to the apex of each root, where it is usually very thick. Cementum resembles bone in structure and chemical composition in that it contains, sparingly, the lacunae and canaliculi that characterize true bone; in the thicker portions of the cementum, the lamellae and Haversian canals peculiar to bone also are found. As a result of aging, the cementum increases in thickness and the pulp chamber also becomes partially filled with a hard substance that is intermediate in structure between dentine and bone (referred to herein as "osteodentine"). It appears to be formed by a slow conversion of the dental pulp, which shrinks or even disappears.

The periodontal ligament, or periodontal membrane 26, is the layer of periodontal tissue which forms a cushion between the cementum 24 and the bone 14, 16, 18; it holds the tooth in position by suspending it in the socket (alveolus) of the jawbone. The periodontal ligament is a highly organized tissue which is formed from periodontal fibroblasts. It organizes the collagen fibers which pass directly from the bone of the jaw into the cementum.

Thus, as used herein, "tooth" refers to a natural or synthetic composition essentially defining the shape of a natural mammalian tooth, having a solid tooth body, including a crown and tooth root. "Periodontium" defines the tissues which surround the tooth in the tooth socket and includes both periodontal ligament and cementum. "Gingiva" defines the dense fibrous tissue, covered by oral mucosa, that envelopes the alveolar bone (tooth socket) processes of the upper and lower jaws, as well as the mineralized tooth wall as it emerges from the periodontium. "Viable" tissue means living, substantially healthy tissue essentially free of microorganisms and infection associated therewith. In particular, viable tissue means viable dental tissue such as enamel, dentine, tooth pulp, gingiva, cementum and periodontal ligament. "Enhancing viability" of dental tissue means improving the structural and functional integrity of living tissue, including improving the clinical status of damaged or diseased tissue. "Viable tooth" refers to an implanted natural tooth with a living tooth root. "Inhibit loss" of dental tissue, as used herein, means inhibiting damage to, and/or loss of, dental tissue and includes regenerating lost, damaged or diseased tissue and/or inhibiting additional damage thereto.

"Residual dentine" means naturally formed, healthy dentine tissue, e.g., adjoining a carious or gingival lesion, particularly a lesion from which infected dentine has been ablated and/or bacterial plaque or tartar has been debrided. Naturally formed dentine tissue comprises tubules, the dental canaliculi, extending generally radially through the dentine from the layer of odontoblasts lining the pulp chamber wall (described above in connection with FIGURE 1). Thus, a dentinal surface "transverse to the lumina of dental canaliculi" is a dentine surface disposed on any plane that intersects rather than parallels the lumina of one or more dental canaliculi. A "dentinal" surface can define a natural boundary of naturally formed dentine, or a fresh surface of dentine exposed by drilling or other dental techniques, or by fracture or chipping of the tooth wall. A treatment or stimulation "apposite" to a dentinal surface means a treatment or stimulation in juxtaposition or close proximity to the dentinal surface (e.g., separated from said surface by up to about a 1mm thickness of intervening tissue such as residual dentine). "Reparative dentine" comprises atubular dentine matrix elaborated by mature or proliferating odontoblasts or other competent cells of the pulp connective tissue, and can be formed within the pulp chamber of a mammalian tooth.

"Symptom alleviating cofactor" refers to one or more conventional pharmaceuticals which can, if desired, be included in compositions of this invention and which alleviate or mitigate one or more of the symptoms typically associated with loss of or damage to dental tissue. Exemplary cofactors include antibiotics, antiseptics, non-steroidal antiinflammatory agents, anaesthetics and analgesics.

### II. Useful Morphogens

Morphogens useful in this invention include eukaryotic proteins originally identified as osteogenic proteins (see U.S. Patent 5,011,691,), such as the OP-1, OP-2, OP-3 and CBMP2 proteins (Seq. ID Nos. 4-9, 15-22, 25 and 26), as well as amino acid sequence-related proteins such as DPP (Seq. ID No. 10, from Drosophila), Vgl (Seq. ID No. 11, from Xenopus), Vgr-1 (Seq. ID No. 12, from mouse), GDF-1 (Seq. ID Nos. 13, 30 and 31, from humans, see Lee (1991), 88 PNAS 4250-4254), 60A (Seq. ID Nos. 23 and 24, from Drosophila, see Wharton et al. (1991), 88 PNAS 9214-9218), dorsalin-1 (from chick, see Basler et al. (1993), 73 Cell 687-702 and GenBank accession number L12032) and GDF-5 (from mouse, see Storm et al. (1994), 368 Nature 639-643). Additional useful morphogens include biosynthetic morphogen constructs disclosed in U.S. Pat. No. 5,011,691, e.g., COP-1, 3-5, 7 and 16. See also U.S. Pat. No. 4,968,590.

Naturally occurring proteins identified and/or appreciated herein to be morphogens form a distinct subgroup within the loose evolutionary grouping of sequence-related proteins known as the TGFβ superfamily or supergene family. The naturally occurring morphogens share substantial amino acid sequence homology in their C-terminal regions (domains). Typically, the above-mentioned naturally occurring morphogens are translated as a precursor, having an N-terminal signal peptide sequence, typically less than about 30 residues, followed by a "pro" domain that is cleaved to yield the mature C-terminal domain. The signal peptide is cleaved rapidly upon translation, at a cleavage site that can be predicted in a given sequence using the method of Von Heijne (1986), 14 Nucleic Acids Research 4683-4691. The pro domain typically is about three times larger than the fully processed mature C-terminal domain. Herein, the "pro" form of a morphogen refers to a morphogen comprising a folded pair of polypeptides each comprising the pro and mature domains of a morphogen polypeptide. Typically, the pro form of a morphogen is more soluble than the mature form under physiological conditions. The pro form appears to be the primary form secreted from cultured mammalian cells.

Table I, below, summarizes various naturally occurring morphogens identified to date, including their nomenclature as used herein, their Seq. ID references, and publication sources for the amino acid sequences for the full length proteins not included in the Seq. Listing. Each of the generic terms set forth in Table I is intended and should be understood to embrace morphogenically active proteins expressed from nucleic acids encoding the identified sequence mentioned below and set forth in the sequence listing, or a morphogenically active fragment or precursor thereof, including functional equivalents thereof such as naturally occurring and biosynthetic variants thereof. Naturally occurring variants thereof include allelic variant forms isolated from other individuals of a single biological species, and phylogenetic counterpart (species) variant forms isolated from phylogenetically distinct biological species.

**TABLE I**

| | |
|---|---|
| "OP-1" | Refers generically to morphogenically active proteins expressed from nucleic acid encoding the human OP-1 protein disclosed in Seq. ID No. 4 ("hOP-1"), and includes at least mouse OP-1, Seq. ID No. 5 ("mOP-1"). In each of human and mouse OP-1, Seq. ID Nos. 4 and 5, the conserved seven cysteine skeleton is defined by residues 38 to 139. cDNA sequences and amino acid sequences encoded therein and corresponding to the full length proteins are provided in Seq. ID Nos. 15 and 16 (hOP1) and Seq. ID Nos. 17 and 18 (mOP1.) The mature proteins are defined by residues 293-431 (hOP1) and 292-430 (mOP1). The "pro" regions of the proteins, cleaved to yield the mature, morphogenically active proteins are defined essentially by residues 30-292 (hOP1) and residues 30-291 (mOP1). |
| | |
| "OP-2" | Refers generically to morphogenically active proteins expressed from a nucleic acid encoding the human OP-2 protein disclosed in Seq. ID No. 6 ("hOP-2"), and includes at least mouse OP-2 ("mOP-2", Seq. ID No. 7). In each of human and mouse OP-2, the conserved seven cysteine skeleton is defined by residues 38 to 139 of Seq. ID Nos. 6 and 7. cDNA sequences and amino acid sequences encoded therein and corresponding to the full length proteins are provided in Seq. ID Nos. 19 and 20 (hOP2) and Seq. ID Nos. 21 and 22 (mOP2.) The mature proteins are defined essentially by residues 264-402 (hOP2) and 261-399 (mOP2). The "pro" regions of the proteins, cleaved to yield the mature, morphogenically active proteins are defined essentially by residues 18-263 (hOP2) and residues 18-260 (mOP1). |
| | |
| "OP-3" | Refers generically to morphogenically active proteins expressed from a nucleic acid encoding the mouse OP-3 protein disclosed in Seq. ID No. 26 ("mOP-3"). The conserved seven cysteine domain is defined by residues 298 to 399 of Seq. ID No. 26, which shares greater than 79% amino acid identity with the corresponding mOP-2 and hOP-2 sequences, and greater than 66% identity with the corresponding OP-1 sequences. A cDNA sequence encoding the above-mentioned amino acid sequence is provided in Seq. ID No. 25. OP-3 is unique among the morphogens identified to date in that the residue at position 9 in the conserved seven cysteine domain (e.g., residue 315 of Seq. ID No. 26) is a serine, whereas other morphogens typically have a tryptophan at this location. |
| | |
| "CBMP2" | Refers generically to morphogenically active proteins expressed from a nucleic acid encoding the CBMP2 proteins, including at least human CBMP2A ("CBMP2A(fx)", Seq ID No. 8) and human CBMP2B ("CBMP2B(fx)", Seq. ID No. 9). The amino acid sequence for the full length proteins, referred to in the literature as BMP2A and BMP2B, or BMP2 and BMP4, appear in Wozney, et al. (1988), 242 Science 1528-1534. The pro domain for BMP2 (BMP2A) likely includes residues 25-248; the mature protein, residues 249-396. The pro domain for BMP4 (BMP2B) likely includes residues 25-256; the mature protein, residues 257-408. |
| | |
| "DPP(fx)" | refers to proteins encoded by the Drosophila DPP gene and defining the conserved seven cysteine skeleton (Seq. ID No. 10). The amino acid sequence for the full length protein appears in Padgett, et al (1987), 325 Nature 81-84. The pro domain likely extends from the signal peptide cleavage site to residue 456; the mature protein likely is defined by residues 457-588. |
| | |
| "Vgl(fx)" | refers to proteins encoded by the Xenopus Vg1 gene and defining the conserved seven cysteine skeleton (Seq. ID No. 11). The amino acid sequence for the full length protein appears in Weeks (1987), 51 Cell 861-867. The prodomain likely extends from the signal peptide cleavage site to residue 246; the mature protein likely is defined by residues 247-360. |
| | |
| "Vgr-1(fx)" | refers to proteins encoded by the murine Vgr-1 gene and defining the conserved seven cysteine skeleton (Seq. ID No. 12). The amino acid sequence for the full length protein appears in Lyons, et al, (1989), 86 PNAS 4554-4558. The prodomain likely extends from the signal peptide cleavage site to residue 299; the mature protein likely is defined by residues 300-438. |
| | |
| "GDF-1(fx)" | refers to proteins encoded by the human GDF-1 gene and defining the conserved seven cysteine skeleton (Seq. ID No. 13). The cDNA and encoded amino sequence for the full length protein are provided in Seq. ID. Nos. 30 and 31. The prodomain likely extends from the signal peptide cleavage site to residue 214; the mature protein likely is defined by residues 215-372. |
| | |
| "60A" | refers generically to morphogenically active proteins expressed from nucleic acid (e.g., the Drosophila 60A gene) encoding 60A protein or morphogenically active fragments thereof (see Seq. ID Nos. 23 and 24 wherein the cDNA and encoded amino acid sequence for the full length protein are provided). "60A(fx)" refers to the protein sequences defining the conserved seven cysteine skeleton (residues 354 to 455 of Seq. ID No. 24.) The prodomain likely extends from the signal peptide cleavage site to residue 324; the mature protein likely is defined by residues 325-455. The 60A protein is considered likely herein to be a phylogenetic counterpart variant of the human and mouse OP-1 genes; Sampath et al. (1993), 90 PNAS 6004-6008. |
| | |
| "BMP3(fx)" | refers to proteins encoded by the human BMP3 gene and defining the conserved seven cysteine skeleton (Seq. ID No. 26). The amino acid sequence for the full length protein appears in Wozney et al. (1988), 242 Science 1528-1534. The pro domain likely extends from the signal peptide cleavage site to residue 290; the mature protein likely is defined by residues 291-472. |
| | |
| "BMP5(fx)" | refers to proteins encoded by the human BMP5 gene and defining the conserved seven cysteine skeleton (Seq. ID No. 27). The amino acid sequence for the full length protein appears in Celeste, et al. (1991), 87 PNAS 9843-9847. The pro domain likely extends from the signal peptide cleavage site to residue 316; the mature protein likely is defined by residues 317-454. |
| | |
| "BMP6(fx)" | refers to proteins encoded by the human BMP6 gene and defining the conserved seven cysteine skeleton (Seq. ID No. 28). The amino acid sequence for the full length protein appears in Celeste, et al. (1990), 87 PNAS 9843-5847. The pro domain likely includes extends from the signal peptide cleavage site to residue 374; the mature sequence likely includes residues 375-513. |

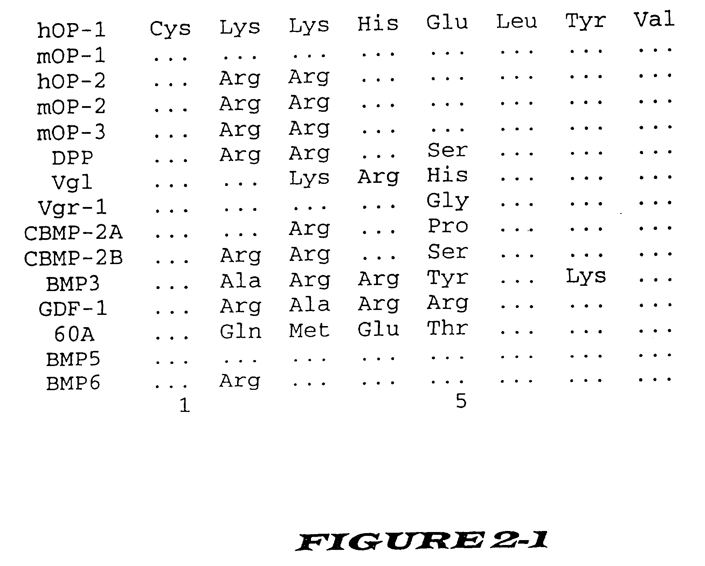

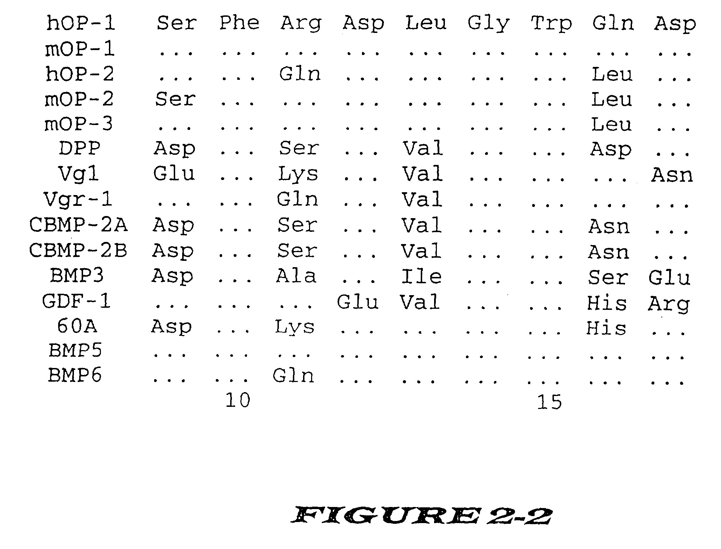

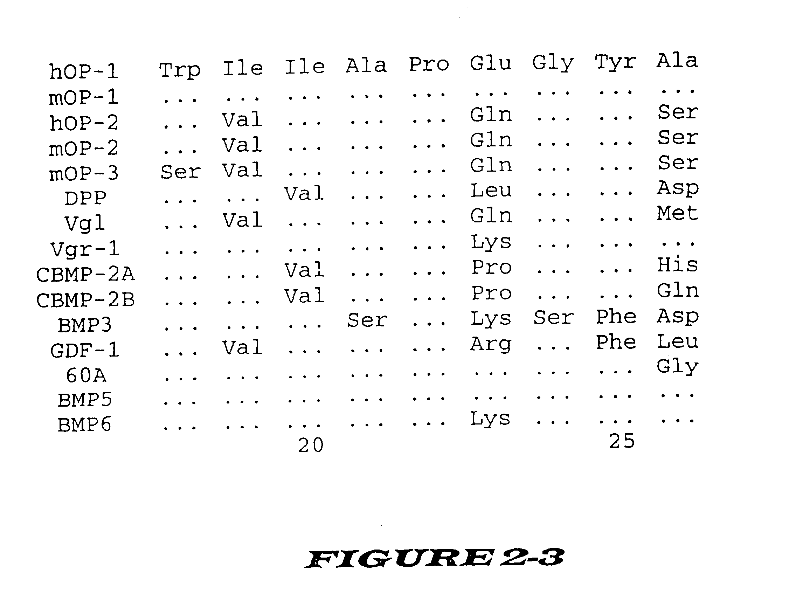

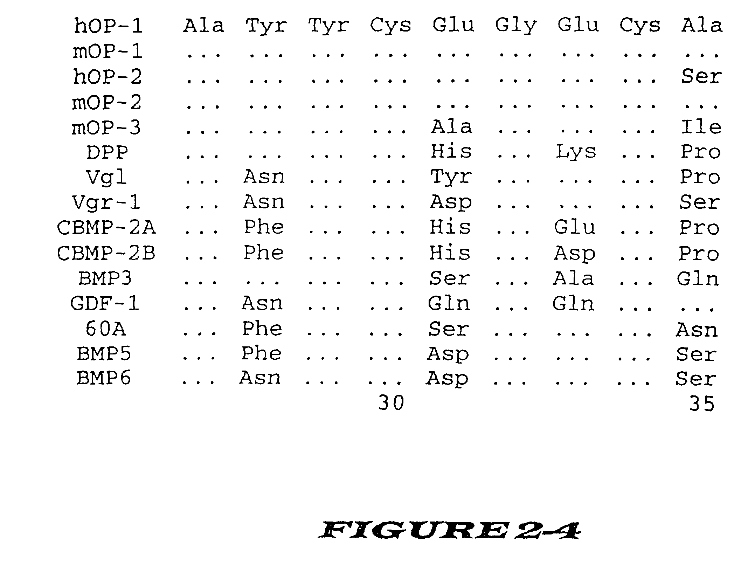

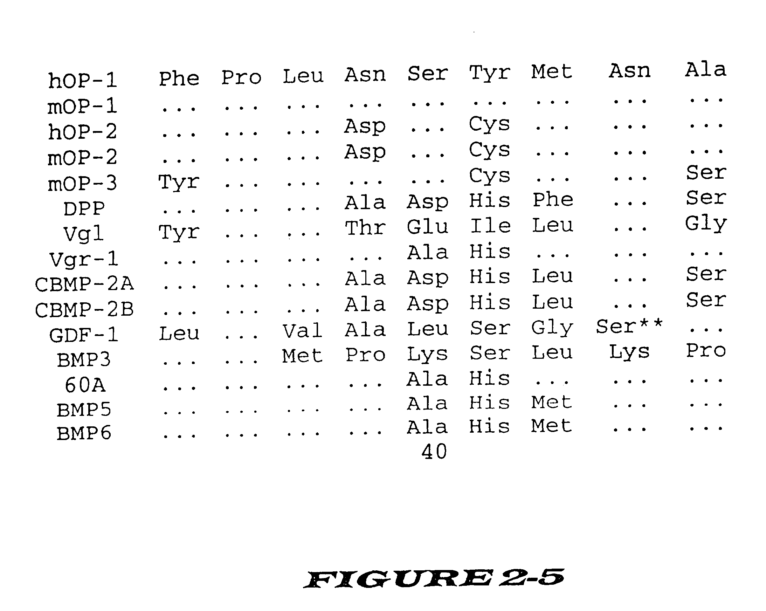

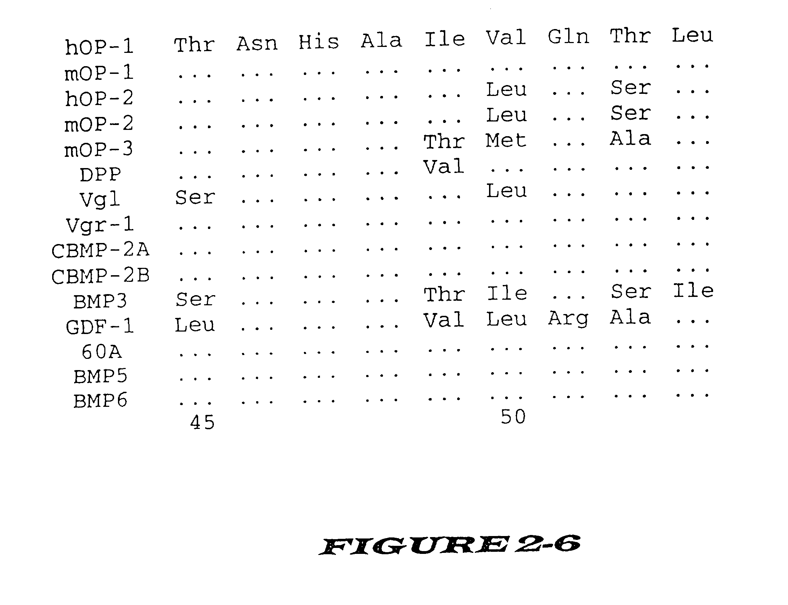

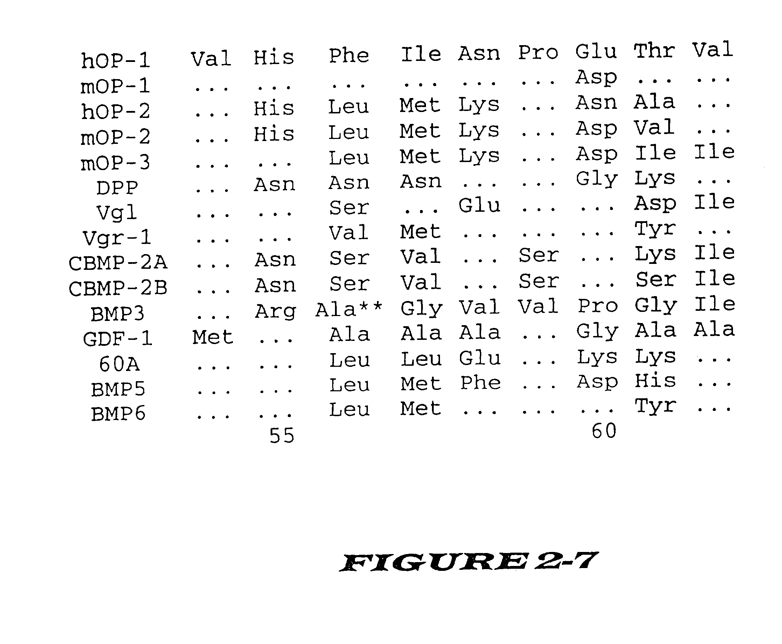

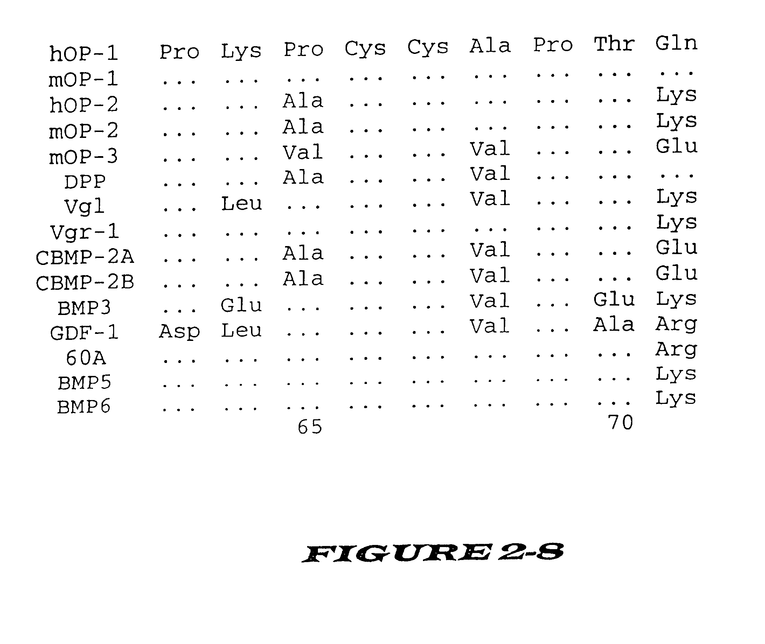

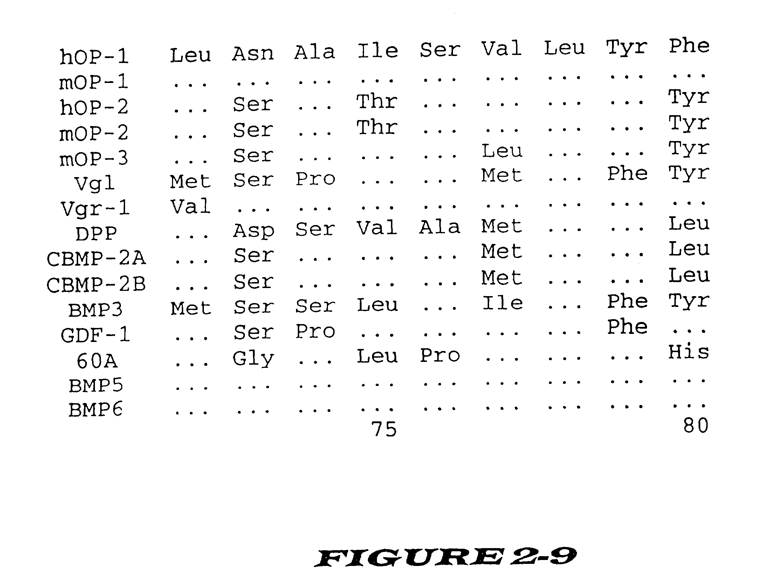

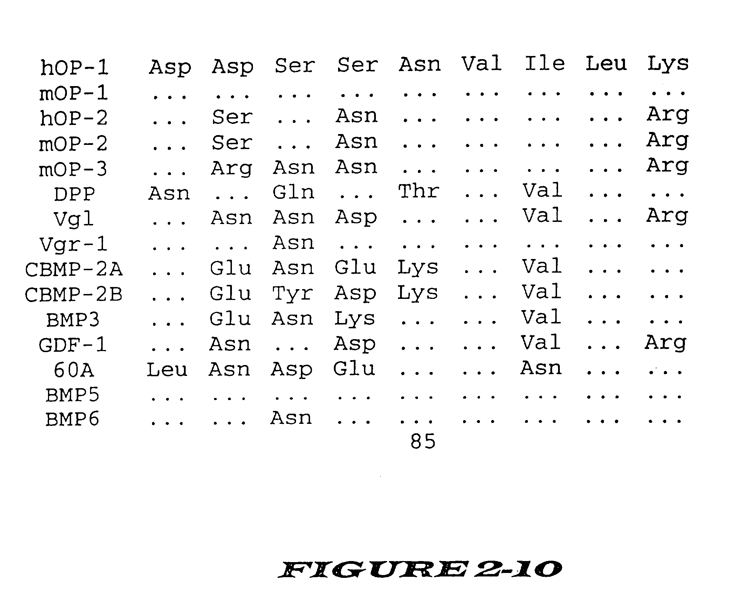

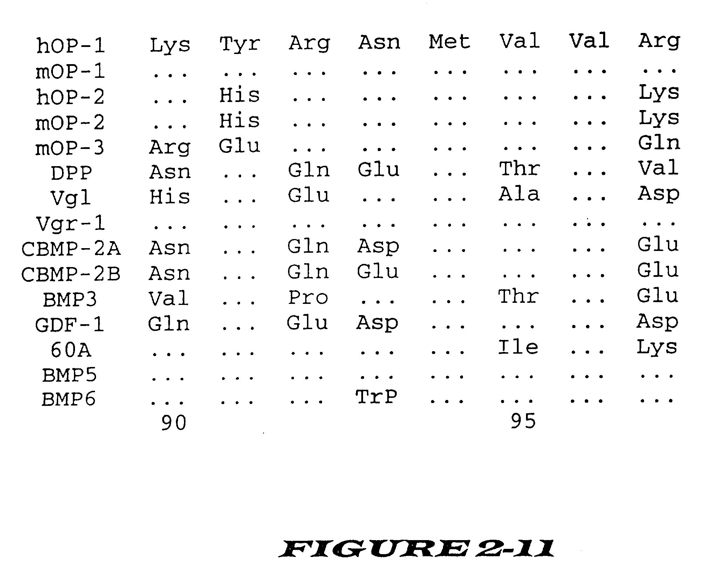

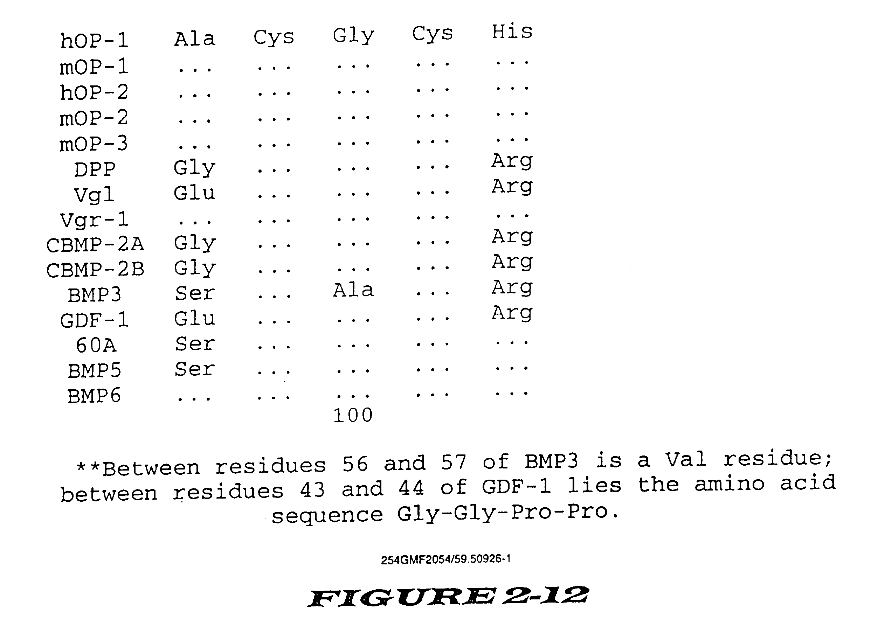

As shown in FIGURE 2, the OP-2 and OP-3 proteins have an additional cysteine residue in the conserved C-terminal region (e.g., see residue 41 of Seq. ID Nos. 6 and 7), in addition to the conserved cysteine skeleton or domain in common with the other known proteins in this family. The GDF-1 protein has a four amino acid insert within the conserved skeleton (residues 44-47 of Seq. ID No. 13) but this insert likely does not interfere with the relationship of the cysteines in the folded structure. Further, the CBMP2 proteins are missing one amino acid residue within the cysteine skeleton. Thus, these morphogen polypeptides illustrate principles of alignment used herein with respect to the preferred reference morphogen sequence of human OP-1, residues 38-139 of Seq. ID No. 4.

In certain preferred embodiments, morphogens useful herein include those in which the amino acid sequences of morphogen polypeptides comprise a sequence sharing at least 70% amino acid sequence homology or "similarity", and preferably 80% homology or similarity with a reference morphogen selected from the foregoing naturally occurring morphogens. Preferably, the reference morphogen is human OP-1, and the reference sequence thereof is the C-terminal seven cysteine domain present in morphogenically active forms of human OP-1, residues 38-139 of Seq. ID No. 4. Morphogens useful herein accordingly include allelic, phylogenetic counterpart and other variants of the preferred reference sequence, whether naturally-occurring or biosynthetically produced (e.g., including "muteins" or "mutant proteins"), as well as novel members of the morphogenic family of proteins including the morphogens set forth and identified above, e.g., in Table I. Certain particularly preferred morphogen polypeptides share at least 60% amino acid identity with the preferred reference sequence of human OP-1, still more preferably at least 65% amino acid identity therewith.

In other preferred embodiments, the family of morphogen polypeptides useful in the present invention, and members thereof, are defined by a generic amino acid sequence. For example, Generic Sequence 7 (Seq. ID No. 1) and Generic Sequence 8 (Seq. ID No. 2) disclosed below, accommodate the homologies shared among preferred morphogen protein family members identified to date, including at least OP-1, OP-2, OP-3, CBMP2A, CBMP2B, BMP3, 60A, DPP, Vg1, BMP5, BMP6, Vgr-1, and GDF-1 (Seq. ID Nos. 4-15, 24, and 26-29). The amino acid sequences for these proteins are described herein (see Sequence Listing) and/or in the art, as summarized above. The generic sequences include both the amino acid identity shared by these sequences in the C-terminal domain, defined by the six and seven cysteine skeletons (Generic Sequences 7 and 8, respectively), as well as alternative residues for the variable positions within the sequence. The generic sequences provide an appropriate cysteine skeleton where inter- or intramolecular disulfide bonds can form, and contain certain critical amino acids likely to influence the tertiary structure of the folded proteins. In addition, the generic sequences allow for an additional cysteine at position 41 (Generic Sequence 7) or position 46 (Generic Sequence 8), thereby encompassing the morphogenically active sequences of OP-2 and OP-3. wherein each Xaa independently is selected from a group of one or more specified amino acids defined as follows: "Res." means "residue" and Xaa at res.2 = (Tyr or Lys); Xaa at res.3 = Val or Ile); Xaa at res.4 = (Ser, Asp or Glu); Xaa at res.6 = (Arg, Gln, Ser, Lys or Ala); Xaa at res.7 = (Asp or Glu); Xaa at res.8 = (Leu, Val or Ile); Xaa at res. 11 = (Gln, Leu, Asp, His, Asn or Ser); Xaa at res.12 = (Asp, Arg, Asn or Glu); Xaa at res. 13 = (Trp or Ser); Xaa at res.14 = (Ile or Val); Xaa at res.15 = (Ile or Val); Xaa at res.16 (Ala or Ser); Xaa at res.18 = (Glu, Gln, Leu, Lys, Pro or Arg); Xaa at res.19 = (Gly or Ser); Xaa at res.20 = (Tyr or Phe); Xaa at res.21 = (Ala, Ser, Asp, Met, His, Gln, Leu or Gly); Xaa at res.23 = (Tyr, Asn or Phe); Xaa at res.26 = (Glu, His, Tyr, Asp, Gln, Ala or Ser); Xaa at res.28 = (Glu, Lys, Asp, Gln or Ala); Xaa at res.30 = (Ala, Ser, Pro, Gln, Ile or Asn); Xaa at res.31 = (Phe, Leu or Tyr); Xaa at res.33 = (Leu, Val or Met); Xaa at res.34 = (Asn, Asp, Ala, Thr or Pro); Xaa at res.35 = (Ser, Asp, Glu, Leu, Ala or Lys); Xaa at res.36 = (Tyr, Cys, His, Ser or Ile); Xaa at res.37 = (Met, Phe, Gly or Leu); Xaa at res.38 = (Asn, Ser or Lys); Xaa at res.39 = (Ala, Ser, Gly or Pro); Xaa at res.40 = (Thr, Leu or Ser); Xaa at res.44 = (Ile, Val or Thr); Xaa at res.45 = (Val, Leu, Met or Ile); Xaa at res.46 = (Gln or Arg); Xaa at res.47 = (Thr, Ala or Ser); Xaa at res.48 = (Leu or Ile); Xaa at res.49 = (Val or Met); Xaa at res.50 = (His, Asn or Arg); Xaa at res.51 = (Phe, Leu, Asn, Ser, Ala or Val); Xaa at res.52 = (Ile, Met, Asn, Ala, Val, Gly or Leu); Xaa at res.53 = (Asn, Lys, Ala, Glu, Gly or Phe); Xaa at res.54 = (Pro, Ser or Val); Xaa at res.55 = (Glu, Asp, Asn, Gly, Val, Pro or Lys); Xaa at res.56 = (Thr, Ala, Val, Lys, Asp, Tyr, Ser, Gly, Ile or His); Xaa at res.57 = (Val, Ala or Ile); Xaa at res.58 = (Pro or Asp); Xaa at res.59 = (Lys, Leu or Glu); Xaa at res.60 = (Pro, Val or Ala); Xaa at res.63 = (Ala or Val); Xaa at res.65 = (Thr, Ala or Glu); Xaa at res.66 = (Gln, Lys, Arg or Glu); Xaa at res.67 = (Leu, Met or Val); Xaa at res.68 = (Asn, Ser, Asp or Gly); Xaa at res.69 = (Ala, Pro or Ser); Xaa at res.70 = (Ile, Thr, Val or Leu); Xaa at res.71 = (Ser, Ala or Pro); Xaa at res.72 = (Val, Leu, Met or Ile); Xaa at res.74 = (Tyr or Phe); Xaa at res.75 = (Phe, Tyr, Leu or His); Xaa at res.76 = (Asp, Asn or Leu); Xaa at res.77 = (Asp, Glu, Asn, Arg or Ser); Xaa at res.78 = (Ser, Gln, Asn, Tyr or Asp); Xaa at res.79 = (Ser, Asn, Asp, Glu or Lys); Xaa at res.80 = (Asn, Thr or Lys); Xaa at res.82 = (Ile, Val or Asn); Xaa at res.84 = (Lys or Arg); Xaa at res.85 = (Lys, Asn, Gln, His, Arg or Val); Xaa at res.86 = (Tyr, Glu or His); Xaa at res.87 = (Arg, Gln, Glu or Pro); Xaa at res.88 = (Asn, Glu, Trp or Asp); Xaa at res.90 = (Val, Thr, Ala or Ile); Xaa at res.92 = (Arg, Lys, Val, Asp, Gln or Glu); Xaa at res.93 = (Ala, Gly, Glu or Ser); Xaa at res.95 = (Gly or Ala) and Xaa at res.97 = (His or Arg).

Generic Sequence 8 (Seq. ID No. 2) includes all of Generic Sequence 7 and in addition includes the following sequence (Seq. ID No. 14) at its N-terminus: Accordingly, beginning with residue 7, each "Xaa" in Generic Seq. 8 is a specified amino acid defined as for Generic Seq. 7, with the distinction that each residue number described for Generic Sequence 7 is shifted by five in Generic Seq. 8. Thus, "Xaa at res.2 =(Tyr or Lys)" in Gen. Seq. 7 refers to Xaa at res. 7 in Generic Seq. 8. In Generic Seq. 8, Xaa at res.2 = (Lys, Arg, Ala or Gln); Xaa at res.3 = (Lys, Arg or Met); Xaa at res.4 = (His, Arg or Gln); and Xaa at res.5 = (Glu, Ser, His, Gly, Arg, Pro, Thr, or Tyr).

As noted above, certain currently preferred morphogen polypeptide sequences useful in this invention have greater than 60% identity, preferably greater than 65% identity, with the amino acid sequence defining the preferred reference sequence of hOP-1. These particularly preferred sequences include allelic and phylogenetic counterpart variants of the OP-1 and OP-2 proteins, including the Drosophila 60A protein. Accordingly, in certain particularly preferred embodiments, useful morphogens include active proteins comprising pairs of polypeptide chains within the generic amino acid sequence herein referred to as "OPX" (Seq. ID No. 3), which defines the seven cysteine skeleton and accommodates the homologies between several identified variants of OP1 and OP2. As described therein, each Xaa at a given position independently is selected from the residues occurring at the corresponding position in the C-terminal sequence of mouse or human OP1 or OP2 (see Seq. ID Nos. 4-7 and/or Seq. ID Nos. 15-22).

In still other preferred embodiments, useful morphogen polypeptides have amino acid sequences comprising a sequence encoded by nucleic acid that hybridizes, under stringent hybridization conditions, to DNA or RNA encoding reference morphogen sequences, e.g., C-terminal sequences defining the conserved seven cysteine domains of OP1 or OP2, e.g., nucleotides 1036-1341 and nucleotides 1390-1695 of Seq. ID No. 15 and 19, respectively. As used herein, stringent hybridization conditions are defined as hybridization according to known techniques in 40% formamide, 5 X SSPE, 5 X Denhardt's Solution, and 0.1% SDS at 37°C overnight, and washing in 0.1 X SSPE, 0.1% SDS at 50°C.

As noted above, morphogens useful in the present invention generally are dimeric proteins comprising a folded pair of the above polypeptides. Morphogens are inactive when reduced, but are active as oxidized homodimers and when oxidized in combination with other morphogens of this invention to produce heterodimers. Thus, members of a folded pair of morphogen polypeptides in a morphogenically active protein can be selected independently from any of the specific morphogen polypeptides mentioned above.

The morphogens useful in the methods, compositions and devices of this invention include proteins comprising any of the polypeptide chains described above, whether isolated from naturally-occurring sources, or produced by recombinant DNA or other synthetic techniques, and includes allelic and phylogenetic counterpart variants of these proteins, as well as biosynthetic variants (muteins) thereof, and various truncated and fusion constructs. Deletion or addition mutants also are envisioned to be active, including those which may alter the conserved C-terminal six or seven cysteine domain, provided that the alteration does not functionally disrupt the relationship of these cysteines in the folded structure. Accordingly, such active forms are considered the equivalent of the specifically described constructs disclosed herein. The proteins may include forms having varying glycosylation patterns, varying N-termini, a family of related proteins having regions of amino acid sequence homology, and active truncated or mutated forms of native or biosynthetic proteins, produced by expression of recombinant DNA in host cells.

The morphogenic proteins can be expressed from intact or truncated cDNA or from synthetic DNAs in procaryotic or eucaryotic host cells, and purified, cleaved, refolded, and dimerized to form morphogenically active compositions. Currently preferred host cells include *E. coli* or mammalian cells, such as CHO, COS or BSC cells. A detailed description of the morphogens useful in the methods, compositions and devices of this invention is disclosed in copending U.S. Serial Nos. 07/752,764 (published as WO92/15323), filed August 30, 1991, and 07/667,724 (abandoned in favor of 07/752,764), filed March 11, 1991.

Thus, in view of this disclosure, skilled genetic engineers can isolate genes from cDNA or genomic libraries of various different biological species, which encode appropriate amino acid sequences, or construct DNAs from oligonucleotides, and then can express them in various types of host cells, including both procaryotes and eucaryotes, to produce large quantities of active proteins capable of stimulating the morphogenesis of, and/or inhibiting damage to, mammalian dental tissues.

As noted above, a protein is morphogenic herein generally if it induces the developmental cascade of cellular and molecular events that culminate in the formation of new, organ-specific tissue. Preferably, a morphogen comprises a pair of polypeptides having a sequence that corresponds to or is functionally equivalent to at least the conserved C-terminal six or seven cysteine skeleton of human OP-1, included in Seq. ID No. 4. The morphogens generally are competent to induce all of the following biological functions in a morphogenically permissive environment: stimulating proliferation of progenitor cells; stimulating the differentiation of progenitor cells; stimulating the proliferation of differentiated cells; and supporting the growth and maintenance of differentiated cells. Details of how the morphogens useful in this invention first were identified, as well as a description on how to make, use and test them for morphogenic activity are disclosed in U.S.S.Nos. 07/752,764 (published as WO92/15323) and 07/667,274 (abandoned in favor of 07/752,764). As disclosed therein, the morphogens can be purified from naturally-sourced material or recombinantly produced from procaryotic or eucaryotic host cells, using the genetic sequences disclosed therein. Alternatively, novel morphogenic sequences can be identified following the procedures disclosed therein.

Exemplary useful morphogens include naturally derived proteins comprising a pair of polypeptides, the amino acid sequences of which comprise one or more of the sequences disclosed in the Sequence Listing and FIGURE 2. Other useful sequences include those of the naturally derived morphogens dorsalin-1 and GDF-5, discussed herein in connection with Table I, as well as biosynthetic constructs disclosed in U.S. Pat. 5,011,691 (e.g., COP-1, COP-3, COP-4, COP-5, COP-7, and COP-16).

Accordingly, certain preferred morphogens useful in the methods and compositions of this invention can be described as morphogenically active proteins having amino acid sequences sharing 70% or, preferably, 80% homology (similarity) with a reference morphogen sequence described above, e.g., residues 38-139 of Seq. ID No. 4, where "homology" is as defined herein above. Alternatively, in other preferred embodiments, morphogens useful in the methods and compositions disclosed herein fall within the family of polypeptides described by Generic Sequence 7, Seq. ID No. 1, more preferably by Generic Sequence 8, Seq. ID No. 2.

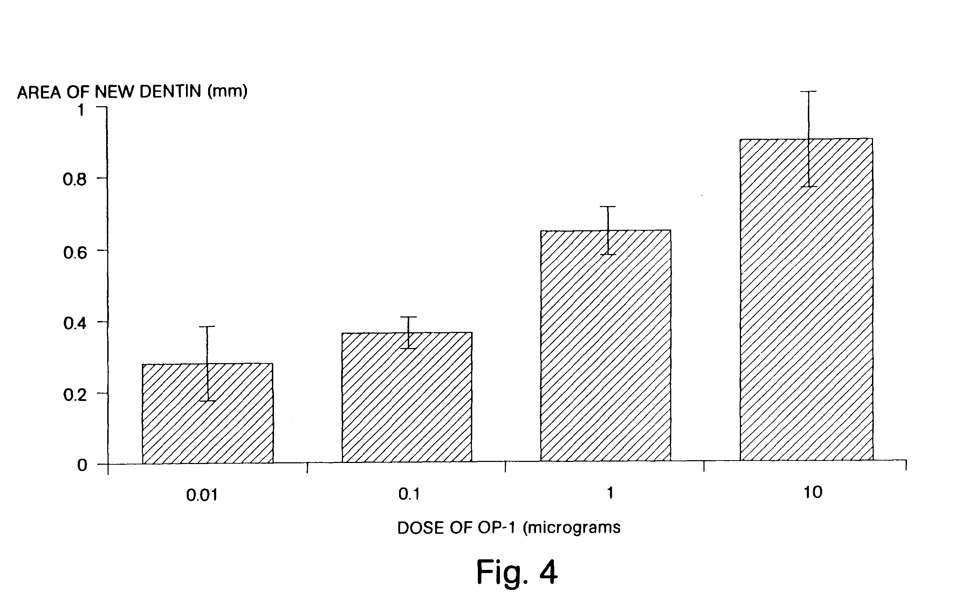

FIGURE 2 herein sets forth an alignment of the amino acid sequences of the active regions of naturally occurring proteins that have been identified or appreciated herein as morphogens, including human OP-1 (hOP-1, Seq. ID Nos. 4 and 15-16), mouse OP-1 (mOP-1, Seq. ID Nos. 5 and 17-18), human and mouse OP-2 (Seq. ID Nos. 6, 7, and 19-22), mouse OP-3 (Seq. ID Nos. 25-26), CBMP2A (Seq. ID No. 8), CBMP2B (Seq. ID No. 9), BMP3 (Seq. ID No. 27), DPP (from Drosophila, Seq. ID No. 10), Vg1, (from Xenopus, Seq. ID No. 11), Vgr-1 (from mouse, Seq. ID No. 12), GDF-1 (from mouse and/or human, Seq. ID Nos. 13, 30 and 31), 60A protein (from Drosophila, Seq. ID Nos. 23 and 24), BMP5 (Seq. ID No. 28) and BMP6 (Seq. ID No. 29). The sequences are aligned essentially following the method of Needleman et al. (1970), 48 J. Mol. Biol., 443-453, calculated using the Align Program (DNAstar, Inc). In FIGURE 2, three dots indicates that the amino acid in that position is the same as the corresponding amino acid in hOP-1. Three dashes indicates that no amino acid is present in that position, and are included for purposes of illustrating homologies. For example, amino acid residue 60 of CBMP-2A and CBMP-2B is "missing". Of course, both of these amino acid sequences in this region comprise Asn-Ser (residues 58, 59), with CBMP-2A then comprising Lys and Ile, whereas CBMP-2B comprises Ser and Ile. FIGURE 2 also illustrates the handling of insertions in the morphogen amino acid sequence: between residues 56 and 57 of BMP3 is an inserted Val residue; between residues 43 and 44 of GDF-1 is inserted the amino acid sequence, Gly-Gly-Pro-Pro. Such deviations from the reference morphogen sequence are ignored for purposes of calculating the defined relationship between, e.g., GDF-1 and hOP-1. As is apparent from the amino acid sequence comparisons set forth in FIGURE 4, significant amino acid changes can be made from the reference sequence while retaining morphogenic activity. For example, while the GDF-1 protein sequence depicted in FIGURE 4 shares only about 50% amino acid identity with the hOP1 sequence described therein, the GDF-1 sequence shares greater than 70% amino acid sequence homology (or "similarity") with the hOP1 sequence, where "homology" or "similarity" includes allowed conservative amino acid substitutions within the aligned sequence, e.g., as defined by Dayhoffet al., (1979) 5 Atlas of Protein Sequence and Structure Suppl. 3, pp.345-362, (M.O. Dayhoff, ed., Nat'1 BioMed. Res. Fd'n, Washington D.C.).

The currently most preferred protein sequences useful as morphogens in this invention include those having greater than 60% identity, preferably greater than 65% identity, with the amino acid sequence defining the conserved six or seven cysteine skeleton of hOP1 (e.g., residues 43-139 or 38-139 of Seq. ID No. 5). These most preferred sequences include both allelic and phylogenetic counterpart variants of the OP-1 and OP-2 proteins, including the Drosophila 60A protein. Accordingly, in still another preferred aspect, the invention includes morphogens comprising species of polypeptide chains having the generic amino acid sequence referred to herein as "OPX", which defines the seven cysteine domain and accommodates the identities and homologies between the various identified OP1 and OP2 proteins. OPX is presented in Seq. ID No. 3. As described therein, each Xaa at a given position independently is selected from the residues occurring at the corresponding position in the C-terminal sequence of mouse or human OP1 or OP2 (see FIGURE 2 and Seq. ID Nos. 4-7 and/or Seq. ID Nos. 15-22).

Alternatively, an effective amount of an agent competent to stimulate endogenous morphogen levels in a mammal may be administered by any of the routes described herein. For example, an agent competent to stimulate morphogen production and/or secretion from periodontal tissue, gingiva, alveolar bone tissue in the tooth socket, or pulp tissue, may be provided to a mammal, e.g., by direct administration of the morphogen-stimulating agent to dental tissue. Alternatively, the morphogen-stimulating agent may induce morphogen expression and/or secretion at a distant site (e.g., at a tissue locus other than dental tissue), with the expressed morphogen circulating to dental tissue competent to take up the morphogen and respond thereto. A method for identifying and testing agents competent to modulate the levels of endogenous morphogens in a given tissue is described in detail in prior related U.S.S.Nos. 07/938,021 (published as WO93/05172) and 07/752,859 (published as WO93/05751. Briefly, candidate compounds can be identified and tested by incubation *in vitro* with a test tissue or cells thereof, or a cultured cell line derived therefrom, for a time sufficient to allow the compound to affect the production, i.e., the expression and/or secretion, of a morphogen produced by the cells of that tissue. Here, suitable tissue, or cultured cells of a suitable tissue, preferably can be selected from renal epithelium, dental fibroblasts, cementoblasts, odontoblasts and osteoblasts.

### III. Formulations and Methods for Administration

The morphogens can be provided to a dental tissue surface, e.g., a dentinal or gingival surface, by any suitable means. Preferably, the morphogen, or a morphogen-stimulating agent, is provided directly to the tissue surface by topical administration. Alternatively, the morphogen can be provided to the tissue by, for example, local injection. While not currently preferred, systemic injection also may be a viable administration route under certain circumstances, such as to treat advanced or chronic disease states, or as a preventive measure in individuals at extreme risk of disease. A detailed description of considerations for systemic administration, including oral and parenteral administration, is disclosed, for example in U.S.S.No. 08/165,511 (published as WO93/04692).

Where the morphogen is provided directly to a dentinal surface, it can be administered as part of a biocompatible formulation that may be a liquid, gel or solid. For example, it can be dispersed in an aqueous medium that does not impair the mammal's physiologic fluid or salt balance. The aqueous medium for the morphogen thus may comprise normal physiologic saline (0.85% or 0.15 M NaCl), pH 7.0-7.4. The aqueous morphogen formulation can be made, for example, by dissolving the morphogen in 50% ethanol containing acetonitrile in 0.1% trifluoroacetic acid (TFA) or 0.1% HCl, or equivalent solvents. One volume of the resultant solution then is added, for example, to ten volumes of phosphate buffered saline (PBS), which further can include 0.1-0.2% human serum albumin (HSA) or another acceptable carrier protein. The resultant solution preferably is vortexed extensively. The morphogen further can be dispersed in and associated with a carrier capable of maintaining the morphogen at the administered locus. Useful formulations for some embodiments herein include viscous compositions and evaporative compositions. Biocompatible compositions that increase viscosity of the formulation include glycerol, polyalkylene glycols such as polyethylene glycol, oils of vegetable origin, hydrogenated naphthalenes, and the like. Useful evaporative compositions include physiologically acceptable, e.g., biologically inert, liquids that evaporate under physiological conditions so as to leave a residue of morphogen on the tissue surface. Evaporative liquids include low molecular weight organic or inorganic compounds such as water, ethanol, isopropanol, acetic acid and the like that do not adversely affect tissue function or tissue structural integrity prior to evaporating.

The formulation also can include an *in vivo* bioresorbable carrier material that acts as a controlled release delivery vehicle. Useful carriers can include biocompatible, preferably biodegradable structural components from, e.g., an extracellular matrix, such as collagen, laminin, hyaluronic acid, and the like, or polymeric materials, such as polylactic, polybutyric and polyglycolic acids. The carrier also can comprise an acellular tissue matrix, substantially depleted in nonstructural components, such as a demineralized, guanidine-extracted bone, dentine, periodontal ligament or cementum matrix. Details for preparing such matrices are disclosed in U.S.S.N. 07/752,764 (published as WO92/15323). Other useful controlled release carriers in which the morphogen can be dispersed are described in U.S. Pat. Nos. 4,975,526 and 4,919,939. Such carriers are envisioned to be particularly useful where the morphogen is used to seal a cavity.

Preferably, morphogen compositions that are viscous, evaporative or comprise a bioresorbable carrier are suitable for topical administration to a dentinal or gingival surface, and can inhibit recession or enhance regenerative healing of gingival tissue as well as stimulating morphogenesis of dentine tissue.

If desired, a given morphogen can be made more soluble in the aqueous composition by association with a suitable molecule. For example, the pro form of a morphogenic protein typically is more soluble or dispersible in physiological solutions than the corresponding mature form. In fact, endogenous morphogens are thought to be transported (e.g., secreted and circulated) in the mammalian body in this form. This soluble form of the protein can be obtained from culture medium of morphogen-secreting mammalian cells e.g., cells transfected with nucleic acid encoding and competent to express the morphogen. Alternatively, a soluble species can be formulated by complexing the mature dimer (or an active fragment thereof) with a morphogen pro domain or a solubility-enhancing fragment thereof (described more fully below). Other components, including various serum proteins, also can be useful to enhance morphogen solubility.

Finally, the morphogens or morphogen-stimulating agents provided herein can be administered alone or in combination with other molecules, particularly symptom alleviating cofactors. Useful pharmaceutical cofactors for mitigating symptoms associated with damage to dental tissue include antiseptics, antibiotics, anaesthetics and analgesics. Preferred antiseptics for use in the present system include chlorhexidine and tibezonium iodide; preferred antibiotics include tetracycline, aminoglycosides such as neomycin, gentamycin, kanamycin, tobramycin, netilmicin, sisomicin, amicamycin, their sulfates or other derivatives, macrolides such as erythromycin, its salts and other derivatives, spiramycin, josamicin or miocamicin, penicillins such as ampicillin, amoxicillin and the like, and cephalosporins, for example, cefaclor, cefadroxil, cefazolin, cefoperazone, cefotaxime, cephalothin, cefalexin, ceforanide, cefonicide or ceftriaxone. Preferred anaesthetics/analgesics include amide-type local anaesthetics such as lidocaine, mepivacaine, pyrrocaine, bupivacaine, prilocaine, etidocaine, or other widely used anaesthetics such as procaine.

Other cofactors include non-steroidal anti-inflammatory agents. However, the morphogens described herein themselves can modulate the body's inflammatory/immune response to an initial tissue injury. Specifically, and as described in detail in U.S.S.N. 08/165,511 (published as WO93/04692), in the presence of a morphogen, proinflammatory effector cells induced to migrate to a site of tissue injury do not become significantly activated Without being limited to any given theory, it is thought that, in the presence of the morphogen, damaged tissue is induced to undergo a recapitulation of tissue morphogenesis, where progenitor cells are induced to proliferate and differentiate in a tissue-specific manner, and new, functional, organized tissue is formed to replace the damaged or lost tissue, rather than disorganized, fibrous scar tissue.

The formulated compositions contain therapeutically effective amounts of the morphogen, e.g., amounts which provide appropriate concentrations of the morphogen to the dentinal surface for a time sufficient to stimulate morphogenesis of dentine and/or production of dentine matrix apposite thereto. As will be appreciated by those skilled in the art, the concentration of the compounds described in a therapeutic composition of the present invention will vary depending upon a number of factors, including the biological efficacy of the selected morphogen, the chemical characteristics (e.g., hydrophobicity) of the compounds employed, the formulation of the compound excipients, the administration route, and the treatment envisioned, including whether reparative dentine is to be induced at a distance, e.g., up to about 0.5mm, from the site of application. The preferred dosage to be administered also is likely to depend on such variables such as the condition of the dental tissues particularly of the dentinal surface to which morphogen is to be applied, the size of the tooth or dentinal surface to be treated, extent of dental tissue loss or recession, and the overall health status of the particular patient. In general, 0.00001-1000 mg of morphogen are sufficient with 0.0001-100 mg being preferable and 0.001 to 10 mg being even more preferable for primate teeth, including human teeth. No obvious morphogen induced pathological lesions arise when mature morphogen (e.g., OP-1, 20 mg) is administered daily to normal growing rats for 21 consecutive days. Moreover, 10 mg systemic injections of morphogen (e.g., OP-1) injected daily for 10 days into normal newborn mice does not produce any gross abnormalities.

Practice of the invention, including additional preferred aspects and embodiments thereof, will be still more fully understood from the following examples, which are presented herein for illustration only and should not be construed as limiting the invention in any way.

### IV. Examples

### Example 1: Morphogen-Induced Dentinogenesis in Mammalian Teeth

The following studies demonstrate the efficacy of morphogens in inducing dentine tissue morphogenesis in model mammals. Human dental pulp has been observed to respond unpredictably to injury. Currently, this represents a basic clinical problem in dentistry. Accordingly, primates are used herein as model mammals for demonstration of dentine regeneration. Those skilled in the dental arts will understand and appreciate the correlation between human and nonhuman primate dental biology.

Recombinant human osteogenic protein-1 (hOP-1, Seq. ID No. 4), when applied to freshly cut primate residual dentine, stimulated significantly more reparative dentine formation than calcium hydroxide paste (a conventional treatment). The response to OP-1 was dependent upon the concentration applied to the tooth as a cavity liner as well as the thickness of the residual dentine. The response to calcium hydroxide similarly was dependent upon the thickness of residual dentine.

Dentine matices have been shown to contain bone morphogenetic protein (BMP) activity (Bang and Urist (1967), **94** *Arch. Surg*. 781-789; Youmans and Urist (1967), **12** *Arch. Oral. Biol.* 999-1008; Butler *et al.* (1977), **56** *J. Dent. Res.* 288-232; Bessho *et al.* (1990), **70** *J. Dent. Res.* 171-175), growth factors (Finklemen *et al.* (1990), **5** *J. Bone Min. Res.* 717-723) and dentinogenic activity (Anneroth and Bang (1972), **23** *Odont. Rev.* 315-328; Nakashima, M. (1989), **5** *Endodont. Dent. Traumat.* 279-286; Nakashima, M. (1990), **35** *Arch. Oral. Biol.* 493-497; Nakashima, M. (1990), **35** *Arch. Oral. Biol.* 277-281; Tziafas and Kolokuris (1990), **69** *.J. Dent. Res.* 75-81; Tziafas *et al.* (1992), **37** *Arch. Oral. Biol.* 119-128; Smith *et al.* (1994), **39** *Arch. Oral. Biol.* 13-22). Impure extracts of dentine with BMP activity (Nakashima, M. (1990), **35** *Arch. Oral. Biol.* 493-497; Nakashima, M. (1990), **35** *Arch. Oral. Biol.* 277-281), recombinant BMP-2, and BMP-4 (Nakashima, M. (1994), **73** *J. Dent. Res.* 1515-1522) and recombinant human osteogenic protein-1 (OP-1, BMP-7) (Rutherford *et al.* (1993), **38** *Arch. Oral. Biol.* 571-576; Rutherford *et al,* (1994), **39** *Arch. Oral. Biol.* 833-838) induce reparative dentineogenesis when placed on partially amputated pulps in mature adult teeth, see also U.S.S.Nos. 07/752,764 (published as WO92/15323) and 08/155,343 (published as WO94/06399). In addition, dental pulps (Vaino *et al.* (1993), **75** *Cell.* 45-58; Heikinheimo, H. (1994), **73** *.J. Dent. Res.* 590-597) or cells derived from dental pulps (Takeda *et al.* (1994), **15** *Bone* 467-470) differentially express some morphogen genes. Accordingly, the present study explored whether solubilized OP-1 induced dentine formation when placed on freshly cut dentine surfaces in monkey permanent teeth.

Ninety (90) incisor, premolar and molar permanent teeth were anesthetized with Carbocaine (Cook-Waite) without vasoconstrictor, isolated by rubber dam, cleaned with a coolant. The variation in the area of the pulpal floors was less than 10% and the mean thickness of the residual floor dentine varied from approximately 0.1 to 0.9 mm between different teeth (as measured histomorphometrically). The pulpal floors were covered a fixed volume of an evaporative solution containing 0.01, 0.1, 1 or 10µg OP-1 in acid-alcohol (28.5% ethanol, 0.025% HCL), acid-alcohol alone, a thin layer of calcium hydroxide paste (Dycal, L.D. Caulk, Wilmington DE) or filled without a liner (no treatment). The cavities were filled with Ketac Silver (ESPE-Premier, Norristown, PA) according to standard reconstructive techniques. It will be recognized that any standard dental reconstructive material could be used. The animals were euthanized two months following surgery, specimens obtained and analyzed as described in the literature (Rutherford *et al.* (1993), **38** *Arch. Oral. Biol.* 571-576.

All procedures described above and involving animals were approved by and performed in an accredited animal care facility with extensive experience managing non-human primates. These studies were conducting using 5 adolescent (mixed dentition) male *Macaca fasicularis* of approximately 4-6 kg each. Dental procedures were performed on animals heavily sedated with, e.g., ketamine (15 mg/kg body wt.) and acepromazine (0.55 mg/kg body wt) supplemented with local intraoral infiltration anesthesia (without vasoconstrictor).

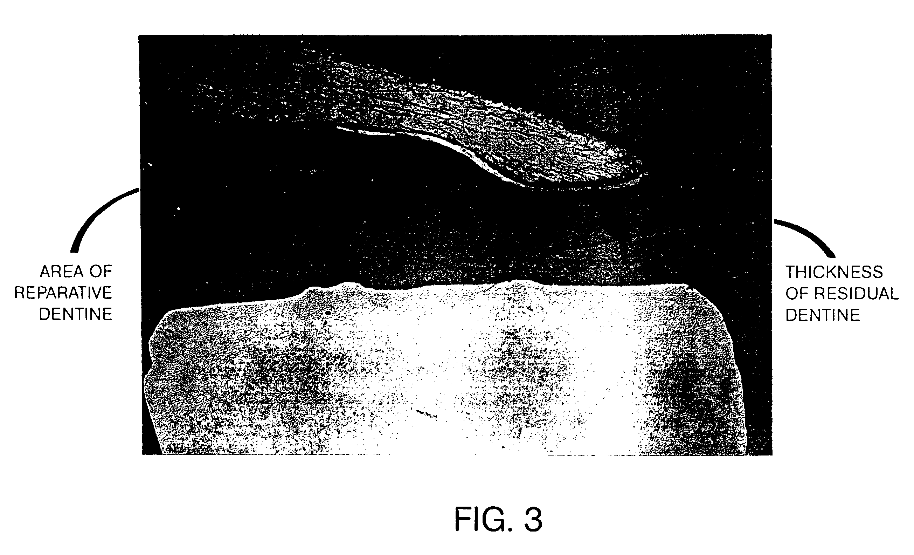

The variable amounts of reparative dentine observed in this study typically were limited in area to the dentinal surface transverse to the luminae of cut dentinal canaliculi. FIGURE 3 is a digitized video image of a typical tissue section prepared from an OP-1 treated animal by standard histological techniques. FIGURE 3 shows that reparative dentine formed deep to those dentinal canaliculi cut during preparation of the tooth. In most cases, the reparative dentine was present in all sections in which both the pulpal floor of the cavity preparation and the subjacent pulp chamber were evident. The spatial relationship of the mass of reparative dentine to the pulpal floor appeared to be governed by the orientation of the dentinal canaliculi to the long axis of the tooth and to the surface area of cut dentine intersecting the canaliculi. This spatial orientation suggests that OP-1 diffused through the dentinal canaliculi.

Indeed, the area of new dentine formation two months after morphogen treatment further depended on the dose of OP-1 applied. FIGURE 4 shows histomorphometric results illustrating this relationship. The mean thickness of the residual dentine was determined by averaging three separate and representative histomorphometric measurements in each of 5 sections distributed over 75% of the surface area of the cavity preparation. In FIGURE 4, the mean area of reparative dentine was determined by averaging three replicate histomorphometric measurements in each of five (5) tissue sections distributed over 75% of the surface area of the cavity preparation. In contrast, there were no significant differences between the amount of reparative dentine deep to the cut dentinal canaliculi in teeth to which no liners were applied (no treatment) and those treated with evaporative carrier alone.

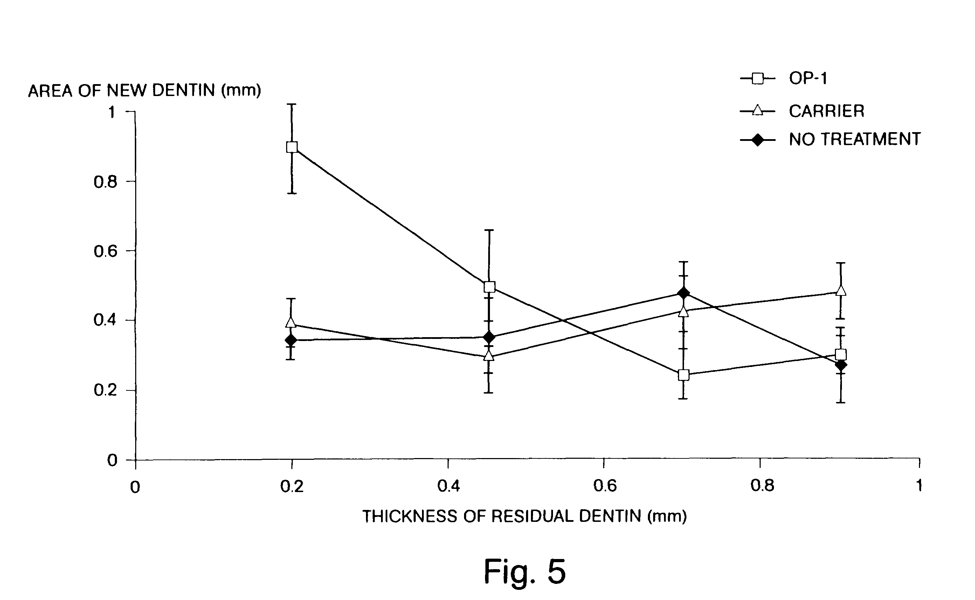

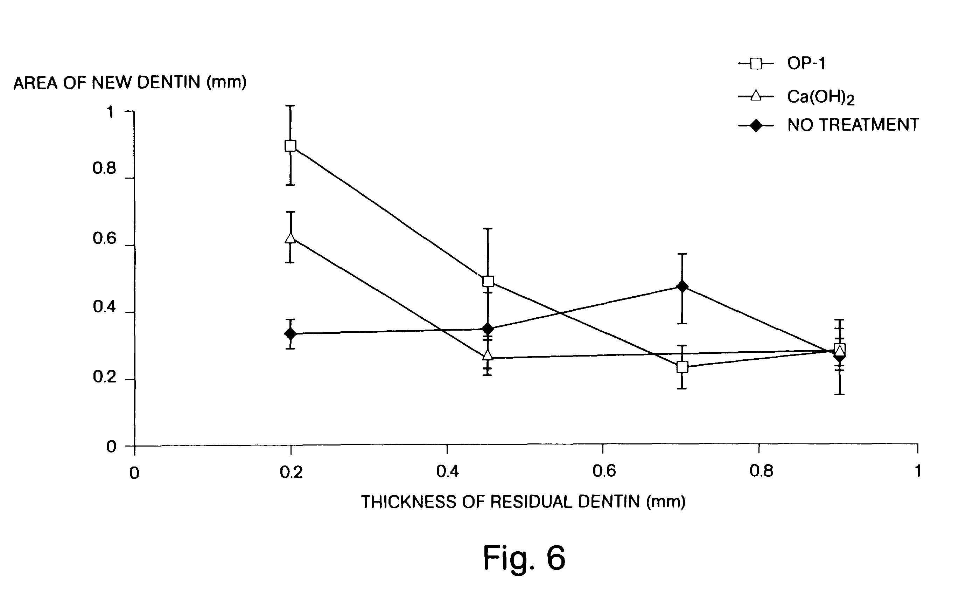

As shown in FIGURES 5 and 6, equivalent amounts of OP-1 (e.g., 10µg in fixed equivalent volumes per tooth) stimulated significantly more reparative dentine two months after treatment than all other treatments attempted, including calcium hydroxide. The degree of stimulation related to the thickness of residual dentine separating the site of morphogen application from the pulp chamber wall, and became particularly evident as the thickness of residual dentine approached 0.2 mm. Each graphed residual dentine value (0.2, 0.45, 0.75 and 0.9 mm) represents a group of calculated values which ranged up to ± 0.15mm. Thus, the area of reparative dentine present two (2) months after lining the cavities with 10 µg OP-1, a thin layer of calcium hydroxide, or evaporative carrier alone is expressible as a function of the thickness of the residual dentine remaining in the pulpal floor. More reparative dentine was present in OP-1 treated than calcium hydroxide treated teeth (ANOVA, Scheffe's F, P<0.05), in calcium hydroxide than carrier treated teeth (P<0.05), and in OP-1 than carrier treated teeth (P<0.01). OP-1 at 1µg and calcium hydroxide were equipotent over the range of thicknesses of residual dentine (not shown). Smaller amounts of OP-1 were poorly effective in cavities of the size assessed in this study.

Resection of the dentinal canaliculi may result in odontoblast death, particularly in the deeper preparations (Lee *et al.* (1992), *AM. J. Den.* 64-68). However, it is possible that the tooth preparation procedure utilized preserved odontoblasts even in the deepest preparations (Smith *et al.* (1994), **39** *Arch. Oral. Biol.* 13-22). Hence, the dentine formed in these studies may be reactionary dentine, formed by stimulation of the phenotypic function the original odontoblasts, or reparative dentine formed by newly differentiated cells deep to the lost odontoblasts (Lesot *et al*. (1993), **3** *Cells and Materials* 201-217; Smith *et al.* (1994), **39** *Arch. Oral. Biol.* 13-22). The design utilized in these studies did not permit temporal observations of the odontoblast layer deep to the cut dentinal canaliculi. Earlier studies demonstrated the capacity of OP-1 complexed to an insoluble collagen-based carrier to stimulate reparative dentine when placed directly upon partially amputated pulps (U.S.S.N. 08/155,343 (published as WO94/06399) and Rutherford *et al.* (1993), **38** *Arch. Oral. Biol.* 571-576; Rutherford *et al.* (1994), **39** *Arch. Oral. Biol.* 833-838). Partial pulp amputation obviously removes the layer of odontoblasts, exposing the deeper fibrous connective tissue of the pulp. Human pulp cells are responsive to OP-1 *in vitro,* further suggesting that pulp itself contains responsive (competent) cells. The specific phenotypes of these OP-1 responsive pulp cells have not yet been identified conclusively.

### Example 2. Preparation of Soluble Morphogen Complexes useful in Stimulating Dentineogesis

A currently preferred form of the morphogen useful herein, having improved solubility in aqueous solutions, is a dimeric morphogenic protein comprising at least the C-terminal seven cysteine domain characteristic of the morphogen family, complexed with a peptide comprising a pro region of a member of the morphogen family, or a solubility-enhancing fragment thereof, or an allelic, species or other sequence variant thereof. Preferably, the dimeric morphogenic protein is complexed with two pro region peptides. Also, the dimeric morphogenic protein preferably is noncovalently complexed with the pro region peptides. The pro region peptides preferably comprise at least the N-terminal eighteen amino acids that define the pro domain of a given naturally occurring morphogen, or an allelic or phylogenetic counterpart variant thereof. In other preferred embodiments, peptides defining substantially the full length pro domain are used.

Other soluble forms of morphogens include dimers of the uncleaved pro forms of these proteins, as well as "hemi-dimers" wherein one subunit of the dimer is an uncleaved pro form of the protein, and the other subunit comprises the mature form of the protein, including truncated forms thereof, preferably noncovalently associated with a cleaved pro domain peptide.

As described above and in U.S.S.N. 08/040,510 (published as WO94/03600, useful pro domains include the full length pro regions, as well as various truncated forms hereof, particularly truncated forms cleaved at proteolytic Arg-Xaa-Xaa-Arg (Seq. ID No. 32) cleavage sites within the pro domain polypeptidle. For example, in OP-1, possible pro sequences include sequences defined by residues 30-292 (full length form); 48-292; and 158-292. Soluble OP-1 complex stability is best enhanced when the pro region comprises the full length form rather than a truncated form, such as the residues 48-292 truncated form, in that residues 30-47 show sequence homology to the N-terminal portions of other morphogens, and currently are believed to have particular utility in enhancing complex stability for all morphogens. Accordingly, currently preferred pro domains include peptides comprising at least the N-terminal fragment, e.g., amino acide residues 30-47 of a naturally occurring morphogen pro domain, or a biosynthetic variant thereof that retains the solubility and/or stability enhancing properties of the naturally-occurring peptide.

As will be appreciated by those having ordinary skill in the art, useful sequences encoding the pro region can be obtained from genetic sequences encoding known morphogens. Alternatively, chimeric pro regions can be constructed from the sequences of one or more known morphogens. Still another option is to create a synthetic sequence variant of one or more known pro region sequences.

In another preferred aspect, useful pro region peptides include polypeptide chains comprising an amino acid sequence encoded by a nucleic acid that hybridizes under stringent conditions with a DNA or RNA sequence encoding at least the N-terminal eighteen amino acids of the pro region sequence for OP1 or OP2, e.g., nucleotides 136-192 and 152-211 of Seq. ID No. 15 and 19, respectively.

### 2.1 Isolation of soluble morphogen complex from conditioned media or body fluid

Morphogens are expressed from mammalian cells as soluble complexes. Typically, however the complex is disassociated during purification, generally by exposure to denaturants often added to the purification solutions, such as detergents, alcohols, organic solvents, chaotropic agents and compounds added to reduce the pH of the solution. Provided below is a currently preferred protocol for purifying the soluble proteins from conditioned media (or, optionally, a body fluid such as serum, cerebrospinal or peritoneal fluid), under non-denaturing conditions. The method is rapid, reproducible and yields isolated soluble morphogen complexes in substantially pure form.

Soluble morphogen complexes can be isolated from conditioned media using a simple, three step chromatographic protocol performed in the absence of denaturants. The protocol involves running the media (or body fluid) over an affinity column, followed by ion exchange and gel filtration chromatographies. The affinity column described below is a Zn-IMAC column. The present protocol has general applicability to the purification of a variety of morphogens, all of which are anticipated to be isolatable using only minor modifications of the protocol described below. An alternative protocol also envisioned to have utility includes an immunoaffinity column, created using standard procedures and, for example, using antibody specific for a given morphogen pro domain (complexed, for example, to a protein A-conjugated Sepharose column). Protocols for developing immunoaffinity columns are well described in the art, (see, for example, Guide to Protein Purification, M. Deutscher, ed., Academic Press, San Diego, 1990, particularly sections VII and XI thereof).

In this study, OP-1 was expressed in mammalian (CHO, chinese hamster ovary) cells as described in the art (see, for example, international application US90/05903 (WO91/05802). The CHO cell conditioned media containing 0.5% FBS was initially purified using Immobilized Metal-Ion Affinity Chromatography (IMAC). The soluble OP-1 complex from conditioned media binds very selectively to the Zn-IMAC resin and a high concentration of imidazole (50 mM imidazole, pH 8.0) is required for the effective elution of the bound complex. The Zn-IMAC step separates the soluble OP-1 from the bulk of the contaminating serum proteins that elute in the flowthrough and 35 mM imidazole wash fractions. The Zn-IMAC purified soluble OP-1 is next applied to an S-Sepharose cation-exchange column equilibrated in 20 mM NaPO₄ (pH 7.0) with 50 mM NaCl. This S-Sepharose step serves to further purify and concentrate the soluble OP-1 complex in preparation for the following gel filtration step. The protein was applied to a Sephacryl S-200HR column equilibrated in TBS. Using substantially the same protocol, soluble morphogens also can be isolated from one or more body fluids, including serum, cerebrospinal fluid or peritoneal fluid.

IMAC was performed using Chelating-Sepharose (Pharmacia) that had been charged with three column volumes of 0.2 M ZnSO₄. The conditioned media was titrated to pH 7.0 and applied directly to the ZN-IMAC resin equilibrated in 20 mM HEPES (pH 7.0) with 500 mM NaCl. The Zn-IMAC resin was loaded with 80 mL of starting conditioned media per mL of resin. After loading, the column was washed with equilibration buffer and most of the contaminating proteins were eluted with 35 mM imidazole (pH 7.0) in equilibration buffer. The soluble OP-1 complex then is eluted with 50 mM imidazole (pH 8.0) in 20 mM HEPES and 500 mM NaCl.

The 50 mM imidazole eluate containing the soluble OP-1 complex was diluted with nine volumes of 20 mM NaPO₄ (pH 7.0) and applied to an S-Sepharose (Pharmacia) column equilibrated in 20 mM NaPO₄ (pH 7.0) with 50 mM NaCl. The S-Sepharose resin was loaded with an equivalent of 800 mL of starting conditioned media per mL of resin. After loading the S-Sepharose column was washed with equilibration buffer and eluted with 100 mM NaCl followed by 300 mM and 500 mM NaCl in 20 mM NaPO4 (pH 7.0). The 300 mM NaCl pool was further purified using gel filtration chromatography. Fifty mls of the 300 mm NaCl eluate was applied to a 5.0 X 90 cm Sephacryl S-200HR (Pharmacia) equilibrated in Tris buffered saline (TBS), 50 mM Tris, 150 mM NaCl (pH 7.4). The column was eluted at a flow rate of 5 mL/minute collecting 10 mL fractions. The apparent molecular of the soluble OP-1 was determined by comparison to protein molecular weight standards (alcohol dehydrogenase (ADH, 150 kDa), bovine serum albumin (BSA, 68 kDa), carbonic anhydrase (CA, 30 kDa) and cytochrome C (cyt C, 12.5 kDa). The purity of the S-200 column fractions was determined by separation on standard 15% polyacrylamide SDS gels stained with coomassie blue. The identity of the mature OP-1 and the pro-domain was determined by N-terminal sequence analysis after separation of the mature OP-1 from the pro-domain using standard reverse phase C18 HPLC.

The soluble OP-1 complex elutes with an apparent molecular weight of 110 kDa. This agrees well with the predicted composition of the soluble OP-1 complex with one mature OP-1 dimer (35-36 kDa) associated with two pro-domains (39 kDa each). Purity of the final complex can be verified by running the appropriate fraction in a reduced 15% polyacrylamide gel.

The complex components can be verified by running the complex-containing fraction from the S-200 or S-200HR columns over a reverse phase C18 HPLC column and eluting in an acetonitrile gradient (in 0.1% TFA), using standard procedures. The complex is dissociated by this step, and the pro domain and mature species elute as separate species. These separate species then can be subjected to N-terminal sequencing using standard procedures (see, for example, Guide to Protein Purification, M. Deutscher, ed., Academic Press, San Diego, 1990, particularly pp. 602-613), and the identity of the isolated 36kD, 39kDa proteins confirmed as mature morphogen and isolated, cleaved pro domain, respectively. N-terminal sequencing of the isolated pro domain from mammalian cell produced OP-1 revealed 2 forms of the pro region, the intact form (beginning at residue 30 of Seq. ID No. 16) and a truncated form, (beginning at residue 48 of Seq. ID No. 16.) N-terminal sequencing of the polypeptide subunit of the isolated mature species reveals a range of N-termini for the mature sequence, beginning at residues 293, 300, 313, 315, 316, and 318, of Seq. ID No. 16, all of which are active as demonstrated by the standard bone morphogenesis assay set forth in U.S.S.N. 07/752,764 (published as WO92/15323).

### 2.2 In Vitro Soluble Morphogen Complex Formation

As an alternative to purifying soluble complexes from culture media or a body fluid, soluble complexes can be formulated from purified pro domains and mature dimeric species. Successful complex formation apparently requires association of the components under denaturing conditions sufficient to relax the folded structure of these molecules, without affecting disulfide bonds. Preferably, the denaturing conditions mimic the environment of an intracellular vesicle sufficiently such that the cleaved pro domain has an opportunity to associate with the mature dimeric species under relaxed folding conditions. The concentration of denaturant in the solution then is decreased in a controlled, preferably step-wise manner, so as to allow proper refolding of the dimer and pro regions while maintaining the association of the pro domain with the dimer. Useful denaturants include 4-6M urea or guanidine hydrochloride (GuHCl), in buffered solutions of pH 4-10, preferably pH 6-8. The soluble complex then is formed by controlled dialysis or dilution into a solution having a final denaturant concentration of less than 0.1-2M urea or GuHCl, preferably 1-2 M urea of GuHCl, which then preferably can be diluted into a physiological buffer. Protein purification/renaturing procedures and considerations are well described in the art, and details for developing a suitable renaturing protocol readily can be determined by one having ordinary skill in the art. One useful text on the subject is Guide to Protein Purification, M. Deutscher, ed., Academic Press, San Diego, 1990, particularly section V Complex formation also may be aided by addition of one or more chaperone proteins.

### 2.3 Stability of Soluble Morphogen Complexes

The stability of the highly purified soluble morphogen complex in a physiological buffer, e.g., tris-buffered saline (TBS) and phosphate-buffered saline (PBS), can be enhanced by any of a number of means. Currently preferred is by means of a pro region that comprises at least the first 18 amino acids of the pro sequence (e.g., residues 30-47 of Seq. ID NO. 16 for OP-1), and preferably is the full length pro region. Residues 30-47 show sequence homology to the N-terminal portion of other morphogens and are believed to have particular utility in enhancing complex stability for all morphogens. Other useful means for enhancing the stability of soluble morphogen complexes include three classes of additives. These additives include basic amino acids (e.g., L-arginine, lysine and betaine); nonionic detergents (e.g., Tween 80 or NonIdet P-120); and carrier proteins (e.g., serum albumin and casein). Useful concentrations of these additives include 1-100 mM, preferably 10-70 mM, including 50 mM, basic amino acid;, 0.01-1.0%, preferably 0.05-0.2%, including 0.1% (v/v) nonionic detergent;, and 0.01-1.0%, preferably 0.05-0.2%, including 0.1% (w/v) carrier protein.

## Claims

1. Use of a morphogen for the manufacture of a medicament for use in:
(a) stimulating morphogenesis of dentine in a mammalian tooth; or
(b) stimulating phenotypic expression of mammalian odontoblasts; or
(c) stimulating production of dentine matrix by mammalian odontoblasts; or
(d) increasing thickness of a mammalian tooth wall; or
(e) reducing risk of fracture in a mammalian tooth; or
(f) desensitizing a mammalian tooth to perception of pressure or temperature; or
(g) sealing a cavity in a mammalian tooth; wherein said medicament is applyied to a dentinal surface.

2. The use of claim 1 wherein the dentinal surface either: (i) adjoins a site of lost or damaged enamel, dentine or cementum tissue, such as a cavity, of said tooth, or (ii) adjoins a site of lost or damaged gingival tissue.

3. The use of claim 1 or claim 2 wherein the dentinal surface has been treated either to:- (i) ablate damaged or infected enamel, dentine or cementum tissue from the site of said cavity, or (ii) debride damaged gingival, enamel, dentine or cementum tissue from said dentinal surface.

4. The use of any one of claims 1 to 3 comprising the application of said morphogen in an amount effective for stimulating formation of reparative dentine apposite said dentinal surface.

5. The use of claim 4 wherein said dentinal surface is transverse to luminae of dental canaliculi within said tooth.

6. The use of any one of the preceding claims wherein said dentinal surface is separated from the pulp chamber wall of said tooth by up to about 1 mm of residual dentine.

7. The use of any one of the preceding claims wherein:
(A) said morphogen is solubilised in a physiologically acceptable vehicle or an evaporative vehicle; or
(B) said morphogen is adsorbed on a biocompatible, acellular matrix suitable for sealing or filling defects in mammalian teeth.

8. The use of claim 7(A) wherein the morphogen is solubilized by the preparative step of solubilising said morphogen in a physiologically acceptable vehicle or an evaporative vehicle, said vehicle optionally further comprising a cofactor that mitigates symptoms associated with tooth damage.

9. The use of claim 7(B) wherein the morphogen is adsorbed by the preparative step of adsorbing said morphogen on a biocompatible, acellular matrix suitable for sealing or filling defects in mammalian teeth, said matrix optionally further comprising a cofactor that mitigates symptoms associated with tooth damage.

10. The use of any one of the preceding claims wherein said morphogen comprises a dimeric protein that induces morphogenesis of mammalian dentine tissue, said dimeric protein comprising a pair of folded polypeptides, the amino acid sequence of each of which comprises
(i) a sequence sharing at least 70% homology with the C-terminal seven cysteine domain of human OP1, residues 38-139 of Seq. ID No. 4;
(ii) a sequence encoded by a nucleic acid that hybridizes under stringent conditions with nucleic acid encoding said domain of human OP1; or
(iii) a sequence defined by Generic Sequence 8, Seq. ID No. 2.

11. The use of claim 10 wherein: (i) said sequence of said morphogen polypeptides is defined by OPX, Seq. ID No. 3 and/or (ii) the morphogen is obtained from culture medium of morphogen-secreting mammalian cells.

12. The use of claim 10 or 11 wherein said sequence of said morphogen polypeptides is selected independently in each said polypeptide from the sequences of the C-terminal seven cysteine domains of human OP1, mouse OP1, human OP2, mouse OP2, mouse OP3, Drosophila 60A protein, Xenopus Vgl, mouse Vgr-1, mouse GDF-1, Drosophila DPP, CBMP2A, CBMP2B, BMP3, BMP5, BMP6 (shown in Seq. ID Nos. 4, 5, 6, 7, 8, 9, 10, 11, 12, 13, 24, 26, 27, 28 and 29), and allelic, phylogenetic and biosynthetic variants thereof.

13. The use of claim 10 or 11 wherein said sequence of said morphogen polypeptides is selected, in each said polypeptide, from the sequences of the C-terminal seven cysteine domains of human OP-1, human OP-2, mouse OP-1, mouse OP-2, mouse OP-3 and Drosophila 60A protein (shown in seq. ID Nos. 4, 5, 6, 7, 24 and 26), and allelic, phylogenetic and biosynthetic variants thereof.

14. The use of claim 10 or 11 wherein said morphogen is solubilised by association with at least one morphogen prodomain polypeptide or solubility-enhancing fragment thereof.

## Patentansprüche

1. Verwendung eines Morphogens zur Herstellung eines Medikamentes zur Verwendung bei:
(a) Stimulieren der Dentinmorphogenese in einem Säugetierzahn; oder
(b) Stimulieren der phänotypischen Expression von Säugetier- Odontoblasten; oder
(c) Stimulieren der Erzeugung von Dentinmatrix durch Säugetier- Odontoblasten; oder
(d) Erhöhen der Dicke eines Säugetierzahnes; oder
(e) Vermindern des Bruchrisikos bei einem Säugetierzahn; oder
(f) Desensibilisieren eines Säugetierzahns gegenüber dem Wahrnehmungsvermögen von Druck oder Temperatur; oder
(g) Versiegeln einer Höhle in einem Säugetierzahn; wobei das Medikament auf eine Dentinal-Oberfläche aufgebracht wird.

2. Verwendung nach Anspruch 1, bei der die Dentinal-Oberfläche entweder: (i) an eine Stelle verlorenen oder beschädigten Zahnschmelzes, Dentin- oder Zementgewebes, wie beispielsweise einer Höhle dieses Zahnes, angrenzt oder (ii) an eine Stelle eines verlorenen oder beschädigten Zahnfleisch-Gewebes angrenzt.

3. Verwendung nach Anspruch 1 oder Anspruch 2, bei der die Dentinal-Oberfläche entweder zum: (i) Abtragen von beschädigtem oder infiziertem Zahnschmelz, Dentin- oder Zement gewebe von der Stelle dieser Kavität oder zum (ii) Reinigen von geschädigtem Zahnfleisch-, Zahnschmelz-, Dentin- oder Zementgewebe von dieser Dentinal-Oberfläche behandelt wird.

4. Verwendung nach einem der Ansprüche 1-3, die die Anwendung des Morphogens in einer zur Stimulierung der Bildung von Reparaturdentin wirksamen Menge umfaßt, die der Dentinal-Oberfläche angemessen ist.

5. Verwendung nach Anspruch 4, bei der die Dentinal-Oberfläche quer zu Hohlräumen von Dentalkanälchen innerhalb des Zahns verläuft.

6. Verwendung nach einem der vorhergehenden Ansprüche, bei der die Dentinal- Oberfläche von der Pulpakammerwand des Zahns von bis zu ungefähr 1 Millimeter Restdentin getrennt ist.

7. Verwendung nach einem der vorhergehenden Ansprüche, bei der:
(A) Das Morphogen in einem physiologisch akzeptablen Träger oder einem Verdunstungsträger solubilisiert ist; oder
(B) das Morphogen an eine biokompatible, azelluläre Matrix adsorbiert ist, die zum Versiegeln oder Füllen von Defekten in Säugetierzähnen geeignet ist.

8. Verwendung nach Anspruch 7A, bei der das Morphogen durch den Vorbereitungsschritt, das Morphogen in einem physiologisch akzeptablen Träger oder in einem Verdunstungsträger zu solubilisieren, solubilisiert wird, wobei der Träger optional weiterhin einen Co-Faktor umfaßt, der mit Zahnbeschädigung verbundene Symptome lindert.

9. Verwendung nach Anspruch 7B, bei der das Morphogen durch den Vorbereitungsschritt des Adsorbierens des Morphogens an eine biokompatible, azelluläre Matrix adsorbiert wird, die zum Versiegeln und Füllen von Defekten in Säugetierzähnen geeignet ist, wobei die Matrix optional weiterhin einen Co-Faktor umfaßt, der mit einer Zahnbeschädigung verbundene Symptome lindert.

10. Verwendung nach einem der vorhergehenden Ansprüche, bei der das Morphogen ein dimeres Protein umfaßt, das die Morphogenese von Säugetier-Dentingewebe induziert, wobei das dimere Protein zwei gefaltete Polypeptide umfaßt, deren Aminosäuresequenz jeweils
(i) eine Sequenz, die zumindest 70% Homologie mit der C-terminalen 7-Cystein-Domäne von menschlichem OP1, Reste 38 bis 139 von Sequenz ID Nr. 4, teilt;
(ii)eine Sequenz, die von einer Nukleinsäure kodiert wird, die unter stringenten Bedingungen mit einer Nukleinsäure hybridisiert, die diese Domäne von menschlichem OP1 kodiert; oder
(iii)eine Sequenz umfaßt, die durch Gattungssequenz 8, Sequenz ID-Nr. 2 definiert ist.

11. Verwendung nach Anspruch 10, bei der: (i) die Sequenz dieser Morphogen- Polypeptide durch OPX, Sequenz ID-Nr. 3 definiert ist und/oder (ii) das Morphogen aus Kulturmedium Morphogen-sezernierender Säugetierzellen gewonnen wird.

12. Verwendung nach Anspruch 10 oder 11, bei der die Sequenz des Morphogen- Polypeptids unabhängig bei jedem dieser Polypeptide aus den Sequenzen der C-Terminalen 7-Cystein-Domänen von menschlichem OP1, Maus-OP1, menschlichem OP2, Maus-OP2, Maus-OP3, Drosophila 60A-Protein, Xenopus Vgl, Maus Vgr-1, Maus-GDF-1, Drosophila-DPP, CBMP2A, CBMP2B, BMP3, BMP5, BMP6 (dargestellt in den Sequenz ID-Nr. 4, 5, 6, 7, 8, 9, 10, 11, 12, 13, 24, 26, 27, 28 und 29) und Allel-, phylogenetische und biosynthetische Varianten hiervon, ausgewählt ist.

13. Verwendung nach Anspruch 10 oder 11, bei der die Sequenz des Morphogen- Polypeptids bei jedem Polypeptid aus den Sequenzen der C-Terminalen 7-Zystein-Domänen von menschlichem OP1, menschlichem OP2, Maus-OP1, Maus-OP2, Maus-OP3 und Drosophila 60A-Protein (dargestellt in den Sequenz ID-Nr. 4, 5, 6, 7, 24 und 26) und Allel-, phylogenetischen und biosynthetischen Varianten hiervon, ausgewählt ist.

14. Verwendung nach Anspruch 10 oder 11, bei der das Morphogen durch Verbindung mit zumindest einem Morphogen-Prodomän-Polypeptid oder Löslichkeits-verbessernden Bruchstücks hiervon solubilisiert wird.

## Revendications

1. Utilisation d'un morphogène pour la fabrication d'un médicament pour une utilisation :
(a) pour la stimulation de la morphogenèse de la dentine de la dent d'un mammifère; ou
(b) pour la stimulation de l'expression phénotypique des odontoblastes de mammifères; ou
(c) pour la stimulation de la production de la matrice de la dentine par des odontoblastes de mammifères; ou
(d) pour l'augmentation de l'épaisseur de la paroi d'une dent de mammifère; ou
(e) pour la réduction du risque de fracture d'une dent de mammifère; ou
(f) pour la désensibilisation de la dent d'un mammifère à la perception d'une pression ou d'une température; ou
(g) pour le scellage d'une cavité d'une dent de mammifère;
au cours de laquelle le dit médicament est appliqué sur une surface dentinale.

2. Utilisation selon la revendication 1 au cours de laquelle la surface dentinale soit: (i) est contiguë à un site de perte ou d'endommagement de l'émail, de la dentine ou d'un tissu du cément, tel qu'une cavité de cette dent soit (ii) est contiguë à un site de perte ou d'endommagement d'un tissu gingival.

3. Utilisation selon la revendication 1 ou 2 au cours de laquelle la surface dentinale a été traitée soit pour:- (i) pratiquer l'ablation d'un émail, d'une dentine, ou d'un tissu du cément endommagé ou infecté à partir du site de cette cavité soit (ii) débrider 1 'émail, la dentine ou le tissu du cément gingival endommagé à partir de cette surface dentinale.

4. Utilisation selon l'une quelconque des revendication 1 à 3 comprenant l'application de ce morphogène en une quantité efficace pour stimuler la formation de la dentine réparatrice imposée à cette surface dentinale.

5. Utilisation selon la revendication 4 au cours de laquelle cette surface dentinale est transversale par rapport aux lumières des canalicules dentaires à l'intérieur de cette dent.

6. Utilisation selon l'une quelconque des revendications précédentes au cours de laquelle cette surface dentinaire est séparée de la paroi de la chambre de la pulpe de cette dent par jusqu'à environ 1 mm de dentine résiduelle.

7. Utilisation selon l'une quelconque des revendications précédentes au cours de laquelle:
(A) le dit morphogène est solubilisé dans un véhicule physiologiquement acceptable ou un véhicule évaporateur; ou
(B) le dit morphogène est adsorbé sur une matrice acellulaire, biocompatible, appropriée pour fermer ou combler des défauts dans les dents de mammifères.

8. Utilisation selon la revendication 7 (A) au cours de laquelle le morphogène est solubilisé par l'étape préparatoire de solubilisation de ce morphogène dans un véhicule physiologiquement acceptable ou un véhicule évaporateur, ce véhicule comprenant en outre facultativement un cofacteur qui mitige les symptômes associés à un dommage d'une dent.

9. Utilisation selon la revendication 7 (B) au cours de laquelle le morphogène est adsorbé par l'étape préparatoire d'adsorption de ce morphogène sur une matrice acellulaire biocompatible, appropriée pour fermer ou combler des défauts dans les dents des mammifères, cette matrice comprenant en outre un cofacteur qui mitige les symptômes associés à un dommage d'une dent.

10. Utilisation selon l'une quelconque des revendications précédentes au cours de laquelle ce morphogène comprend un protéine dimère qui induit la morphogenèse d'un tissu de la dentine d'un mammifère, cette protéine dimère contenant une paire de polypeptides repliés, dont la séquence d'aminoacides de chacune d'entr'elles comprend
(i) une séquence partageant au moins 70 % d'homologie avec le domaine C-terminal à sept cystéines de l'OP1 humaine, les résidus 38-139 de la séquence ID No. 4;
(ii) une séquence codée par un acide nucléique qui s'hybride sous des conditions stringentes avec un acide nucléique codant pour ce domaine de l'OP1 humaine; ou
(iii) une séquence définie par la séquence générique 8, Seq. ID No. 2.

11. Utilisation selon la revendication 10 au cours de laquelle: (i) cette séquence de ces polypeptides morphogènes est définie par OPX, Seq. ID No. 3 et/ou (ii) on obtient le morphogène à partir d'un milieu de culture de cellules de mammifère sécrétant un morphogène.

12. Utilisation selon la revendication 10 ou 11 au cours de laquelle cette séquence de ces polypeptides morphogènes est choisie indépendamment dans chacun de ces polypeptides provenant des séquences des domaines C-terminaux à sept cystéines de l'OP1 humaine, OP1 de souris, OP2 humaine, OP2 de souris, OP3 de souris, protéine 60A de la drosophile, Vgl de xénope, Vgr-1 de souris, GDF-1 de souris, DPP de drosophile, CBMP2A, CBMP2B, BMP3, BMP5, BMP6 (montrés dans les Seq. ID No. 4, 5, 6, 7, 8, 9, 10, 11, 12, 13, 24, 26, 27, 28 et 29), des variants allèles, phylogénétiques et biosynthétiques de ceux-ci.

13. Utilisation selon la revendication 10 ou 11 au cours de laquelle cette séquence de ces polypeptides morphogènes est choisie, dans chacun de ces polypeptides, à partir des séquences des domaines C-terminaux à sept cystéines de l'OP1 humaine, OP2 humaine, OP1 de souris, OP2 de souris, OP3 de souris et de la protéine 60A de la drosophile (montrés dans les Seq. ID No. 4, 5, 6, 7, 24 et 26), et les variants allèles, phylogénétiques et biosynthétiques de ceux-ci.

14. Utilisation selon la revendication 10 ou 11 dans laquelle le dit morphogène est solubilisé par association avec au moins un polypeptide du prodomaine du morphogène ou du fragment d'activation de la solubilité de celui-ci.
